(19) Europäisches Patentamt
European Patent Office
Office européen des brevets

(11) Veröffentlichungsnummer: **0 403 828 A1**

# (12) EUROPÄISCHE PATENTANMELDUNG

(21) Anmeldenummer: **90110036.2**

(51) Int. Cl.5: **C07K 5/02, A61K 37/64**

(22) Anmeldetag: **26.05.90**

(30) Priorität: **09.06.89 DE 3918896**
**01.12.89 DE 3939747**

(43) Veröffentlichungstag der Anmeldung:
**27.12.90 Patentblatt 90/52**

(84) Benannte Vertragsstaaten:
**AT BE CH DE DK ES FR GB GR IT LI LU NL SE**

(71) Anmelder: **BAYER AG**

**D-5090 Leverkusen 1 Bayerwerk(DE)**

(72) Erfinder: **Bender, Wolfgang, Dr.**
**Claudiusweg 5**
**D-5600 Wuppertal 1(DE)**
Erfinder: **Schmidt, Gunter, Dr.**
**Pahlkestrasse 63**
**D-5600 Wuppertal 1(DE)**
Erfinder: **Knorr, Andreas, Dr.**
**Trillser 10**
**D-4006 Erkrath(DE)**
Erfinder: **Stasch, Johannes-Peter, Dr.**
**Schneewittchenweg 37**
**D-5600 Wuppertal 1(DE)**

## (54) Renininhibitorische Peptide, Verfahren zur ihrer Herstellung und ihre Verwendung in Arzneimitteln.

(57) Die vorliegende Erfindung betrifft neue renininhibitorische Peptide der allgemeinen Formel (I)

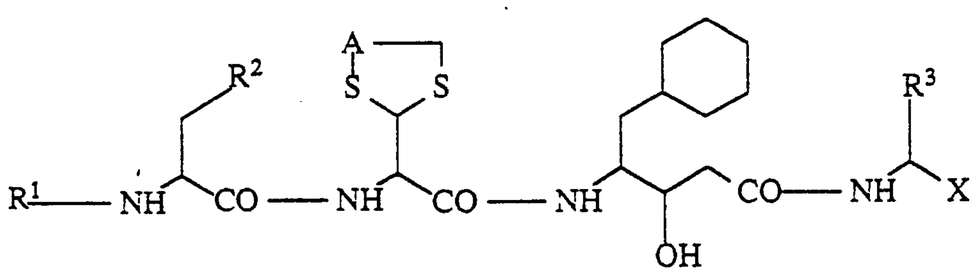

in welcher $R^1$, $R^2$, $R^3$, A und X die in der Beschreibung angegebene Bedeutung haben, Verfahren zu ihrer Herstellung und ihre Verwendung in Arzneimitteln, insbesondere in kreislaufbeeinflussenden Arzneimitteln.

EP 0 403 828 A1

## Renininhibitorische Peptide, Verfahren zu ihrer Herstellung und ihre Verwendung in Arzneimitteln

Die vorliegende Erfindung betrifft neue renininhibitorische Peptide, Verfahren zu ihrer Herstellung und ihre Verwendung in Arzneimitteln, insbesondere in kreislaufbeeinflussenden Arzneimitteln.

Renin ist ein proteolytisches Enzym, das überwiegend von den Nieren produziert und ins Plasma sezerniert wird. Es ist bekannt, daß Renin in vivo vom Angiotensinogen das Dekapeptid Angiotensin I abspaltet. Angiotensin I wiederum wird in der Lunge, den Nieren oder anderen Geweben zu dem blutdruckwirksamen Oktapeptid Angiotensin II abgebaut. Die verschiedenen Effekte des Angiotensin II wie Vasokonstriktion, $Na^+$-Retention in der Niere, Aldosteronfreisetzung in der Nebenniere und Tonuserhöhung des sympathischen Nervensystems wirken synergistisch im Sinne einer Blutdruckerhöhung.

Die Aktivität des Renin-Angiotensin-Systems kann durch die Hemmung der Aktivität von Renin oder dem Angioten sin-Konversionsenzym (ACE) sowie durch Blockade von Angiotensin II-Rezeptoren pharmakologisch manipuliert werden. Die Entwicklung von oral einsetzbaren ACE-Hemmern hat somit zu neuen Antihypertensiva geführt (vgl. DOS 3 628 650, Am. J. Med 77, 690, 1984).

Ein neuerer Ansatz ist, in die Renin-Angiotensin-Kaskade zu einem früheren Zeitpunkt einzugreifen, nämlich durch Inhibition der hochspezifischen Peptidase Renin.

Bisher wurden verschiedene Arten von Renininhibitoren entwickelt: Reninspezifische Antikörper, Phospholipide, Peptide mit der N-terminalen Sequenz des Prorenins, synthetische Peptide als Substratanaloga und modifizierte Peptide.

In der EP-A 202 73 696, EP-A 202 78 158 und in der PCT WO 86/04901 werden renininhibitorische Peptide beschrieben, bei denen der Bedeutungsumfang von $R^2$ (siehe erfindungsgemäße Verbindungen) auch heterocyclische Reste erfaßt, ohne jedoch einen Hinweis auf die spezielle 1,3-Dithialan oder 1,3-Dithiongruppe oder einen konkreten Vertreter dieser Stoffklasse zu geben. Es wurden nun Peptide gefunden, bei denen der übliche Aminosäurerest -His (Histidin) durch einen 1,3-Dithiolan- oder 1,3-Dithionrest ersetzt wurde und die überraschenderweise eine gute renininhibitorische Wirkung besitzen.

Die Erfindung betrifft Peptide der allgemeinen Formel (I)

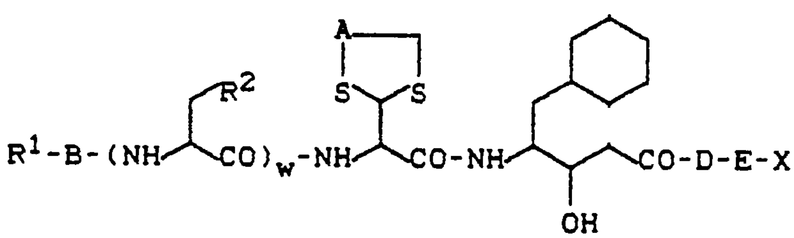 (I)

in welcher

$R^1$ - für Wasserstoff oder

- für Alkoxycarbonyl mit bis zu 8 Kohlenstoffatomen oder Benzyloxycarbonyl steht oder
- für eine Gruppe der Formel $R^3$-CO- steht

worin

$R^3$ - Morpholino oder die Reste

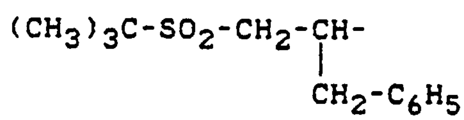

oder 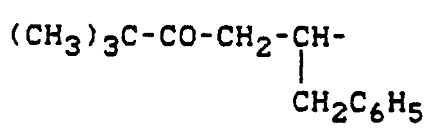

bedeutet

oder

- eine Gruppe der Formel $-NR^4R^5$ bedeutet

worin
$R^4$ und $R^5$ gleich oder verschieden sind und Wasserstoff, geradkettiges oder verzweigtes Alkyl mit bis zu 8 Kohlenstoffatomen oder Phenyl bedeuten
oder
- den Rest

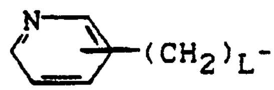

bedeutet
worin
L - die Zahl 0, 1 oder 2 bedeutet
B - für eine direkte Bindung oder
- für eine Aminosäuregruppierung der Formel

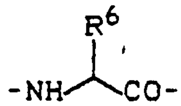

oder

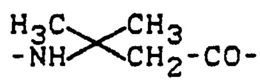

steht
worin
$R^6$ - geradkettiges oder verzweigtes Alkyl mit bis zu 8 Kohlenstoffatomen bedeutet, das gegebenenfalls durch Hydroxy, Carboxy, Alkoxy oder Alkoxycarbonyl mit bis zu 6 Kohlenstoffatomen oder Benzyloxy substituiert ist
oder
- für Prolin steht
in ihrer L-Form, D-Form oder als D,L-Isomerengemisch
$R^2$ - für Aryl mit 6 bis 10 Kohlenstoffatomen steht, das gegebenenfalls bis zu 3-fach gleich oder verschieden durch Halogen, Nitro, Cyano, Hydroxy, gerad kettiges oder verzweigtes Alkoxy mit bis zu 8 Kohlenstoffatomen, Acetoxy, Benzyloxy oder durch eine Gruppe der Formel O-CO-$R^7$ substituiert ist
worin
$R^7$ geradkettiges oder verzweigtes Alkyl mit bis zu 8 Kohlenstoffatomen bedeutet
w - eine Zahl 0 oder 1 bedeutet
A - für eine $-CH_2-$ oder für eine $CH_2$-$CH_2$-Gruppe steht,
D und E gleich oder verschieden sind und
- für eine direkte Bindung oder
- für eine Aminosäuregruppierung der Formel

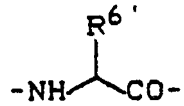

stehen
worin
$R^{6'}$ die oben angegebene Bedeutung von $R^6$ hat und mit dieser gleich oder verschieden ist in ihrer L-Form, D-Form oder als D,L-Isomerengemisch
X - für Hydroxy, Benzyloxy oder Alkoxy mit bis zu 8 Kohlenstoffatomen oder Morpholino steht oder
- für eine Gruppe der Formel $-NHR^8$ steht
worin
$R^8$ - Wasserstoff oder
- geradkettiges oder verzweigtes Alkyl mit bis zu 10 Kohlenstoffatomen bedeutet, das gegebe nenfalls durch Hydroxy, Phenyl, Pyridyl oder durch den Rest der Formel

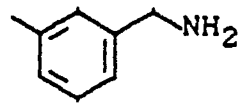

substituiert ist
oder
- für einen Rest der Formel

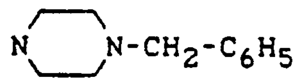

steht
und deren physiologisch unbedenklichen Salze.

Die erfindungsgemäßen Verbindungen der allgemeinen Formel (I), haben mehrere asymmetrische Kohlenstoffatome. Sie können unabhängig voneinander in der D- oder der L-Form vorliegen. Die Erfindung umfaßt die optischen Antipoden ebenso wie die Isomerengemische oder Racemate. Bevorzugt liegen die Aminosäurereste unabhängig voneinander in der optisch reinen, bevorzugt in der L-Form vor.

Die erfindungsgemäßen Verbindungen der allgemeinen Formel (I) können in Form ihrer Salze vorliegen. Dies können Salze der erfindungsgemäßen Verbindungen mit anorganischen oder organischen Säuren oder Basen sein. Zu den Säureadditionsprodukten gehören bevorzugt Salze mit Salzsäure, Bromwasserstoffsäure, Iodwasserstoffsäure, Schwefelsäure, Phosphorsäure oder mit Carbonsäuren wie Essigsäure, Propionsäure, Oxalsäure, Glykolsäure, Bernsteinsäure, Maleinsäure, Hydroxymaleinsäure, Methylma leinsäure, Fumarsäure, Adipinsäure, Äpfelsäure, Weinsäure, Zitronensäure, Benzoesäure, Zimtsäure, Milchsäure, Ascorbinsäure, Salicylsäure, 2-Acetoxybenzoesäure. Nicotinsäure, Isonicotinsäure, oder Sulfonsäuren wie Methansulfonsäure, Ethansulfonsäure, Benzolsulfonsäure, Toluolsulfonsäure, Naphthalin-2-sulfonsäure oder Naphthalindisulfonsäure.

Bevorzugte Verbindungen der allgemeinen Formel (I) sind solche, in denen
$R^1$ - für Wasserstoff oder
- für Alkoxycarbonyl mit bis zu 6 Kohlenstoffatomen oder Benzyloxycarbonyl steht oder
- für eine Gruppe der Formel $R^3$-CO- steht
worin
$R^3$ - Morpholino oder die Reste

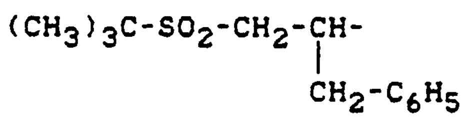

oder 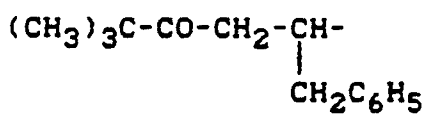

bedeutet
oder
- eine Gruppe der Formel $-NR^4R^5$ bedeutet
worin
$R^4$ und $R^5$ gleich oder verschieden sind und Wasserstoff, geradkettiges oder verzweigtes Alkyl mit bis zu 6 Kohlenstoffatomen oder Phenyl bedeuten
oder
- den Rest

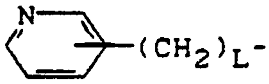

bedeutet
worin
L - die Zahl 0, 1 oder 2 bedeutet
B - für eine direkte Bindung oder
- für eine Aminosäuregruppierung der Formel

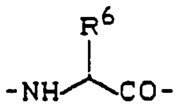

steht
worin
$R^6$ - geradkettiges oder verzweigtes Alkyl mit bis zu 6 Kohlenstoffatomen bedeutet, das gegebenenfalls durch Hydroxy, Alkoxy mit bis zu 4 Kohlenstoffatomen oder Benzyloxy substituiert ist
oder
- für Prolin steht
in ihrer L-Form, D-Form oder als D,L-Isomerengemisch
$R^2$ - für Phenyl oder Naphthyl steht, das gegebenenfalls bis zu 3-fach gleich oder verschieden durch Fluor, Chlor, Brom, Jod, Hydroxy, Nitro, Cyano, geradkettiges oder verzweigtes Alkoxy mit bis zu 6 Kohlenstoffatomen, Acetoxy oder Benzyloxy substituiert ist
w - eine Zahl 0 oder 1 bedeutet
A - für eine $-CH_2-$ oder für eine $-CH_2-CH_2-$Gruppe steht,
D und E gleich oder verschieden sind und
- für eine direkte Bindung oder
- für eine Aminosäuregruppierung der Formel

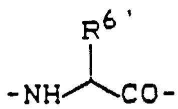

stehen
worin
$R^{6'}$ die oben angegebene Bedeutung von $R^6$ hat und mit dieser gleich oder verschieden ist, in ihrer L-Form, D-Form oder als D,L-Isomerengemisch
X - für Hydroxy, Benzyloxy oder Alkoxy mit bis zu 6 Kohlenstoffatomen oder Morpholino steht oder
- für eine Gruppe der Formel $-NHR^8$ steht
worin
$R^8$ - Wasserstoff oder geradkettiges oder verzweigtes Alkyl mit bis zu 8 Kohlenstoffatomen bedeutet, das gegebe nenfalls durch Hydroxy, Phenyl, Pyridyl oder durch den Rest der Formel

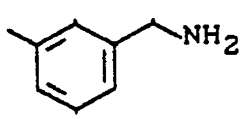

substituiert ist
oder
- für einen Rest der Formel

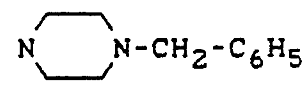

steht
und deren physiologisch unbedenklichen Salze.

Besonders bevorzugt sind Verbindungen der allgemeinen Formel (I),
in welcher
$R^1$ - für Wasserstoff oder
- für Alkoxycarbonyl mit bis zu 4 Kohlenstoffatomen oder Benzyloxycarbonyl steht
- für eine Gruppe der Formel $R^3$-CO- steht
worin
$R^3$ - Morpholino oder eine Gruppe der Formel

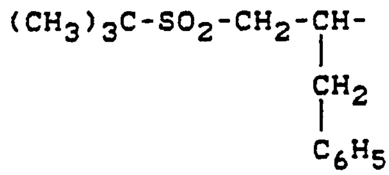

oder $-NR^4R^5$ bedeutet
worin
$R^4$ und $R^5$ gleich oder verschieden sind und Wasserstoff oder geradkettiges oder verzweigtes Alkyl mit bis zu 4 Kohlenstoffatomen oder Phenyl bedeuten
oder
- den Rest

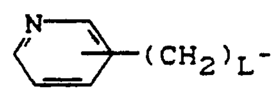

bedeutet
worin
L - die Zahl 0, 1 oder 2 bedeutet
B - für eine direkte Bindung oder
- für eine Aminosäuregruppierung der Formel

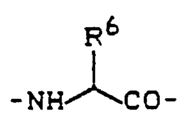

steht,
worin
$R^6$ - geradkettiges oder verzweigtes Alkyl mit bis zu 4 Kohlenstoffatomen bedeutet, das gegebenenfalls durch Hydroxy substituiert ist
oder
- für Prolin steht
in ihrer L-Form, D-Form oder als D,L-Isomerengemisch
$R^2$ - für Phenyl oder Naphthyl steht, das gegebenenfalls bis zu 3-fach gleich oder verschiden durch Fluor, Chlor, Jod, Hydroxy, Methoxy, Ethoxy, Propoxy, tert. Butoxy, Benzyloxy oder Acetoxy substituiert ist,
w - eine Zahl 0 oder 1 bedeutet
A - für eine $-CH_2-$ oder für eine $-CH_2-CH_2-$Gruppe steht,
D und E gleich oder verschieden sind und
- für eine direkte Bindung oder

- für eine Aminosäuregruppierung der Formel

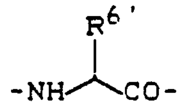

stehen
worin
$R^{6'}$ die oben angegebene Bedeutung von $R^6$ hat und mit dieser gleich oder verschieden ist, in ihrer L-Form, D-Form oder als D,L-Isomerengemisch
X - für Hydroxy, Benzyloxy oder Alkoxy mit bis zu 4 Kohlenstoffatomen oder Morpholino steht oder
- für eine Gruppe der Formel $-NHR^8$ steht
worin
$R^8$ - Wasserstoff oder
- geradkettiges oder verzweigtes Alkyl mit bis zu 6 Kohlenstoffatomen bedeutet, das gegebe nenfalls durch Hydroxy, Phenyl, Pyridyl oder durch den Rest der Formel

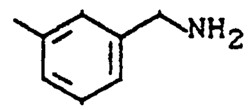

substituiert ist
oder
- für einen Rest der Formel

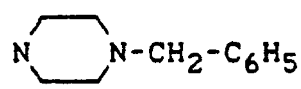

steht
und deren physiologisch unbedenklichen Salze.

Salze der erfindungsgemäßen Verbindungen mit salzbildenden Gruppen können in an sich bekannter Weise hergestellt werden, zum Beispiel durch Umsetzung der erfindungsgemäßen Verbindungen, die saure Gruppen enthalten mit entsprechenden Basen oder durch Umsetzung der erfindungsgemäßen Verbindungen, die basische Gruppen enthalten mit entsprechenden Säuren, jeweils bevorzugt mit den oben aufgeführten Basen bzw. Säuren.

Stereoisomerengemische, insbesondere Diastereomerengemische, können in an sich bekannter Weise, z.B. durch fraktionierte Kristallisation oder Chromatographie in die einzelnen Isomere getrennt werden.

Racemate können in an sich bekannter Weise, z.B. durch Überführung der optischen Antipoden in Diastereomere, gespalten werden.

Die Gruppe der Formel

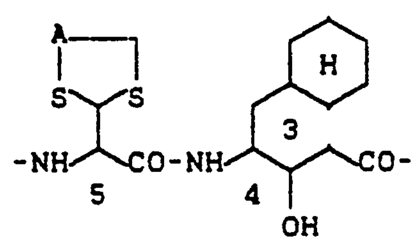

besitzt drei asymmetrische Kohlenstoffatome, die unabhängig voneinander in der R- oder S-Konfiguration vorliegen können. Bevorzugt liegt diese Gruppierung in der 5R, 3S, 4S-Konfiguration, 5R, 3R, 4S-Konfiguration, 5S, 3S, 4S-Konfiguration oder 5S, 3R, 4S-Konfiguration vor, besonders bevorzugt in der 5R,

3S, 4S- oder 5S, 3S, 4S-Konfiguration vor. Ebenso wird die Gruppierung als Isomerengemisch eingesetzt.

Die erfindungsgemäßen Verbindungen der allgemeinen Formel (I)

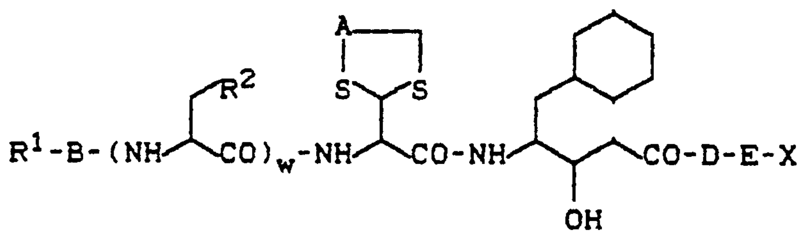

(I),

worin

B, D, E, A, $R^1$, $R^2$, w und X die oben angegebene Bedeutung haben,

erhält man, indem man

[A] Verbindungen der allgemeinen Formel (II)

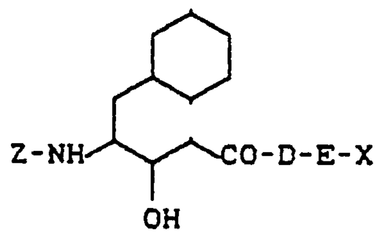

(II),

in welcher

D, E und X die oben angegebene Bedeutung haben

und

Z - für eine Aminoschutzgruppe steht,

zunächst durch Abspaltung die Gruppe Z nach üblichen Methoden in Amine überführt und diese anschließend mit Verbindungen der allgemeinen Formel (III)

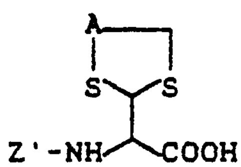

(III),

in welcher

A die oben angegebene Bedeutung hat,

und

Z′ die oben angegebene Bedeutung von Z hat und mit dieser gleich oder verschieden ist,

unter Aktivierung der Carbonsäure nach üblichen Methoden in inerten Lösemitteln zu Verbindungen der allgemeinen Formel (Ia)

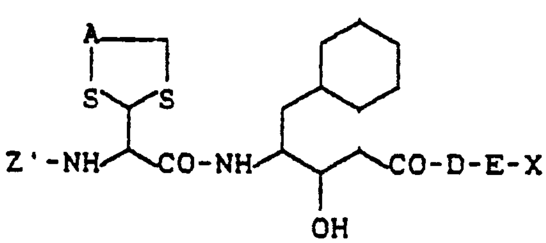

(Ia),

in welcher

Z', A, D, E und X die oben angegebene Bedeutung haben,
umsetzt, die Schutzgruppe Z' nach üblicher Methode abspaltet und in einem weiteren Schritt mit Verbindungen der allgemeinen Formel (IV)

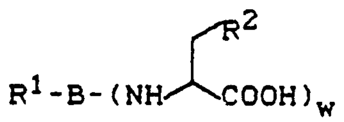 (IV),

in welcher
$R^1$, B, w und $R^2$ die oben angegebene Bedeutung haben,
gegebenenfalls unter der oben erwähnten Carbonsäureaktivierung kondensiert,
und gegebenenfalls die entsprechenden Ester nach üblicher Methode verseift, oder zunächst die Verbindungen der allgemeinen Formeln (III) und (IV) nach der oben angegebenen Methode umsetzt und anschließend mit den Verbindungen der allgemeinen Formel (II) eine weitere Peptidknüpfung anschließt
oder indem man
[B] Verbindungen der allgemeinen Formel (Ib)

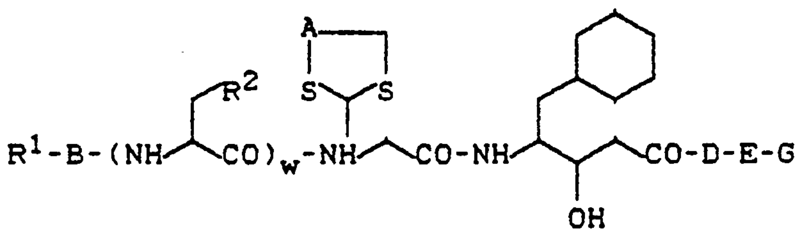 (Ib),

in welcher
A, B, $R^1$, $R^2$, w, D und E die oben angegebene Bedeutung haben,
und
G - für Alkoxycarbonyl mit bis zu 8 Kohlenstoffatomen steht,
nach üblicher Methode zunächst zu den entsprechenden Säuren verseift und in einem weiteren Schritt in Anwesenheit von Hilfsstoffen mit Aminen der allgemeinen Formel (V), oder Morpholin der Formel (VI) oder N-Benzylpiperazin der Formel (VII)
$H_2N\text{-}R^8$ (V),

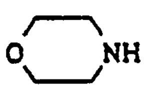 (VI) oder 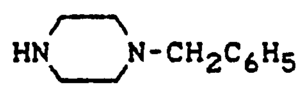 (VII)

in welcher
$R^8$ die oben angegebene Bedeutung hat,
kondensiert,
oder indem man
[C] entweder Verbindungen der allgemeinen Formel (Ic)

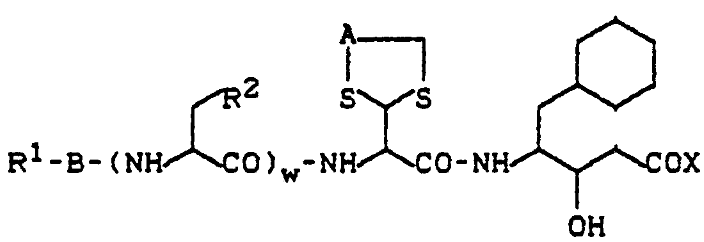 (Ic),

in welcher

A, B, $R^1$, w und $R^2$ die oben angegebene Bedeutung haben,
und
X′ - für Alkoxy mit bis zu 6 Kohlenstoffatomen oder Benzyloxy steht,
oder Verbindungen der allgemeinen Formel (Id)

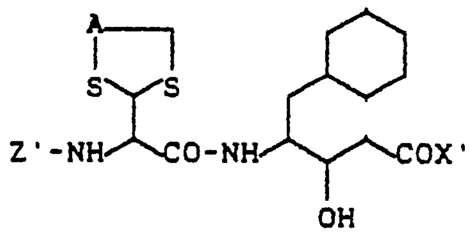

(Id),

in welcher
Z′, A und X′ die oben angegebene Bedeutung haben,
zunächst zu den entsprechenden Säuren nach üblicher Methode verseift und anschließend mit dem Bruchstück der allgemeinen Formel (VIII)

D-E-X (VIII),

in welcher
D, E und X die oben angegebene Bedeutung haben,
in inerten Lösemitteln, gegebenenfalls in Anwesenheit von Hilfsstoffen, umsetzt,
und im Fall der Verbindungen der allgemeinen Formel (Id) in einem nächsten Schritt nach der unter Verfahren [A] beschriebenen Methode unter schrittweiser Abspaltung der jeweiligen Schutzgruppe Z′ mit Verbindungen der allgemeinen Formel (IV) oder (IVa)

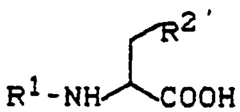 (IV) oder 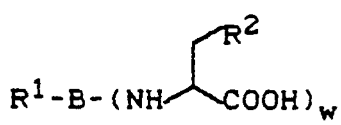 (IVa)

in welcher
$R^1$, B, w und $R^2$ die oben angegebene Bedeutung haben,
umsetzt.

Das Syntheseschema kann durch folgendes Reaktionsschema beispielhaft belegt werden:

[A]

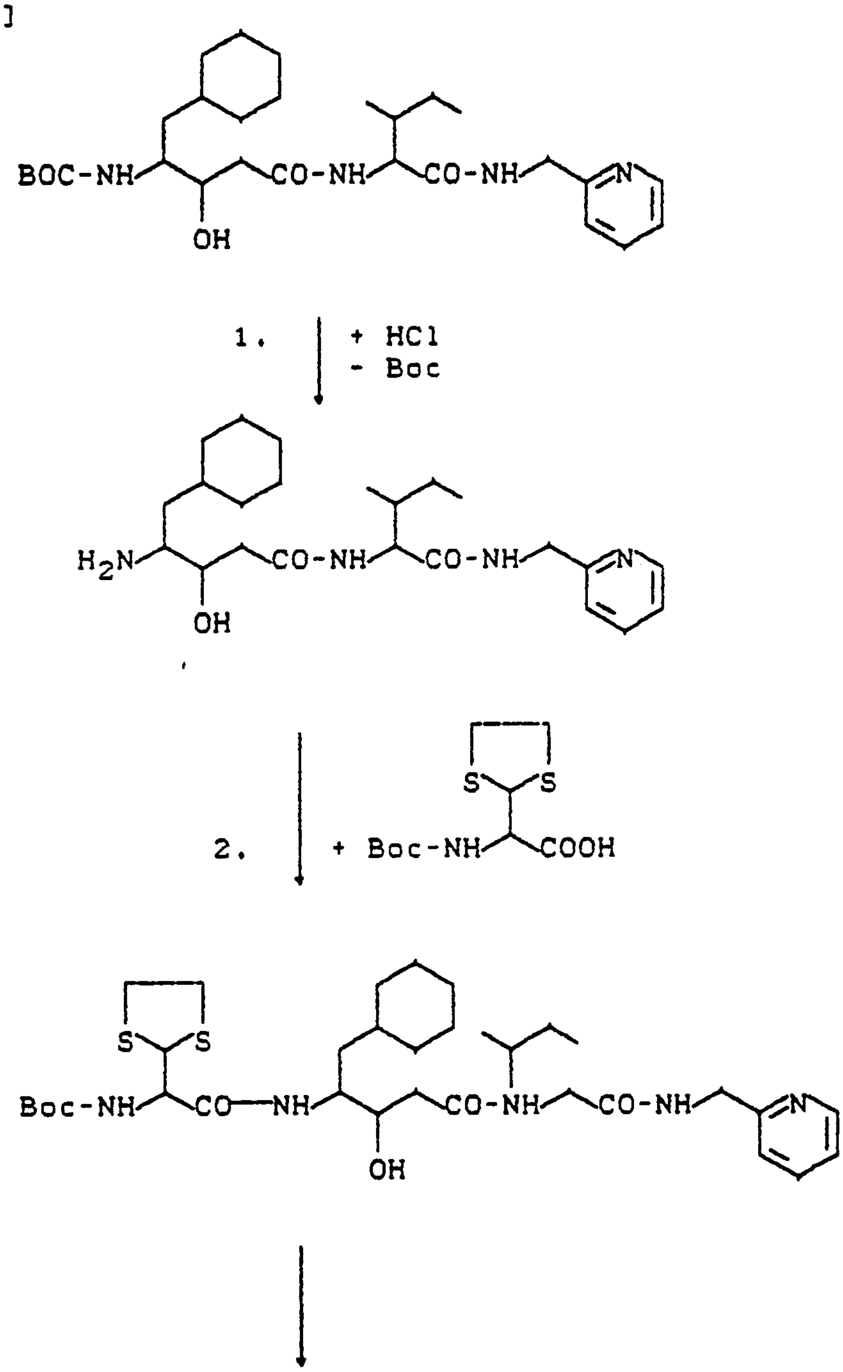

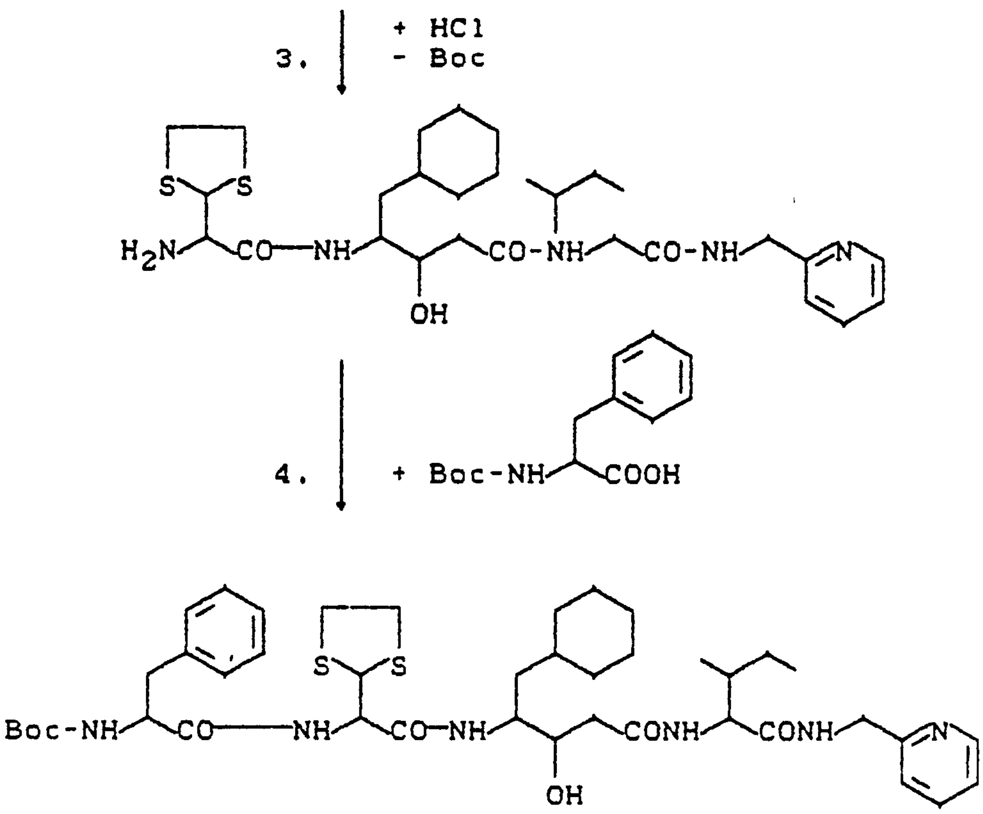


[A]

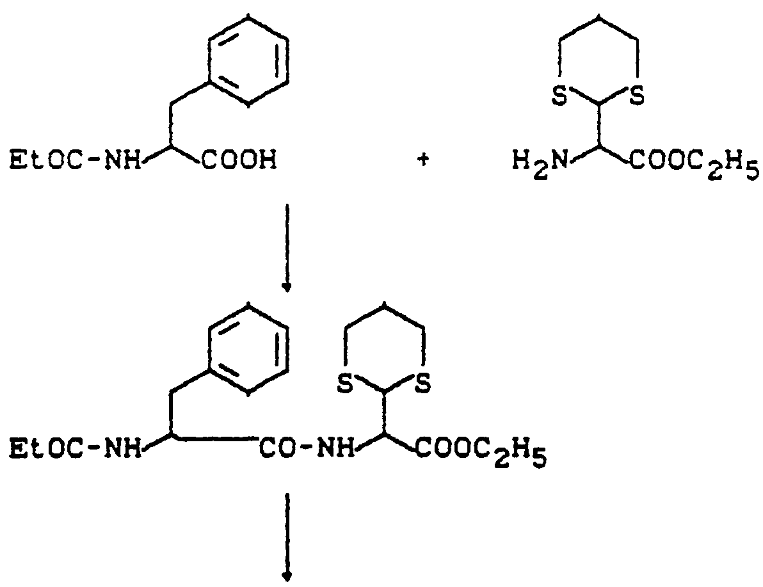

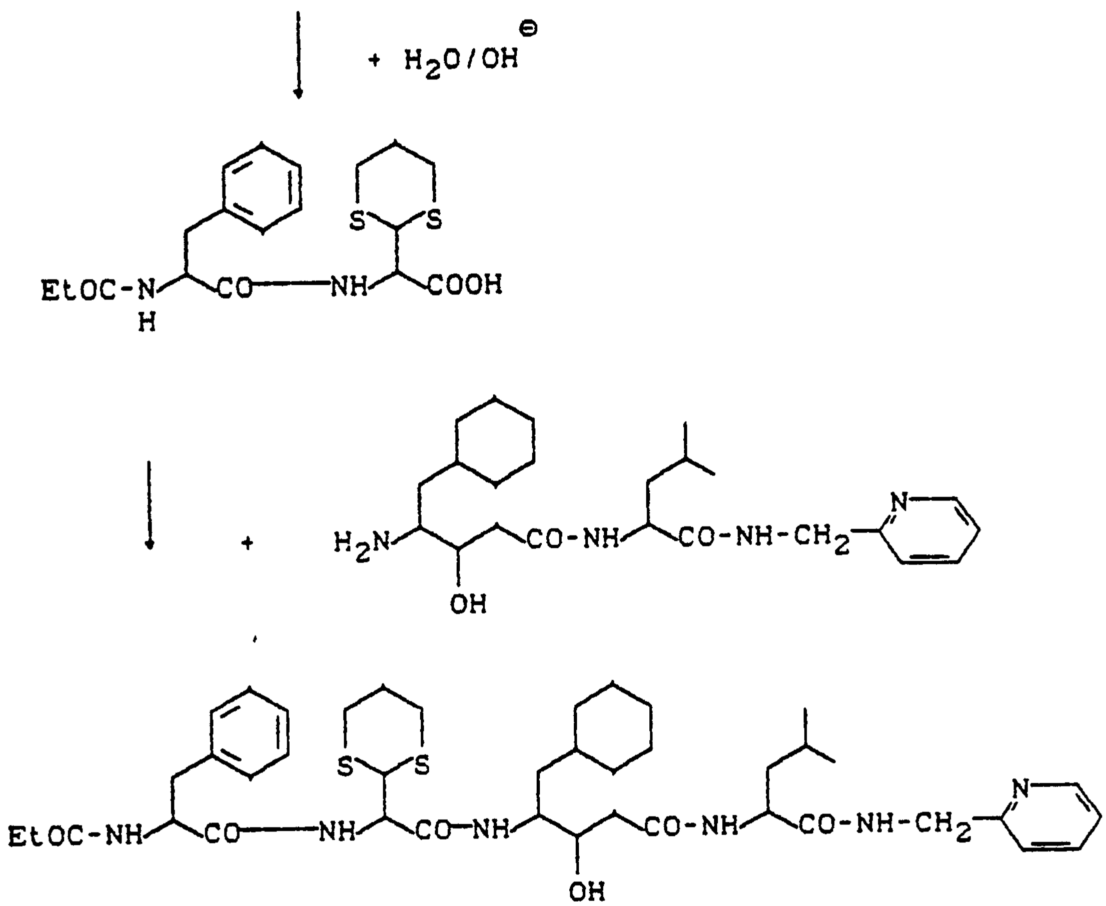


[B]

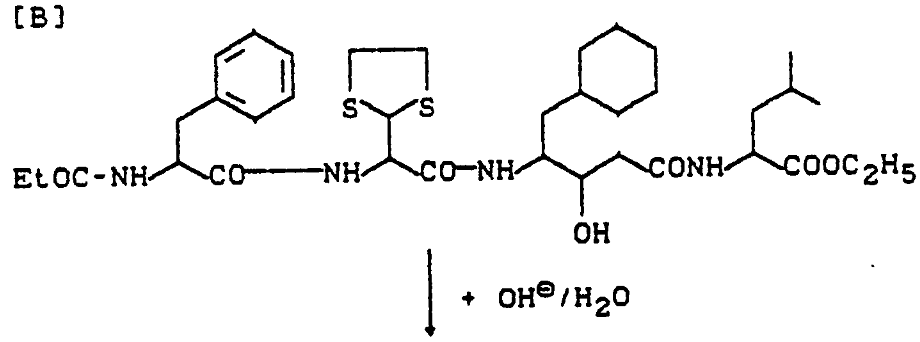

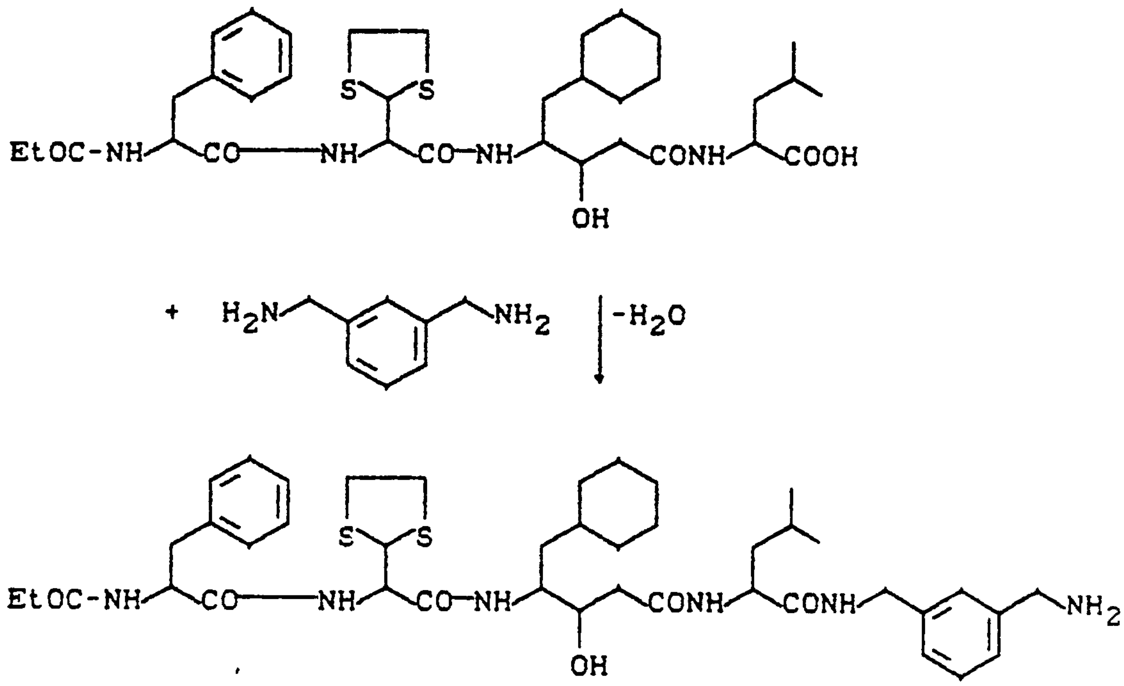


[C]

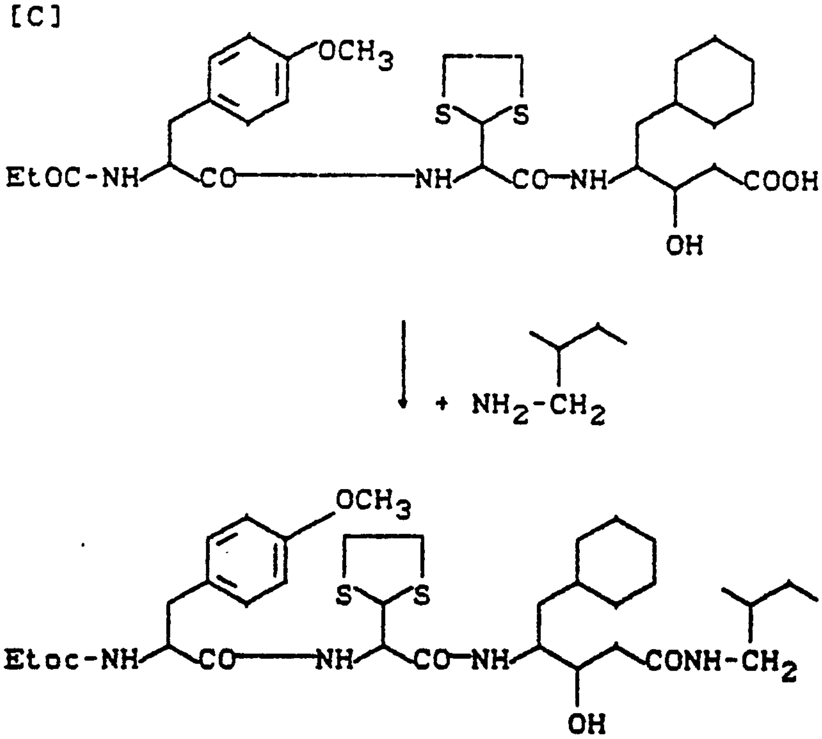

Die Herstellung der erfindungsgemäßen Verbindungen ist auch nach weiteren üblichen Varianten des beschriebenen Verfahrens durchgeführt worden (vgl. z.B. Houben-Weyls "Methoden der organischen Chemie" XV/1 und 2; M. Bodanszky, A. Bodanszky in "The Practice of Peptide Synthesis", Springer Verlag, Berlin, 1984; George R. Pettit in "Synthetic Peptides", Volume 4, Elsevier Scientific Publishing Company, Amsterdam-Oxford-New York, 1976; E. Gross und J. Meienhofer (Editors) in "The Peptides", Vol. 1-3, Academic Press, New York-London-Toronto-Sydney-San Francisco, 1981; M. Bodanszky in "Principles of Peptide Syntheseis", Springer Verlag, Berlin-Heidelberg-New York-Tokyo, 1984; R. Uhmann und K. Radscheit, Offenlegungsschrift, DE 3 411 244 A1) oder auch nach der "Solid-Phase-Methode", wie sie beispielsweise von M. Bodanszky, A. Bodanszky in "The Practice of Peptide Synthesis", Springer-Verlag, Berlin, 1984, oder G. Barany, R.B. Merrifield in "Solid-Phase Peptide Synthesis" aus "The Peptides", Vol 2, S. 3-254, edited by E. Gross, J. Meienhofer, Academic Press, New York-London-Toronto-Sydney-San

Francisco (1980) beschrieben wird.

Als Lösemittel eignen sich bei den Verfahrensvarianten [A], [B] und [C] die üblichen inerten Lösemittel, die sich unter den jeweils gewählten Reaktionsbedingungen nicht verändern. Hierzu gehören Wasser oder organische Lösemittel wie Diethylether, Glykolmono- oder -dimethylether, Dioxan oder Tetrahydrofuran, oder Kohlenwasserstoffe wie Benzol, Toluol, Xylol, Cyclohexan oder Erdölfraktionen oder Halogenkohlenwasserstoffe wie Methylenchlorid, Chloroform, Tetrachlorkohlenwasserstoffe oder Aceton, Dimethylsulfoxid, Dimethylformamid, Hexamethylphosphorsäuretriamid, Essigester, Pyridin, Triethylamin oder Picolin.
Ebenso ist es möglich, Gemische der genannten Lösemittel zu verwenden.
Bevorzugt sind Tetrahydrofuran, Methylenchlorid, Dimethylformamid und Essigester.

Üblicherweise werden die Verfahrensvarianten [A], [B] und [C] in Gegenwart geeigneter Löse- bzw. Verdünnungsmittel, gegebenenfalls in Anwesenheit eines Hilfsstoffes oder Katalysators in einem Temperaturbereich von -80 °C bis 300 °C, bevorzugt von -30 °C bis +30 °C bei normalem Druck durchgeführt. Ebenso ist es möglich, bei erhöhtem oder erniedrigtem Druck zu arbeiten.

Als aktivierte Carboxylgruppe eignen sich bei den Verfahrensvarianten [A], [B] und [C] beispielsweise Carbonsäureazide (erhältlich z.B. durch Umsetzung von geschützten oder ungeschützten Carbonsäurehydraziden mit salpetriger Säure, deren Salzen oder Alkylnitriten (z.B. Isoamylnitrit),
oder ungesättigte Ester, insbesondere Vinylester, (erhältlich z.B. durch Umsetzung eines entsprechenden Esters mit Vinylacetat), Carbamoylvinylester (erhältlich z.B. durch Umsetzung einer entsprechenden Säure mit einem Isoxazoliumreagenz), Alkoxyvinylester (erhältlich z.B. durch Umsetzung der entsprechenden Säuren mit Alkoxyacetylenen, bevorzugt Ethoxyacetylen),
oder Amidinoester z.B. N,N′- bzw. N,N-disubstituierte Amidinoester (erhältlich z.B. durch Umsetzung der ent sprechenden Säure mit einem N,N′-disubsituierten Carbodiimid (bevorzugt Dicyclohexylcarbodiimid, Diisopropylcarbodiimid oder N-(3-Dimethylaminopropyl)-N′-ethylcarbodiimidhydrochlorid) oder mit einem N,N-disubstituierten Cyanamid,
oder Arylester, insbesondere durch elektronenziehende Substituenten substituierte Phenylester, z.B. 4-Nitrophenyl-, 4-Methylsulfonylphenyl-, 2,4,5-Trichlorphenyl-, 2,3,4,5,6-Pentachlorphenyl-, 4-Phenyldiazophenylester (erhältlich z.B. durch Umsetzung der entsprechenden Säure mit einem entsprechend substituierten Phenol, gegebenenfalls in Anwesenheit eines Kondensationsmittels wie z.B. N,N′-Dicyclohexylcarbodiimid, Diisopropylcarbodiimid, N-(3-Dimethylaminopropyl)-N′-ethylcarbodiimid-Hydrochlorid, Isobutylchloroformat, Propanphosphonsäureanhydrid, Benzotriazolyloxytris(dimethylamino)phosphoniumhexafluorphosphat
oder Cyanmethylester (erhältlich z.B. durch Umsetzung der entsprechenden Säure mit Chloracetonitril in Gegenwart einer Base),
oder Thioester, insbesondere Nitrophenylthioester (erhältlich z.B. durch Umsetzung der entsprechenden Säure mit Nitrothiophenolen, gegebenenfalls in Gegenwart von Kondensationsmitteln wie N,N′-Dicyclohexylcarbodiimid, Diisopropylcarbodiimid, N-(3-Dimethylaminopropyl)-N′-ethylcarbodiimid-Hydrochlorid, Isobutylchloroformat, Propanphosphonsäureanhydrid, Benzotriazolyloxytris (dimethylamino)-phosphoniumhexafluorphosphat),
oder Amino- bzw. Amidoester (erhältlich z.B. durch Umsetzung der entsprechenden Säure mit einer N-Hydroxyamino- bzw. N-Hydroxyamido-Verbindung, insbesondere N- Hydroxy-succinimid, N-Hydroxypiperidin, N-Hydroxy-phthalimid, N-Hydroxy-5-norbornen-2,3-dicarbonsäureimid oder 1-Hydroxybenzotriazol, gegebenenfalls in Anwesenheit von Kondensationsmitteln wie N,N′-Dicyclohexylcarbodiimid, Diisopropylcarbodiimid oder N-(3-Dimethylaminopropyl)-N′-ethylcarbodiimid-Hydrochlorid, Isobutylchloroformat oder n-Propanphosphonsäureanhydrid),
oder Anhydride von Säuren, bevorzugt symmetrische oder unsymmetrische Anhydride der entsprechenden Säuren, insbesondere Anhydride mit anorganischen Säuren (erhältlich z.B. durch Umsetzung der entsprechenden Säure mit Thionylchlorid, Phosphorpentoxid oder Oxalylchlorid),
oder Anhydride mit Kohlensäurehalbderivaten z.B. Kohlensäureniederalkylhalbester (erhältlich z.B. durch Umsetzung der entsprechenden Säure mit Halogenameisensäureniedrigalkylestern, z.B. Chlorameisensäuremethylester, -ethylester, -propylester, -isopropylester, -butylester oder -isobutylester oder mit 1-Niedrigalkoxycarbonyl-2-niedrigalkoxy-1,2-dihydrochinolin, z.B. 1-Methoxycarbonyl-2-ethoxy-1,2-dihydrochinolin),
oder Anhydride mit Dihalogenphosphorsäuren (erhältlich z.B. durch Umsetzung der entsprechenden Säure mit Phosphoroxychlorid),
oder Anhydride mit Phosphorsäurederivaten oder Phosphorigsäurederivaten, (z.B. Propanphosphonsäureanhydrid, H. Wissmann und H.J Kleiner, Angew. Chem. Int. Ed. 19, 133 (1980))
oder Anhydride mit organischen Carbonsäuren (erhältlich z.B. durch Umsetzung der entsprechenden Säuren mit einem gegebenenfalls substituierten Niederalkan- oder Phenylalkancarbonsäurehalogenid, insbesondere Phenyl essigsäure-, Pivalinsäure- oder Trifluoressigsäurechlorid),

oder Anhydride mit organischen Sulfonsäuren (erhältlich z.B. durch Umsetzung eines Alkalisalzes einer entsprechenden Säure mit einem Sulfonsäurehalogenid, insbesondere Methan-, Ethan-, Benzol- oder Toluolsulfonsäurechlorid),
oder symmetrische Anhydride (erhältlich z.B. durch Kondensation entsprechender Säuren, gegebenenfalls in Gegenwart von Kondensationsmitteln wie N,N'-Dicyclohexylcarbodiimid, Diisopropylcarbodiimid, N-(3-Dimethylaminopropyl)-N'-ethylcarbodiimid-Hydrochlorid Isobutylchloroformat, Propanphosphonsäureanhydrid oder Benzotriazolyloxy-tris(dimethylamino)phosphoniumhexafluorphosphat).

Reaktionsfähige cyclische Amide sind insbesondere Amide mit fünfgliedrigen Heterocyclen mit 2 Stickstoffatomen und gegebenenfalls aromatischem Charakter, bevorzugt Amide mit Imidazolen oder Pyrazolen (erhältlich z.B. durch Umsetzung der entsprechenden Säuren mit N,N'-Carbonyldiimidazol oder - gegebenenfalls in Gegenwart von Kondensationsmitteln wie z.B. N,N'-Dicyclohexylcarbodiimid, N,N'-Diisopropylcarbodiimid, N-(3-Dimethylaminopropyl)-N'-ethylcarbodiimid-Hydrochlorid, Isobutylchloroformat, Propanphosphonsäureanhydrid, Benzotriazolyloxy-tris(dimethylamino)phosphonium-hexafluorphosphat- mit z.B. 3,5-Dimethyl-pyrazol, 1,2,4-Triazol oder Tetrazol.

Als Aminoschutzgruppe eignen sich die in der Peptidchemie gebräuchlichen Aminoschutzgruppen.

Hierzu gehören bevorzugt: Benzyloxycarbonyl, 4-Brombenzyloxycarbonyl, 2-Chlorbenzyloxycarbonyl, 3-Chlorbenzyloxycarbonyl, Dichlorbenzyloxycarbonyl,3,4-Dimethoxybenzyloxycarbonyl, 3,5-Dimethoxybenzyloxycarbonyl, 2,4-Dimethoxybenzyloxycarbonyl, 4-Methoxybenzyloxycarbonyl, 4-Nitrobenzyloxycarbonyl, 2-Nitrobenzyloxycarbonyl, 2-Nitro-4,5-dimethoxybenzyloxycarbonyl, 3,4,5-Trimethoxybenzyloxycarbonyl, Methoxycarbonyl, Ethoxycarbonyl, Propoxycarbonyl, Isopropoxycarbonyl, Butoxycarbonyl, Isobutoxycarbonyl, tert-Butoxycarbonyl, Pentoxycarbonyl, Isopentoxycarbonyl, Hexoxycarbonyl, Cyclohexoxycarbonyl, Octoxycarbonyl, 2-Ethylhexoxycarbonyl, 2-Iodhexoxycarbonyl, 2-Bromethoxycarbonyl, 2-Chlorethoxycarbonyl, 2,2,2-Trichlorethoxycarbonyl, 2,2,2-Trichlor-tert-butoxycarbonyl, Benzhydryloxycarbonyl, Bis-(4-methoxyphenyl)methoxycarbonyl, Phenacyloxycarbonyl, 2-Trimethylsilylethoxycarbonyl, 2-(Di-n-butyl-methylsilyl)-ethoxycarbonyl, 2-Triphenylsilylethoxycarbonyl, 2-(Dimethyl-tert-butylsilyl)ethoxycarbonyl, Menthyloxycarbonyl, Vinyloxycarbonyl, Allyloxycarbonyl, Phenoxycarbonyl, Tolyloxycarbonyl, 2,4-Dinitrophenoxycarbonyl, 4-Nitrophenoxycarbonyl, 2,4,5-Trichlorphenoxycarbonyl, Naphthyloxycarbonyl, Fluorenyl-9-methoxycarbonyl, Ethylthiocarbonyl, Methylthiocarbonyl, Butylthiocarbonyl, Tert.-Butylthiocarbonyl, Phenylthiocarbonyl, Benzylthiocarbonyl, Methylaminocarbonyl, Ethylaminocarbonyl, Methylthiocarbonyl, Butylthiocarbonyl, tert-Butylthiocarbonyl, Phenylthiocarbonyl, Benzylthiocarbonyl, Methylaminocarbonyl, Ethylaminocarbonyl, Propylaminocarbonyl, iso-Propylaminocarbonyl, Formyl, Acetyl, Propionyl, Pivaloyl, 2-Chloracetyl, 2-Bromacetyl, 2-Iodacetyl, 2,2,2-Trifluoracetyl, 2,2,2-Trichloracetyl, Benzoyl, 4-Chlorbenzoyl, 4-Methoxybenzoyl, 4-Nitrobenzyl, 4-Nitrobenzoyl, Naphthylcarbonyl, Phenoxyacetyl, Adamantylcarbonyl, Dicyclohexylphosphoryl, Diphenylphosphoryl, Dibenzylphosphoryl, Di-(4-nitrobenzyl)phosphoryl, Phenoxyphenylphosphoryl, Diethylphosphinyl, Diphenylphosphinyl, Phthaloyl oder Phthalimido.

Besonders bevorzugte Aminoschutzgruppen sind Benzyloxycarbonyl, 3,4-Dimethoxybenzyloxycarbonyl, 3,5-Dimethoxybenzyloxycarbonyl, 4-Methoxybenzyloxycarbonyl, 4-Nitrobenzyloxycarbonyl, 2-Nitrobenzyloxycarbonyl, 3,4,5-Trimethoxybenzyloxycarbonyl, Methoxycarbonyl, Ethoxycarbonyl, Propoxycarbonyl, Isopropoxycarbonyl, Butoxycarbonyl, Isobutoxycarbonyl, tert-Butoxycarbonyl, Cyclohexoxycarbonyl, Hexoxycarbonyl, Octoxycarbonyl, 2-Bromethoxycarbonyl, 2-Chlorethoxycarbonyl, Phenoxyacetyl, Naphthylcarbonyl, Adamatylcarbonyl, Phthaloyl, 2,2,2-Trichlorethoxycarbonyl, 2,2,2-Trichlor-tert-butoxycarbonyl, Menthyloxycarbonyl, Vinyloxycarbonyl, Allyloxycarbonyl, Phenoxycarbonyl, 4-Nitrophenoxycarbonyl, Fluorenyl-9-methoxycarbonyl, Formyl, Acetyl, Propionyl, Pivaloyl, 2-Chloracetyl, 2-Bromacetyl, 2,2,2-Trifluoracetyl, 2,2,2-Trichloracetyl, Benzoyl, 4-Chlorbenzoyl, 4-Brombenzoyl, 4-Nitrobenzoyl, Phthalimido oder Isovaleroyl [vgl. Th. W. Greene Protective Groups in Organic Synthesi, John Wiley & Sons, New York 1981].

Als Hilfsstoffe werden bevorzugt Kondensationsmittel eingesetzt, die auch Basen sein können, insbesondere wenn die Carboxylgruppe als Anhydrid aktiviert vorliegt.

Bevorzugt werden hier die üblichen Kondensationsmittel wie Carbodiimide z.B N,N'-Diethyl-, N,N'-Dipropyl-, N,N'-Diisopropyl-, N,N'-Dicyclohexylcarbodiimid, N-(3-Dimethylaminoisopropyl)-N'-ethylcarbodiimid-Hydrochlorid, oder Carbonylverbindungen wie Carbonyldiimidazol, oder 1,2-Oxazoliumverbindungen wie 2-Ethyl-5-phenyl-1,2-oxazolium-3-sulfat oder 2-tert-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Benzotriazolyloxy-tris(dimethyl amino)phosphonium-hexafluorophosphat, und als Basen Alkalicarbonate z.B. Natrium- oder Kaliumcarbonat oder -hydrogencarbonat, oder organische Basen wie Trialkylamine z.B. Triethylamin, N-Ethylmorpholin, N-Methylpiperidin oder Diisopropylethylamin eingesetzt.

Die Abspaltung der Aminoschutzgruppe erfolgt in an sich bekannter Weise unter sauren oder basischen Bedingungen, oder reduktiv durch katalytische Hydrierung beispielsweise mit Pd/C in organischen Lösemit-

teln wie Ethern, z.B. Tetrahydrofuran oder Dioxan, oder Alkoholen z.B. Methanol, Ethanol oder Isopropanol.

Die Verseifung der Carbonsäureester erfolgt nach üblichen Methoden in einem der oben aufgeführten Lösemittel, indem man die Ester mit üblichen Basen behandelt, wobei die zunächst entstehenden Salze durch Behandeln mit Säure in die freien Carbonsäuren überführt werden können.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriumethanolat, Natriummethanolat, Kaliumethanolat, Kaliummethanolat oder Kalium-tert.butanolat. Besonders bevorzugt werden Natriumhydroxid oder Kaliumhydroxid eingesetzt.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters eingesetzt. Besonders bevorzugt verwendet man molare Mengen der Reaktanden.

Die Abspaltung der Ester kann ebenfalls nach üblicher Methode mit Säuren, wie beispielsweise Chlorwasserstoffsäure oder Trifluoressigsäure im Fall der tert.-Butylester oder durch Hydrogenolyse im Fall von Benzylestern erfolgen.

Die Verbindungen der allgemeinen Formeln (II), (IV), (IVa) und (VIII) sind an sich bekannt oder können nach üblichen Methoden der Peptidchemie aufgebaut werden, indem man durch Umsetzung eines entsprechenden Bruchstücks bestehend aus einer oder mehreren Aminosäuregruppierungen, mit einer freien, gegebenenfalls in aktivierter Form vorliegenden Carboxylgruppe mit einem komplementierenden Bruchstück, bestehend aus einer oder mehreren Aminosäuregruppierungen, mit einer Aminogruppe, gegebenenfalls in aktivierter Form, herstellt, und diesen Vorgang gegebenenfalls so oft mit entsprechenden Bruchstükken wiederholt, bis man die gewünschten Peptide der allgemeinen Formeln (II), (IV) und (IVa) hergestellt hat, anschließend gegebenenfalls Schutzgruppen abspaltet oder gegen andere Schutzgruppen austauscht.

Hierbei können zusätzliche reaktive Gruppen, wie z.B. Amino- oder Hydroxygruppen, in den Seitenketten der Bruchstücke gegebenenfalls durch übliche Schutzgruppen geschützt werden [vgl. Houben-Weyl, Eugen Müller, Methoden der organischen Chemie, Band XV/1 und Band XV/2, Georg Thieme Verlag, Stuttgart, 1974].

Die Umsetzung mit Morpholin (VI) und N-Benzylpiperazin (VII) oder mit Aminen der Formel (V) erfolgt im allgemeinen in einem der oben aufgeführten inerten Lösemitteln, in Anwesenheit einer der oben aufgeführten Basen, bevorzugt in Triethylamin und Methylenchlorid bei einer Temperatur von -40°C bis 0°C, bevorzugt bei -20°C und Normaldruck.

Im allgemeinen setzt man 1 bis 5, bevorzugt 1,5 bis 1 mol Amin, bezogen auf 1 mol des Reaktionspartners, ein.

Die Verbindungen der allgemeinen Formel (III)

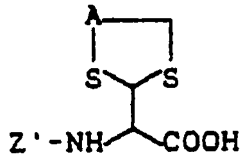 (III),

in welcher
A und Z′ die oben angegebene Bedeutung haben,
sind neu und können hergestellt werden, indem man
Verbindungen der allgemeinen Formel (IX)

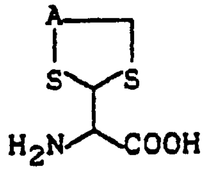 (IX),

in welcher
A die oben angegebene Bedeutung hat,
mit einem einführenden Reagenz, wie beispielsweise (Z'-O-CO)$_2$O, Z'-O-CO-Cl oder (Z'-O-CO)-O-N-succinimid, die Aminoschutzgruppe Z' nach der oben beschriebenen Methode [A] in inerten Lösemitteln in Anwesenheit einer Base, bevorzugt im Gemisch Dioxan/Wasser mit Natriumhydroxid oder in Dioxan mit Triethylamin in einem Temperaturbereich von 0°C bis +50°C, bevorzugt bei Raumtemperatur und Normaldruck, umsetzt.

Die Verbindungen der allgemeinen Formeln (Ia) und (Ib) sind neu und werden nach der unter Verfahren [A] und [B] beschriebenen Methode dargestellt.

Die Verbindungen der allgemeinen Formel (Id) sind ebenfalls neu und können hergestellt werden, indem man
Verbindungen der allgemeinen Formel (III)

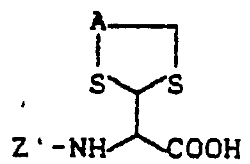
(III),

in welcher
Z' und A die oben angegebene Bedeutung haben,
mit Verbindungen der allgemeinen Formel (X)

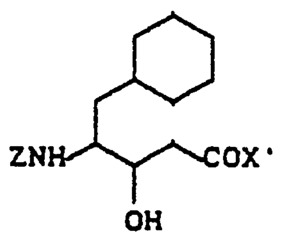
(X),

in welcher
Z und X' die oben angegebene Bedeutung haben,
unter Abspaltung der Schutzgruppe Z, umsetzt.

Die Verbindungen der allgemeinen Formel (X) sind bekannt [vgl. Boger et al., J. Med. Chem. 28, 1779 -1790 (1985)].

Die Verbindungen der allgemeinen Formel (IX) sind bekannt oder können nach bekannten Methoden hergestellt werden [M.P. Mertes, A.A. Ramsey, J. Med. Chem. 12, 342 (1969)].

Die Verbindungen der allgemeinen Formel (Ic) sind ebenfalls neu und werden durch Umsetzung der Verbindungen der Formel (III), (IV) und (X) nach der unter Verfahren [A] beschriebenen üblichen Peptidkupplungsmethode hergestellt.

Die Amine der allgemeinen Formel (V), (VIII) und (IX) sind bekannt [vgl. G.C. Barrett, Chemistry of the Amino Acids, Chapman and Hall, New York, London, 1988 und EP-A2 0278 158].

Morpholin und N-Benzylpiperazin sind bekannt.

Die erfindungsgemäßen Verbindungen besitzen eine kreislaufbeeinflussende Wirkung und können deshalb in Arzneimittel zur Behandlung des Blutdrucks und der Herzinsuffizienz eingesetzt werden.

<u>In vitro Test</u>

Die inhibitorische Stärke der erfindungsgemäßen Peptide gegen endogenes Renin vom Humanplasma wird in vitro be stimmt, Gepooltes Humanplasma wird unter Zusatz von Ethylendiamintetraessigsäure (EDTA) als Antikoagulanz erhalten und bei -20°C gelagert. Die Plasmareninaktivität (PRA) wird als Bildungsrate von Angiotensin I aus endogenem Angiotensinogen und Renin nach Inkubation bei 37°C bestimmt. Die Reaktionslösung enthält 150 µl Plasma, 3 µl 6,6%ige 8-Hydroxychinolinsulfatlösung, 3 µl

10%ige Dimercaprollösung und 144 µl Natriumphosphatpuffer (0,2 M; 0,1% EDTA; pH 5,6) mit oder ohne den erfindungsgemäßen Stoffen in verschiedenen Konzentrationen. Das pro Zeiteinheit gebildete Angiotensin I wird mit einem Radioimmunoassay (Sorin Biomedica, Italien) bestimmt. Die prozentuale Inhibition der Plasmareninaktivität wird berechnet durch Vergleich der hier beanspruchten Substanzen. Der Konzentrationsbereich, in dem die hier beanspruchten Substanzen eine 50% Inhibition der Plasmareninaktivität zeigen, liegen zwischen $10^{-7}$ bis $10^{-10}$ M.

| Beispiel-Nr. | $IC_{50}$ (M) |
|---|---|
| IV | $1,1 \cdot 10^{-7}$ |
| V | $7,0 \cdot 10^{-10}$ |
| XII | $7,4 \cdot 10^{-8}$ |
| XIII | $1,4 \cdot 10^{-10}$ |
| XVI | $2,4 \cdot 10^{-8}$ |
| XVII | $1,9 \cdot 10^{-10}$ |
| XXXII | $1,5 \cdot 10^{-8}$ |
| XXXIII | $2,2 \cdot 10^{-10}$ |

Die neuen Wirkstoffe können in bekannter Weise in die üblichen Formulierungen überführt werden, wie Tabletten, Dragees, Pillen, Granulate, Aerosole, Sirupe, Emulsionen Suspensionen und Lösungen, unter Verwendung inerter, nicht, toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen die ausreichend sind, um den angegebenen, Dosierungsspiel zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Waser als Verdünnungsmittel gegebenenfalls organische Lösungsmittel als Hilfslösungsmittel verwendet werden können.

Als Hilfsstoffe seien beispielsweise aufgeführt:
Wasser, nicht-toxische organische Lösungsmittel, wie Paraffine (z.B. Erdölfraktionen), pflanzliche Öle (z.B. Erdnuß/Sesamöl), Alkohole (z.B: Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester), Polyoxyethylen-Fettalkohol-Ether (z.B. Lignin-Sulfitablaugen, Methylcellulose, Stärke und Polyvinylpyrrolidon) und Gleitmittel (z.B. Magnesiumstearat, Talkum, Stearinsäure und Natriumsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 30 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht des Versuchstieres bzw. der Art der Applikation, aber auch aufgrund der Tierart und deren individuellem Verhalten gegenüber dem Medikament bzw. deren Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

Ausgangsverbindungen

Beispiel 1 (Formel VIII Aminofunktion durch Schutzgruppe Boc geschützt)

N-tert.-Butoxycarbonyl-L-isoleucin-(2-picolyl)amid

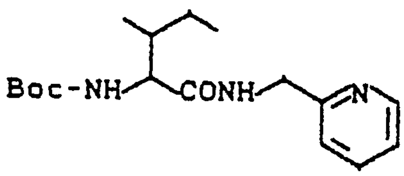

Zu einer Lösung von 100 g (0,432 mol) N-tert.-Butoxycarbonyl-L-Isoleucin und 46,72 g (0,432 mol) 2-Picolylamin in 1 l Methylenchlorid gibt man bei 0°C 419,13 ml (3,024 mol) Triethylamin. Man läßt 10 Minuten rühren, kühlt auf -20°C (Trockeneis/Aceton) und tropft bei dieser Temperatur 365 ml (0,561 mol) einer 50%igen Lösung von Propanphosphonsäureanhydrid in Methylenchlorid (Hoechst AG) hinzu. Der Ansatz wird 1 Stunde bei -20°C weitergerührt und über Nacht auf Raumtemperatur gebracht. Die Reaktionsmischung wird nacheinander 3 mal mit je 300 ml 5%ige Natriumhydrogencarbonatlösung, 3 mal mit je 300 ml Pufferlösung pH 4 (Merck, Art.-Nr. 9435) und schließlich 2 mal mit je 300 ml gesättigter Kochsalzlösung ausgeschüttelt. Die organische Phase wird über Natriumsulfat getrocknet und im Vakuum eingeengt. Das Rohprodukt wird durch Verrühren mit Diethylether kristallisiert, abgesaugt und dann aus Diisopropylether/n-Hexan umkristallisiert.
Ausbeute: 85,9 g (61,8% der Theorie)
DC-System II: $R_f$ = 0,35
DG-System III: $R_f$ = 0,49
DG-System IV: $R_f$ = 0,57

Beispiel 2 (Formel VIII)

L-Isoleucin-(2-picolyl)amid-Dihydrochlorid

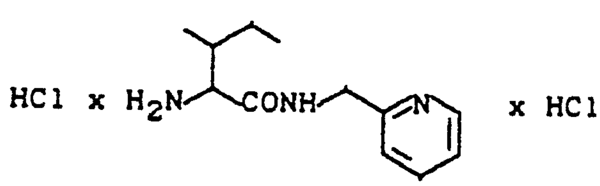

40 g (0,124 mol) der Verbindung aus Beispiel 1 werden mit 250 ml 4 N Salzsäure (Gas) in Dioxan versetzt und unter Eiskühlung gerührt. Die Suspension wird durch Zugabe von 10 ml Methanol in Lösung gebracht. Nach Abreaktion (DC-Kontrolle) engt man am Rotationsverdampfer ein. Nach mehrmaliger Koevaporation mit Diethylether wird das Rohprodukt mit Diethylether verrührt, abgesaugt und im Exsikkator über Kaliumhydroxid getrocknet.
Ausbeute: 37,2 g (100% der Theorie)
DC-System III: $R_f$ = 0,30

Beispiel 3 (Formel II)

N-tert.-Butoxycarbonyl-4S-amino-3S-hydroxy-5-cyclohexylpentanoyl-L-isoleucin-(2-picolyl)amid

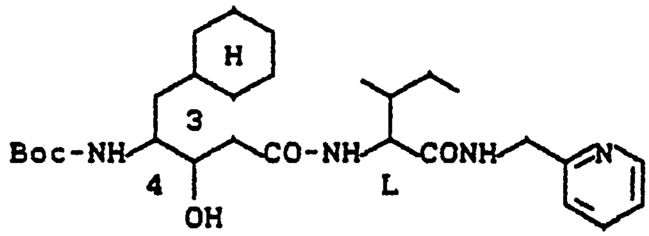

13,2 g (41,8 mmol) N-tert.Butoxycarbonyl-4S-amino-3S-hydroxy-5-cyclohexyl-pentansäure [J. Boger et al., J. Med. Chem. 28, 1779 (1985)] und 13,5 g (46 mmol) der Verbindung aus Beispiel 2 werden mit 420 ml Methylenchlorid versetzt. Die Suspension wird durch Zugabe von 31 ml (230 mmol) Triethylamin in Lösung gebracht und auf -20 °C gekühlt (Aceton/Trockeneis). Unter Rühren tropft man bei dieser Temperatur 39 ml einer 50%igen Lösung von Propanphosphonsäureanhydrid in Methylenchlorid hinzu und läßt den Ansatz über Nacht auf Raumtemperatur kommen. Das Methylenchlorid wird am Rotationsverdampfer abgezogen und der Rückstand in 400 ml Essigester aufgenommen. Die organische Phase wird nacheinander 1 mal mit 200 ml gesättigter Natriumhdyrogencarbonatlösung, 2 mal mit je 200 ml Pufferlösung pH 7 (Merck, Art.-Nr. 9439) und 4 mal mit je 200 ml vollentsalztem Wasser gewaschen. Die Pufferlösungen werden mit je 100 ml Essigester zurückextrahiert. Die vereinigten organischen Phasen werden 2 mal mit je 500 ml gesättigter Kochsalzlösung gewaschen, über Natriumsulfat getrocknet und am Rotationsverdampfer auf ein Volumen von etwa 125 ml eingeengt. Die Lösung wird mit 125 ml n Hexan versetzt und über Nacht in den Kühlschrank (5 °C) gestellt. Das auskristallisierte Produkt wird abgesaugt, mit wenig kaltem Essigester/n-Hexan 1:1 nachgewaschen und im Vakuum getrocknet.
Ausbeute: 17,2 g (79% der Theorie)
MS-DCI; m/z 519 (M+H)
DC-System III: $R_f$ = 0,48
DC-System IV: $R_f$ = 0,63
DC-System V : $R_f$ = 0,48
HPLC-System II: $R_t$ = 6,02 Min

Beispiel 4 (Formel II)

4S-Amino-3S-hydroxy-5-cyclohexyl-pentanoyl-L-isoleucin-(2-picolyl)amid Dihydrochlorid

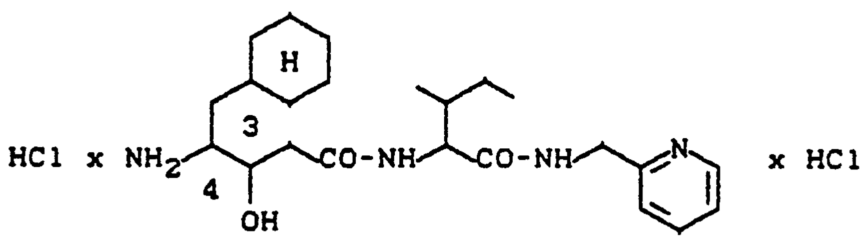

16 g (30,8 mmol) der Verbindung aus Beispiel 3 werden unter Eiskühlung mit 160 ml 4N Salzsäure (Gas) in Dioxan versetzt. Die Suspension wird durch Zugabe von 5 ml Methanol in Lösung gebracht. Nach Abreaktion (DC-Kontrolle) engt man am Rotationsverdampfer ein. Nach mehrmaliger Koevaporation mit Diethylether wird das Rohprodukt mit Diethylether verrührt, abgesaugt und im Exsikkator über Kaliumhydroxid getrocknet.
Ausbeute: 16,56 g (95% der Theorie)
MS-DCI: m/z 419 (M+H)
DC-System III: $R_f$ = 0,35

Beispiel 5 (Formel III)

N-tert.-Butoxycarbonyl-2-R,S-amino-2-[2-(1,3-dithiolano)]essigsäure

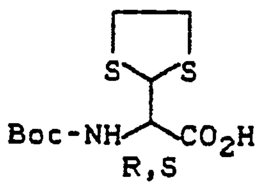

19,9 g (0,11 mol) 2-Amino-2-R,S-2-[2-(1,3-dithiolano)]essigsäure [M.P. Mertes, A.A. Ramsey, J. Med. Chem. 12, 342 (1969)] werden in 100 ml Dioxan gelöst und mit 6,6 g (0,16 mol) Natriumhydroxid und 29 g (0,13 mol) Di-tert.-Butylcarbonat versetzt. Man läßt über Nacht bei Raumtemperatur rühren und zieht das Dioxan am Rotationsverdampfer ab. Die Mischung wird mit 200 ml Wasser aufgefüllt und mit Ether mehrmals extrahiert. Die basische wäßrige Phase wird mit Salzsäure auf pH 3 gestellt und mehrmals mit Methylenchlorid extrahiert. Die vereinigten organischen Phasen werden mit gesättigter Kochsalzlösung gewaschen, über Natriumsulfat getrocknet und eingeengt.
Ausbeute: 18,1 g (60,6% der Theorie)
MS-DCI: m/z 280 (M + H); m/z 297 ($M + NH_4$)
DC-System IV: $R_f$ = 0,55

Beispiel 6 (Formel II)

N-tert.-Butoxycarbonyl-4S-amino-3S-hydroxy-5-cyclohexylpentanoyl-L-leucinethylester

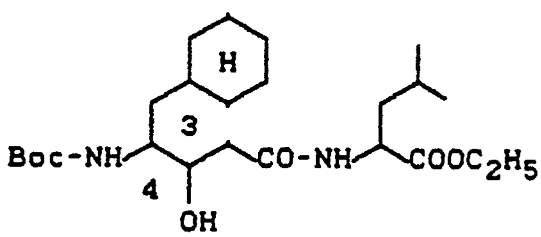

Die Titelverbindung wird analog Beispiel 3 durch Propanphosphonsäureanhydridkupplung von 4,8 g (15,2 mmol) N-tert.-Butoxycarbonyl-4S-amino-3S-hydroxy-5-cyclohexylpentansäure [J. Boger et al., J. Med. Chem. 28, 1779 (1985)] und 3,3 g (16,7 mmol) L-Leucinmethylester Hydrochlorid erhalten.
Ausbeute: 6,4 g (92% der Theorie)
DC-System VI : $R_f$ = 0,85
DC-System VII : $R_f$ = 0,13
DC-System VIII: $R_f$ = 0,55
DC-System IX : $R_f$ = 0,24

Beispiel 7 (Formel II)

4S-Amino-3S-hydroxy-5-cyclohexylpentanoyl-L-leucinethylester Hydrochlorid

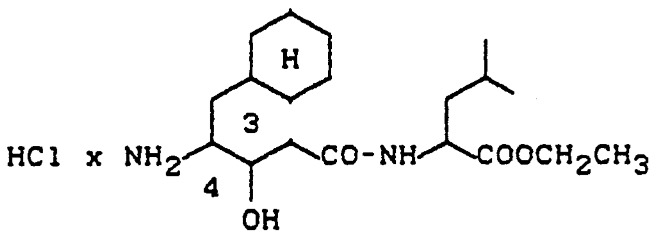

Die Titelverbindung wird analog Beispiel 4 durch Abspaltung der N-tert.-Butoxycarbonylschutzgruppe aus 6,1 g (13,3 mmol) der Verbindung aus Beispiel 6 erhalten.
Ausbeute: 5,1 g (98% der Theorie)
(+) FAB-MS: m/z 357 (M+H); m/z 379 (M+Na).
DC-System III: $R_f$ = 0,37
DC-System IV : $R_f$ = 0,29
DC-System VI : $R_f$ = 0,85
DC-System VII: $R_f$ = 0,13
DC-System VIII: $R_f$ = 0,55
DC-System IX : $R_f$ = 0,24

Beispiel 8

N-tert.-Butoxycarbonyl-4S-amino-3S-hydroxy-5-cyclohexylpentanoyl-(2S-methyl)butylamid

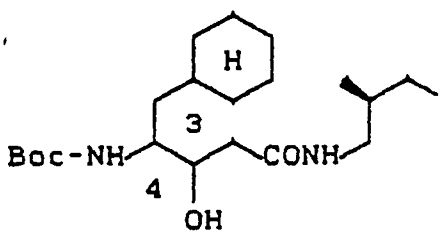

Die Titelverbindung wird analog Beispiel 3 durch Propanphosphonsäureanhydridkupplung von 4,8 g (15,2 mmol) N-tert.-Butoxycarbonyl-4S-amino-3S-hydroxy-5-cyclohexylpentansäure [J. Boger et al., J. Med. Chem. 28, 1779 (1985)] und 1,9 ml (16,7 mmol) (2-S-Methyl)butylamin erhalten.
Ausbeute: 5,4 g (92,4 % der Theorie)
DC-System IV : $R_f$ = 0,72
HPLC-System II : $R_t$ = 13,51 Min
(+) FAB-MS : m/z 385 (M+H); m/z 391 (M+Li)

Beispiel 9

4S-Amino-3S-hydroxy-5-cyclohexylpentanoyl-(2S-methyl)butylamid Hydrochlorid

HCl x 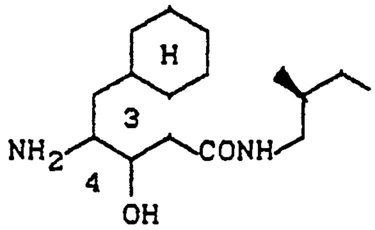

Die Titelverbindung wird analog Beispiel 4 durch Abspaltung der Boc-Schutzgruppe aus 5,4 g (14 mmol) der Verbindung aus Beispiel 8 erhalten.
Ausbeute: 5 g (> 100 % der Theorie, HCl-haltig)
DC-System III : $R_f$ = 0,26
(+)FAB-MS : m/z 285 (M+H)

Beispiel 10

N-Phthaloyl-2-R,S-amino-2-[2-(1,3-dithiano)]essigsäureethylester

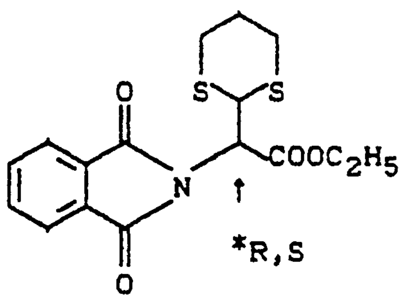

Die Titelverbindung wird nach der in der Literatur für die entsprechende Dithiolanoverbindung beschriebenen Weise [M.P. Mertes, A.A. Ramsey, J. Med. Chem. 12, 342 (1969)] ausgehend von 77,8 g (0,3 mol) α-Phthalimidomalonaldehydethylester und 90 ml (0,9 mol) 1,3-Dimercaptopropan dargestellt. Das Rohprodukt wird durch Kieselgelchromatographie mit Dichlormethan als Eluens gereinigt.
Ausbeute: 74 g (70 % der Theorie)
DC-System II : $R_f$ = 0,89
DC-System XI : $R_f$ = 0,44
MS < HPLC-System II: $R_t$ = 6,63 min
HPLC-DCI : m/z 352 (M+H), m/z 204; m/z 119
$^1$H-NMR (250 MHz, DMSO): δ : 7,92 (m, 4H); 5,25 (d, 1H); 4,62 (d, 1H); 4,15 (g, 2H); 3,03 (m, 1H); 2,72 (m, 2H); 2,57 (m, 1H); 1,95 (m, 1H); 1,78 (m, 1H); 1,14 (t, 3H);

Beispiel 11

2-R,S-Amino-2-[2-(1,3-dithiano)]essigsäureethylester

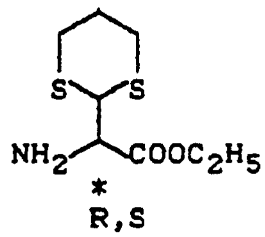

Die Titelverbindung wird nach der in der Literatur für die entsprechende Dithiolanverbindung beschriebenen Weise [M.P. Mertes, A.A. Ramsey, J, Med. Chem. 12, 342 (1969)] ausgehend von 74 g (0,21 mol) der Verbindung aus Beispiel 10 und 17 ml (0,35 mol) Hydrazinhydrat hergestellt.
Ausbeute: 30 g (64 % der Theorie)
DC-System II : $R_f$ = 0,80
(+) FAB-MS : m/z 222 (M+H); m/z 228 (M+Li); m/z 244 (M+Na).
$^1$H-NMR (250 MHz, DMSO): δ : 4,10 (m, 3H); 3,65 (d, 1H); 2,80 (m, 4H); 1,92 (m, 3H); 1,78 (m, 1H); 1,18 (t, 3H).

## Beispiel 12

2-R,S-Amino-2-[2-(1,3-dithiano)]essigsäure

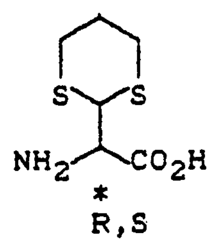

Die Titelverbindung wird nach der in der Literatur für die entsprechende Dithiolanverbindung beschriebenen Weise [M.P. Mertes, A.A. Ramsey, J. Med. Chem. 12, 342 (1969)] durch Kochen der Verbindung aus Beispiel 11 in Ethanol/KOH dargestellt.
DG-System III : $R_f$ = 0,05
DC-System V : $R_f$ = 0,35

## Beispiel 13

N-tert.-Butoxycarbonyl-2-R,S-amino-2-[2-(1,3-dithiano)]essigsäure

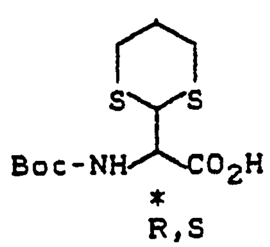

Die Titelverbindung wird analog Beispiel 5 durch Umsetzung der Verbindung aus dem Beispiel 12 mit

Di-tert.-Butylcarbonat erhalten.
DC-System I $R_f$ = 0,19
DC-System II : $R_f$ = 0,12
DC-System IV $R_f$ = 0,55
DC-System V : $R_f$ = 0,86
MS-DCI: m/z 294 (M + H); m/z 311 (M + $NH_4$); m/z 255
$^1$H-NMR (250 MHz, DMSO): $\delta$ : 12,88 (breit, 1H); 7,0 (d, 1H); 4,44 (m, 1H); 4,11 (d, 1H); 2,90 (m, 2H); 2,70 (m, 2H); 1,86 (m, 2H); 1,38 (s, 9H).

Beispiel 14

N-Acetyl-2-R,S-amino-2-[2-(1,3-dithiano)]essigsäureethylester

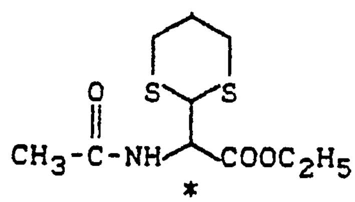

R,S

20 g (90,5 mmol) der Verbindung aus Beispiel 11 werden in 400 ml Dioxan gelöst, mit 10 ml (69 mmol) Triethylamin und schließlich 7,7 ml (81,3 mmol) Acetanhydrid versetzt. Die Mischung wird über Nacht bei Raumtemperatur gerührt, vom Dioxan abgezogen und mit Ether/Bicarbonatlösung verrieben. Der Rückstand wird isoliert, erneut mit Diethylether verrieben, abgesaugt und getrocknet.
Ausbeute: 20,9 g (87,8 % der Theorie)
DC-System IV : $R_f$ = 0,76
DC-System V : $R_f$ = 0,89
HPLC-System II : $R_t$ = 1,78 Min
MS-DCI; m/z 264 (M + H)

Beispiel 15

N-Acetyl-2-R,S-amino-2-[2-(1,3-dithiolano)]essigsäureethylester

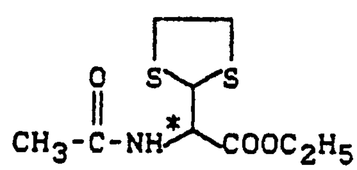

*R,S

Die Titelverbindung wird analog Beispiel 14 ausgehend von 2-R,S-Amino-2-[2-(1,3-dithiolano)-]essigsäureethylester [M.P. Mertes, A.A. Ramsey, J. Med. Chem. 12, 342 (1969)] erhalten.
DC-System I : $R_f$ = 0,75
DC-System II : $R_f$ = 0,67
HPLC-System II : $R_t$ = 1,73 Min
MS-DCI : m/z = 250 (M + H)

Beispiel 16

N-Ethoxycarbonyl-L-phenylalanyl-{2-R,S-amino-2-[2-(1,3-diathiano)]}essigsäureethylester

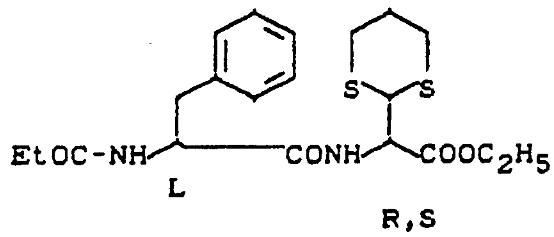

Die Titelverbindung wird analog Beispiel 1 durch Propanphosphonsäureanhydridkupplung der Verbindung aus Beispiel 11 (3 g; 13 mmol) und N-Ethoxycarbonyl-L-phenylalanin erhalten.
Ausbeute: 5 g (87,4 % der Theorie)

DC-System I : $R_f$ = 0,87 } beide Stereoisomere
HPLC-System II : $R_t$ = 4,58 Min }

(+) FAB-MS : m/z = 441 (M+H); m/z = 463 (M+Na)

Beispiel 17

N-Ethoxycarbonyl-L-phenylalanyl-{2-R,S-amino-2-[2-(1,3-dithiano)]}essigsäure

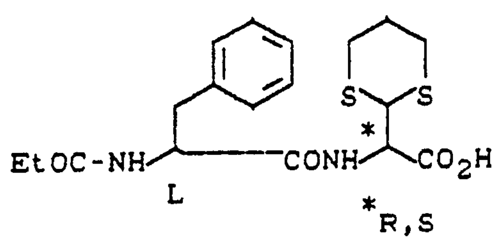

Die Titelverbindung wird analog Beispiel X oder Beispiel XI durch basische Verseifung der Verbindung aus Beispiel 16 (4 g; 9,1 mmol) und Standardaufarbeitung erhalten.
Ausbeute: 3,7 g (97 % der Theorie)
DC-System IV : $R_f$ = 0,63 (Isomer A)
: $R_f$ = 0,54 (Isomer B)
(+) FAB-MS : m/z 413 (M+H): m/z 419 (M+Li).

Herstellungsbeispiele (allgemeine Formeln I, Ia, Ib, Ic und Id)

Beispiel 18

N-tert.-Butoxycarbonyl-L-(3,5-diiodo-4-methoxy)phenylalanin-methylester

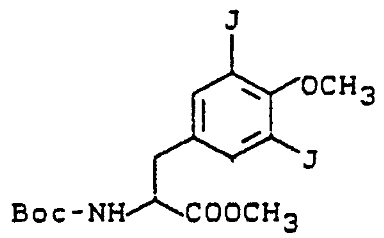

Boc-L-3,5-diiodotyrosin (1,0 g, 1,87 mmol; Bissendorf Biochemicals) wurde mit Methyljodid (352 µl, 5,61 mmol) und Kaliumcarbonat (0,78 g, 5,61 mmol) 3 Tage in 10 ml DMF unter Feuchtigkeitsausschluß (Trockenrohr) gerührt. Nach dem Einengen wurde die Reaktionsmischung mit Wasser (150 ml) verdünnt und dreimal mit Ether (je 50 ml) extrahiert. Die vereinigte organische Phase wurde über Natriumsulfat getrocknet und eingedampft.
Ausbeute: 1,12 g (> 100 % der Theorie, enthält DMF)
DC-System IV: $R_f$ = 0,89
$^1$H-NMR (250 MHz, DMSO): $\delta$ = 7,7 (s, 2H); 7,28 (d, 1H); 4,15 (m, 1H); 3,71 (s, 3H); 3,62 (s, 3H); 2,95 (dd, 1H); 2,74 (dd, 1H); 1,33 (s, 9H).

Beispiel 19

N-tert.-Butoxycarbonyl-L-(3,5-diiodo-4-methoxy)-phenylalanine

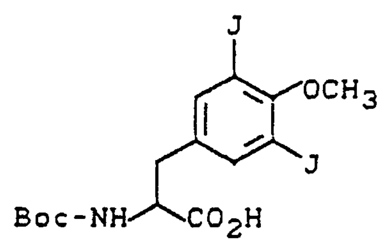

Die Titelverbindung wird analog Beispiel X oder Beispiel XI durch basische Verseifung aus der Verbindung des Beispiels 18 (0,45 g, 0,80 mml) und Standardaufarbeitung erhalten.
Ausbeute: 0,33 g (75 % der Theorie)
DC-System IV: $R_f$ = 0,56
$^1$H-NMR (250 MHz, DMSO): $\delta$ = 12,65 (breit, 1H); 7,71 (s, 2H); 7,09 (d, 1H); 4,08 (m, 1H); 3,70 (s, 3H); 2,96 (dd, 1H); 2,69 (dd, 1H); 1,32 (s, 9H).

Beispiel I

N-tert.-Butoxycarbonyl-2-R,S-amino-2-[2-(1,3-dithiolano)]-acetyl-4S-amino-3S-hydroxy-5-cyclohexyl-pentanoyl-L-isoleucin-(2-picolyl)amid

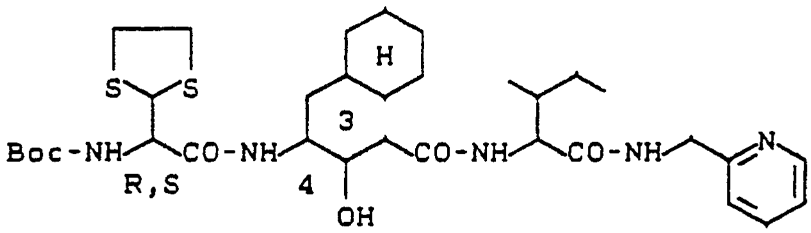

4,4 g (15,7 mmol) der Verbindung aus Beispiel 5 und 7,3 g (15 mmol) der Verbindung aus Beispiel 4 werden mit 20 ml Methylenchlorid und 18,3 ml (105 mmol) Diisopropylethylamin vesetzt. Die Lösung wird gerührt und auf -20 °C gekühlt (Trockeneis/Aceton). Man tropft bei dieser Temperatur 11,7 ml einer 50%igen Lösung von Propanphosphorsäureanhydrid in Methylenchlorid (Hoechst AG) hinzu und laßt den Ansatz über Nacht auf Raumtemperatur kommen. Das Methylenchlorid wird am Rotationsverdampfer abgezogen und der Rückstand in Essigester aufgenommen. Die organische Phase wird je 3 mal mit gesättigter Natriumhydrogencarbonatlösung, Pufferlösung pH 4 (Merck, Artikel-Nr. 9435) und gesättigter Kochsalzlösung ausgeschüttelt, über Natriumsulfat getrocknet, eingeengt und am Hochvakuum getrocknet.
Ausbeute: 9 g (79,8% der Theorie)
(+) FAB-MS: m/z 680 (M+H); m/z 686 (M+Li)
DC-System III: $R_f$ = 0,59
DC-System IV : $R_f$ = 0,67
HPLC-System II: $R_t$ = 6,16 min

Beispiel II

2-R,S-Amino-2-[2-(1,3-dithiolano)]-acetyl-4S-amino-3S-hydroxy-5-cyclohexyl-pentanoyl-L-isoleucin-(2-picolyl)amid Dihydrochlorid

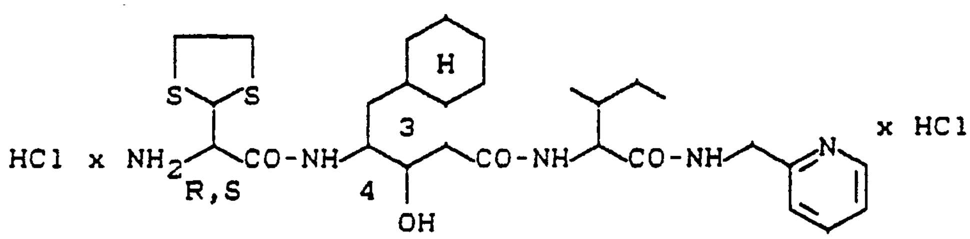

9 g (13,24 mmol) der Verbindung aus Beispiel I werden unter Eiskühlung mit 150 ml 4N Salzsäure in Dioxan versetzt und 4 Stunden gerührt. Das Dioxan/Salzsäure-Gemisch wird am Rotationsverdampfer abdestilliert. Das nach mehrmaliger Koevaporation mit Diethylether erhaltene Produkt wird abgesaugt, mit Diethylether nachgewaschen und im Exsikkator über Kaliumhydroxid getrocknet.
Ausbeute: 8,6 g (100% der Theorie)
(+) FAB-MS: m/z 580 (M+H); m/z 602 (M+Na).
DC-System III: $R_f$ = 0,38 (Isomer A)
$R_f$ = 0,33 (Isomer B)
DC-System IV : $R_f$ = 0,21

Beispiel III

N-tert.-Butoxycarbonyl-L-Phenylalanyl-{2-R,S-amino-2-[2-(1,3-dithiolano)]}-acetyl-4S-amino-3S-hydroxy-5-cyclohexylpentanoyl-L-isoleucin-(2-picolyl)amid

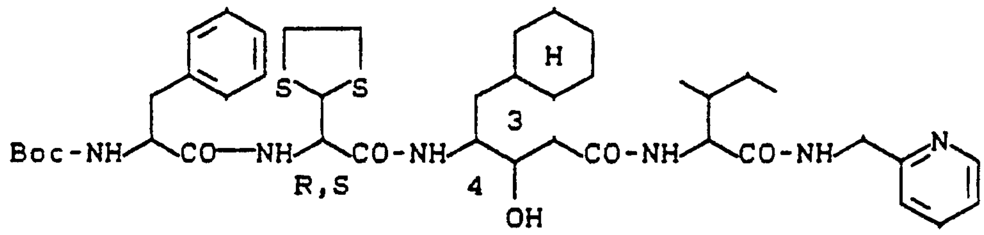

5,7 g (9 mmol) der Verbindung aus Beispiel II und 3,1 g (11,7 mmol) N-tert.Butoxycarbonyl-L-Phenylalanin werden mit 50 ml Methylenchlorid und 11 ml (63 mmol) Diisopropylethylamin versetzt. Die Lösung wird gerührt und auf -20°C gekühlt (Trockeneis/Aceton). Man tropft bei dieser Temperatur 8,2 ml (12,6 mmol) einer 50%igen Lösung von Propanphosphonsäureanhydrid in Methylenchlorid (Hoechst AG) hinzu und läßt den Ansatz über Nacht auf Raumtemperatur kommen. Man gibt 100 ml Methylenchlorid hinzu und schüttelt 3 mal mit gesättigter Natriumhydrogencarbonatlösung, 3 mal mit Pufferlösung pH 7 (Merck, Artikel-Nr. 9439) und einmal mit halbgesättigter Kochsalzlösung aus. Die organische Phase wird über Natriumsulfat getrocknet, eingeengt und am Hochvakuum getrocknet.

Ausbeute: 7,2 g (96% der Theorie)
(+) FAB-MS: m/z 827 (M+H)
DC-System I : $R_f$ = 0,42 (Isomer A)
$R_f$ = 0,38 (Isomer B)
DC-System III: $R_f$ = 0,60 (Isomer A)
$R_f$ = 0,56 (Isomer B)
HPLC-System II: $R_t$ = 14,34 min (Isomer A)
$R_t$ = 12,59 min (Isomer B)

Beispiel IV und Beispiel V

N-tert.-Butoxycarbonyl-L-phenylalanyl{2-S-amino-2-[2-(1,3-dithiolano)]}-acetyl-4S-amino-3S-hydroxy-5-cyclohexylpentanoyl-L-isoleucin-(2-picolyl)amid (Beispiel IV)

N-tert.-Butoxycarbonyl-L-phenylalanyl{2-R-amino-2-[2-(1,3-dithiolano)]}-acetyl-4S-amino-3S-hydroxy-5-cyclohexylpentanoyl-L-isoleucin-(2-picolyl)amid (Beispiel V)

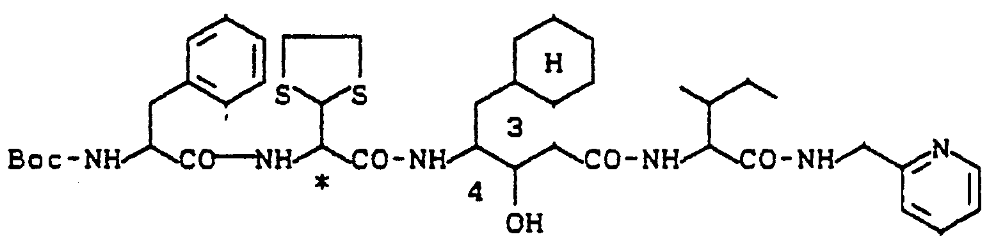

* S = Beispiel 9 (Isomer A)
* R = Beispiel 10 (Isomer B)

7,2 g der Verbindung aus Beispiel III werden an Kieselgel 60 (Merck, Art.-Nr. 9385) 0,040 - 0,063 mm (230 -400 mesh) mit einem Stufengradienten aus Methylenchlorid/Methanol (100/0; 99/1; 98/2; 97/3; 95/5; 9/1) getrennt. Die Detektion erfolgt bei 214 nm, die Fraktionskontrolle dünnschicht- und hochdruckflüssigkeitschromatographisch. Nach Vereinigung und Abrotieren der Lösemittel erhält man:

3,1 g der Verbindung aus Beispiel IV (Isomer A ≡ S)
0,6 g der Verbindung aus Beispiel IV und Beispiel V (Mischfraktion aus A und B)
2,91 g der Verbindung aus Beispiel V (Isomer B ≡ R)

Analytische Daten Isomer A (Beispiel IV)
DC-System III: $R_f$ = 0,60
HPLC-System II: $R_t$ = 14,34 min
(+) FAB-MS: m/z 827 (M+H)
Analytische Daten Isomer B (Beispiel V)
DC-System III: $R_f$ = 0,56
HPLC-System II: $R_t$ = 12,59 min
(+) FAB-MS: m/z 827 (M+H)

Beispiel VI

N-tert.-Butoxycarbonyl-2-R,S-amino-2-[2-(1,3-dithiolano)]-acetyl-4S-amino-3S-hydroxy-5-cyclohexylpentanoyl-L-leucinethylester

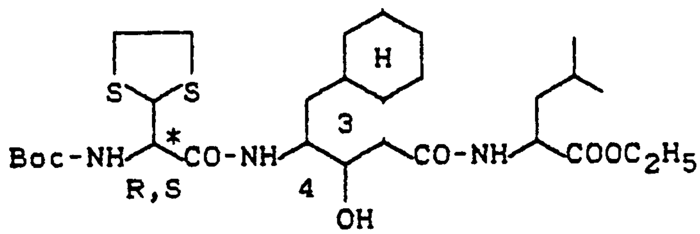

Die Titelverbindung wird analog Beispiel I durch Propanphosphonsäureanhydridkopplung aus 4 g (14,3 mmol) der Verbindung aus Beispiel 5 und 4,6 g (13 mmol) der Verbindung aus Beispiel 7 erhalten. Das Rohprodukt (8,6 g) wird mit einem Stufengradienten aus Methylenchlorid/Methanol (100/0; 98/2; 95/5) über 400 g Kieselgel 60 (Merck, Artikel-Nr. 9385; 0,040 - 0,063 mm) filtriert. Die produktenthaltenden Fraktionen werden vereinigt und eingeengt.
Ausbeute: 5,7 g (66% der Theorie)
DC-System IV: $R_f$ = 0,85
HPLC-System II: $R_t$ = 17,91 min
(+) FAB-MS: m/z 618 (M+H); m/z 640 (M+Na)

Beispiel VII

2-R,S-Amino-2-[2-(1,3-dithiolano)]-acetyl-4S-amino-3S-hydroxy-5-cyclohexylpentanoyl-L-leucinethylester Hydrochlorid

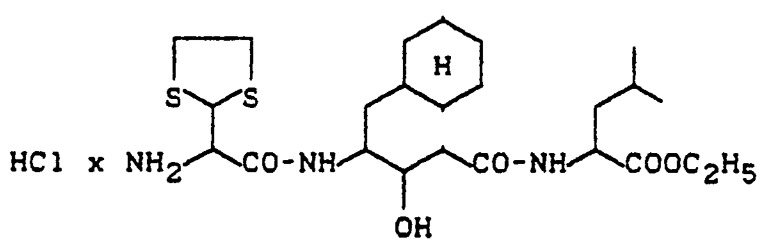

Die Titelverbindung wird analog Beispiel II durch Abspaltung der N-tert.-Butoxycarbonylschutzgruppe aus 5,3 g (8,6 mmol) der Verbindung aus Beispiel VI erhalten.
Ausbeute: 4,7 g (85% der Theorie)
DC-System III: $R_f$ = 0,71 (Isomer A)

$R_f$ = 0,64 (Isomer B)
(+)FAB-MS: m/z 518 (M+H); m/z 540 (M+Na)

Beispiel VIII

N-tert.-Butoxycarbonyl-L-phenylalanyl-{2-R,S-amino-2-[2-(1,3-dithiolano)]}-acetyl-4S-amino-3S-hydroxy-5-cyclohexylpentanoyl-L-leucinethylester

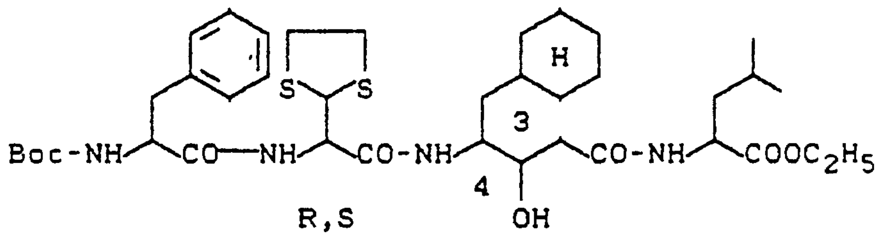

Die Titelverbindung wird analog Beispiel III durch Propanphosphonsäureanhydrid-Kopplung aus 1,7 g (6,6 mmol) N-tert.-Butoxycarbonyl-L-phenylalanin und 2,8 g (5 mmol) der Verbindung aus Beispiel VII erhalten.

Ausbeute: 3 g (78% der Theorie)
DC-System I: $R_f$ = 0,65 (Isomer A)
$R_f$ = 0,57 (Isomer B)
HPLC-System II: $R_t$ = 35,58 min (Isomer A)
$R_t$ = 31,64 min (Isomer B)
(+) FAB-MS: m/z 765 (M+H); m/z 787 (M+Na)

Beispiel IX

N-Ethoxycarbonyl-L-phenylalanyl-{2-R,S-amino-2-[2-(1,3-dithiolano)]}acetyl-4S-amino-3S-hydroxy-5-cyclohexylpentanoyl-L-leucinethylester

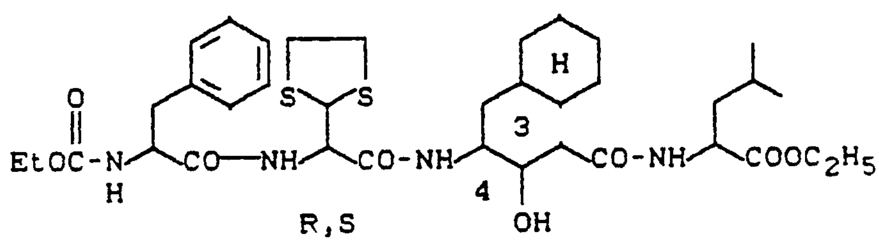

Die Titelverbindung wird analog Beispiel III aus 1,6 g (6,6 mmol) N-Ethoxycarbonyl-L-Phenylalanin und 2,8 g (5 mmol) der Verbindung aus Beispiel VII erhalten.

Ausbeute: 2,3 g (62% der Theorie)
DC-System I: $R_f$ = 0,60 (beide Isomere A+B)
HPLC-System II: $R_t$ = 18,66 (Isomer A)
$R_t$ = 15,31 (Isomer B)
(+)FAB-MS: m/z 737 (M+H); m/z 759 (M+Na)

Beispiel X

N-tert.-Butoxycarbonyl-L-phenylalanyl-{2-R,S-amino-2-[2-(1,3-dithiolano)]}acetyl-4S-amino-3S-hydroxy-5-cyclohexylpentanoyl-L-leucin

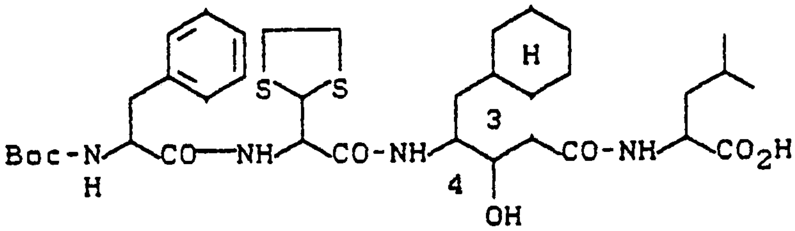

3 g (3,9 mmol) der Verbindung aus Beispiel VIII werden bei Raumtemperatur in 30 ml Dioxan gelöst und mit 4,3 ml einer wäßrigen 1 N Natriumhydroxid-Lösung versetzt. Der Ansatz wird 2 Tage bei Raumtemperatur gerührt und dann mit 1 N wäßriger Salzsäure sauer gestellt. Das organische Lösemittel wird am Rotationsverdampfer abgezogen, der Rückstand wird abfiltriert, in Wasser/Acetonitril 95/5 resuspendiert, eingefroren und lyophilisiert.
Ausbeute: 2,9 g (90% der Theorie)
(+)FAB-MS: m/z 803 (M+2Na-H); m/z 819 (M+Na+K-H).

Beispiel XI

N-Ethoxycarbonyl-L-phenylalanyl-{2-R,S-amino-2-[2-(1,3-dithiolano)]}acetyl-4S-amino-3S-hydroxy-5-cyclohexylpentanoyl-L-leucin

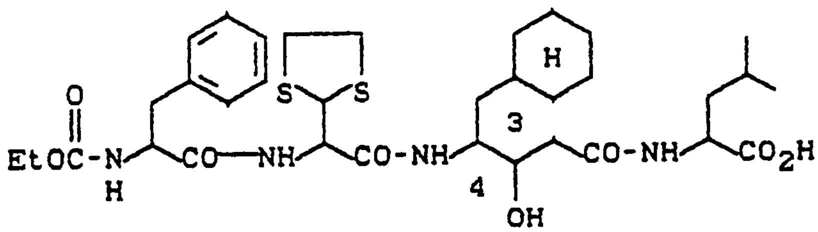

Die Titelverbindung wird analog Beispiel X durch basische Verseifung von 2,3 g (3,1 mmol) der Verbindung aus Beispiel IX erhalten.
Ausbeute: 2,5 g (87% der Theorie)
(+)FAB-MS: m/z 775 (M+3Na-2H); m/z 791 (M+2Na+K-2H)

Beispiel XII und Beispiel XIII

N-Ethoxy-carbonyl-L-phenylalanyl-{2-S-amino-2-[2-(1,3-dithiolano)]}acetyl-4S-amino-3S-hydroxy-5-cyclohexylpentanoyl-L-isoleucin-(2-picolyl)amid (Beispiel XII)

N-Ethoxy-carbonyl-L-phenylalanyl-{2-R-amino-2-[2-(1,3-dithiolano)]}acetyl-4S-amino-3S-hydroxy-5-cyclohexylpentanoyl-L-isoleucin-(2-picolyl)amid (Beispiel XIII)

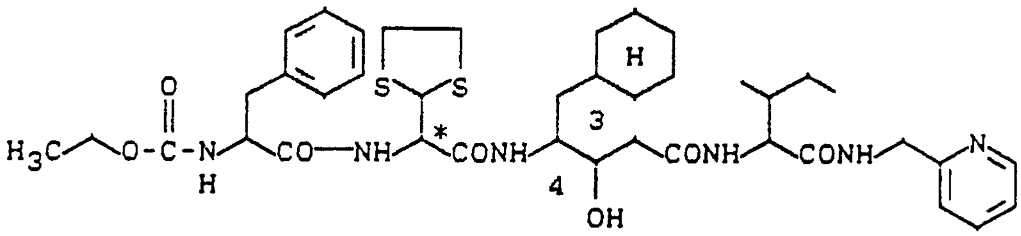

*S = Beispiel XII (Isomer A)
*R = Beispiel XIII (Isomer B)

Die Titelverbindungen wurden durch chromatographische Trennung (analog Beispiel IV und Beispiel V) des analog Beispiel III aus N-Ethoxycarbonyl-L-phenylalanin und der Verbindung aus Beispiel II synthetisierten Isomerengemisches erhalten. Zuerst eluiert das Beispiel XII (Isomer A, S-Isomer), dann das Beispiel XIII (Isomer B, R-Isomer).

Analytische Daten zu Beispiel XII (Isomer A)

DC-System III: $R_f$ = = 0,51
HPLC-System II: $R_t$ = 7,35 min
(+)FAB-MS: m/z = 799 (M+H); m/z = 821 (M+Na)

Analytische Daten zu Beispiel XIII (Isomer B)

DC-System III: $R_f$ = 0,47
HPLC-System II: $R_t$ = 5,81 min
(+)FAB-MS: m/z = 799 (M+H); m/z = 821 (M+Na)

Beispiel XIV und Beispiel XV

N-tert.-Butoxycarbonyl-L-(1-Naphthyl)alanyl-{2-S-amino-2-[2-(1,3-dithiolano)]}acetyl-4S-amino-3S-hydroxy-5-cyclohexylpentanoyl-L-isoleucin-(2-picolyl)amid (Beispiel XIV)

N-tert.-Butoxycarbonyl-L-(1-Naphthyl)alanyl-{2-R-amino-2-[2-(1,3-dithiolano)]}acetyl-4S-amino-3S-hydroxy-5-cyclohexylpentanoyl-L-isoleucin-(2-picolyl)amid (Beispiel XV)

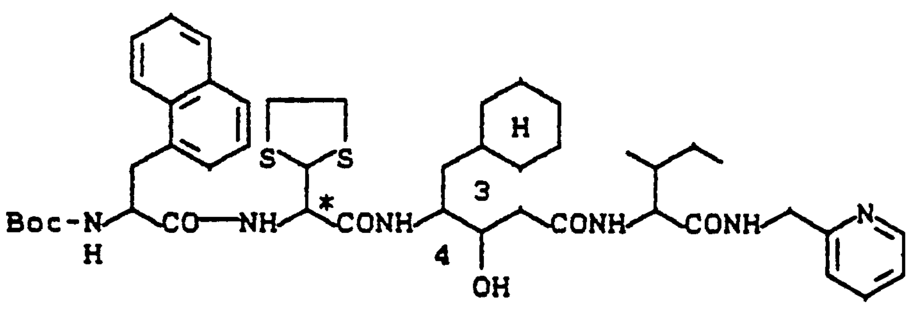

*S = Beispiel XIV (Isomer A)
*R = Beispiel XV (Isomer B)

Die Titelverbindungen wurden durch chromatographische Trennung (analog Beispiel IV und Beispiel V) des analog Beispiel III aus N-tert.-Butoxycarbonyl-L-(1-Naphthyl)alanin und der Verbindung aus Beispiel II synthetisierten Isomerengemisches erhalten. Zuerst eluiert das Beispiel XIV (Isomer A, S-Isomer), dann das Beispiel XV (Isomer B, R-Isomer).

Analytische Daten zu Beispiel XIV (Isomer A)

DC-System I: $R_f$ = 0,59
HPLC-System II: $R_t$ = 24,34 min
(+)FAB-MS: m/z = 877 (M+H); m/z = 899 (M+Na)
Analytische Daten zu Beispiel XV (Isomer B)
DC-System I: $R_f$ = 0,55
HPLC-System II: $R_t$ = 20,49 min
(+)FAB-MS: m/z = 877 (M+H); m/z = 899 (M+Na)

Beispiel XVI und Beispiel XVII

N-tert.-Butoxycarbonyl-L-(4-Methoxy)phenylalanyl-{2-S-amino-2-[2-(1,3-dithiolano)]}acetyl-4S-amino-3S-hydroxy-5-cyclohexylpentanoyl-L-isoleucin-(2-picolyl)amid (Beispiel XVI)

N-tert.-Butoxycarbonyl-L-(4-Methoxy)phenylalanyl-{2-R-amino-2-[2-(1,3-dithiolano)]}acetyl-4S-amino-3S-hydroxy-5-cyclohexylpentanoyl-L-isoleucin-(2-picolyl)amid (Beispiel XVII)

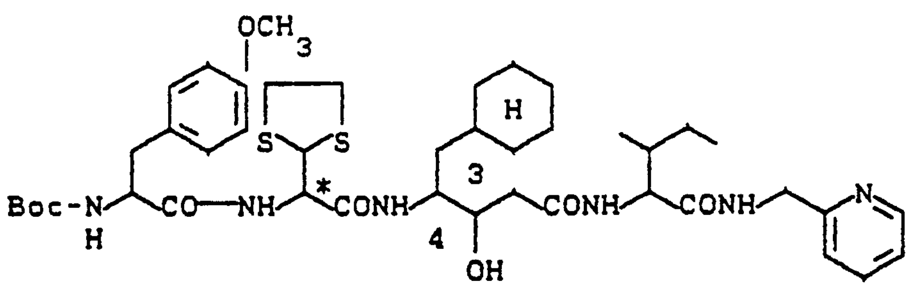

*S = Beispiel XVI (Isomer A)
*R = Beispiel XVII (Isomer B)

Die Titelverbindungen werden durch chromatographische Trennung (analog Beispiel VI und Beispiel V) des analog Beispiel III aus N-tert.-Butoxycarbonyl-L-(4-Methoxy)phenylalanin und der Verbindung aus Beispiel II synthetisierten Isomerengemisches erhalten. Zuerst eluiert das Beispiel XVI (Isomer A, S-Isomer), dann das Beispiel XVII (Isomer B, R-Isomer).
Analytische Daten zu Beispiel XVI (Isomer A)
DC-System 1: $R_f$ = 0,59
HPLC-System II: $R_t$ = 11,77 min
(+)FAB-MS: m/z = 857 (M+H); m/z = 879 (M+Na)
Analytische Daten zu Beispiel XVII (Isomer B)
DC-System I: $R_f$ = 0,56
HPLC-System II: $R_t$ = 10,49 min
(+)FAB-MS: m/z = 857 (M+H); m/z = 879 (M+Na)

Beispiel XVIII

N-tert.-Butoxycarbonyl-2-R,S-amino-2-[2-(1,3-dithiolano)]-acetyl-4S-amino-3S-hydroxy-5-cyclohexylpentansäuremethylester

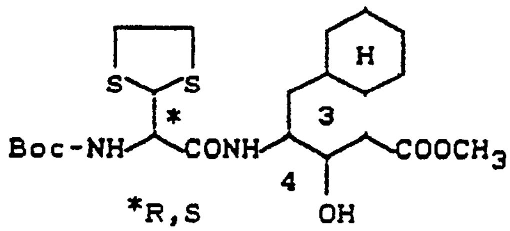

Die Titelverbindung wird analog Beispiel 1 durch Propanphosphonsäureanhydridkopplung aus 2,2 g (7,8 mmol) der Verbindung aus Beispiel 5 und 2,4 g (6,5 mmol) 4-S-Amino-3S-hydroxy-5-cyclohexylpentansäuremethylester Hydrochlorid [erhältlich durch Veresterung der 4S-Amino-3S-hydroxy-5-cyclohexylpentansäure (J. Boger et al., J. Med. Chem. 28, 1779 (1985) bzw. P.F. Schuda et al., J. Org. Chem. 1988, 53, 873-875) mit Diazomethan und Fällung als Hydrochlorid] und nachfolgender Kieselgelchromatographie rein erhalten.
Ausbeute: 3,6 g (88 % der Theorie)

DC-System IV: $R_f$ = 0,88
HPLC-System II: $R_t$ = 7,06 min
} Isomere nicht unterscheidbar

(+)FAB-MS: m/z 497 (M+Li); m/z 441; m/z 397

Beispiel XIX

2-R,S-Amino-2-[2-(1,3-dithiolano)]-acetyl-4S-amino-3S-hydroxy-5-cyclohexylpentansäuremethylester Hydrochlorid

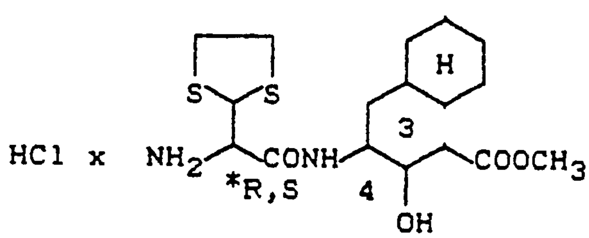

Die Titelverbindung wird analog Beispiel II durch Abspaltung der Boc-Schutzgruppe aus 3,5 g (7,1 mmol) der Verbindung aus Beispiel XVIII erhalten.
Ausbeute: 3,0 g (100 % der Theorie)
DC-System III: $R_f$ = 0,40 (Isomer A)
0,34 (Isomer B)
(+)FAB-MS: m/z 391 (M+H); m/z 397 (M+Li).

Beispiel XX und Beispiel XXI

N-Ethoxycarbonyl-L-phenylalanyl-{2-S-amino-[2-(1,3-dithiolano)]}-acetyl-4S-amino-3S-hydroxy-5-cyclohexylpentansäuremethylester (Beispiel XX)

N-Ethoxycarbonyl-L-phenylalanyl-{2-R-amino-[2-(1,3-dithiolano)]}-acetyl-4S-amino-3S-hydroxy-5-

cyclohexylpentansäuremethylester (Beispiel XXI)

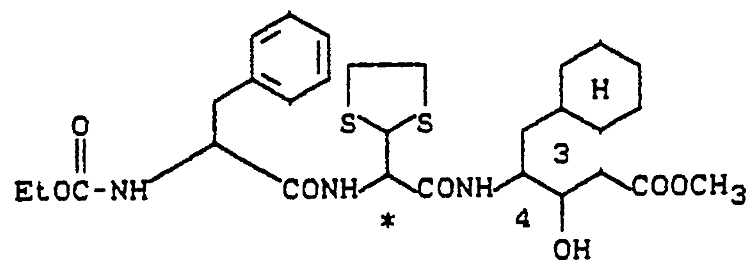

*S = Beispiel XX (Isomer A)
*R = Beispiel XXI (Isomer B)

Die Titelverbindungen werden durch chromatographische Trennung (analog Beispiel IV und Beispiel V) des analog Beispiel III aus 1,3 g (5,5 mmol) N-Ethoxycarbonyl-L-phenylalanin und 1,8 g (4,6 mmol) der Verbindung aus Beispiel XIX erhaltenen Isomerengemisches erhalten. Zuerst eluiert das Beispiel XX (Isomer A, S-Isomer), dann das Beispiel XXI (Isomer B, R-Isomer).

<u>Analytische Daten Isomer A (Beispiel XX):</u>
DC-System I: $R_f$ = 0,90
HPLC-System II: $R_t$ = 7,64 min
(+)FAB-MS: m/z 616 (M+Li); m/z 632 (M+Na).

<u>Analytische Daten Isomer B (Beispiel XXI):</u>
DC-System I: $R_f$ = 0,86
HPLC-System II: $R_t$ = 6,63 min
(+)FAB-MS: m/z 616 (M+Li); m/z 632 (M+Na).

## Beispiel XXII und Beispiel XXIII

N-tert.-Butoxycarbonyl-L-(4-methoxy)phenylalanyl-{2-S-amino-2-[2-(1,3-dithiolano)]}acetyl-4S-amino-3S-hydroxy-5-cyclohexylpentansäuremethylester (Beispiel XXII)

N-tert.-Butoxycarbonyl-L-(4-methoxy)phenylalanyl-{2-R-amino-2-[2-(1,3-dithiolano)]}-acetyl-4S-amino-3S-hydroxy-5-cyclohexylpentansäuremethylester (Beispiel XXII)

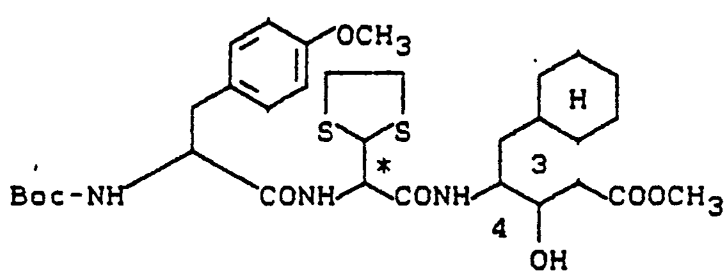

*S = Beispiel XXII (Isomer A)
*R = Beispiel XXIII (Isomer B)

Die Titelverbindungen werden analog Beispiel XX und Beispiel XXI ausgehend von 1,9 g (6,3 mmol) N-tert.-Butoxycarbonyl-L-(4-methoxy)phenylalanin und 2,4 g (5,7 mmol) der Verbindung aus Beispiel XIX erhalten.

<u>Analytische Daten Isomer A (Beispiel XXII):</u>

DC-System II: $R_f$ = 0,58
HPLC-System II: $R_t$ = 12,33 min
(+)FAB-MS; m/z 668 (M+H)
Analytische Daten Isomer B (Beispiel XXIII):
DC-System II: $R_f$ = 0,56
HPLC-System II: $R_t$ = 11,25 min
(+)FAB-MS: m/z 668 (M+H)

Beispiel XXIV

N-Ethoxycarbonyl-L-phenylalanyl-{2-R,S-amino-2-[2-(1,3-dithiolano)]}acetyl-4S-amino-3S-hydroxy-5-cyclohexylpentansäure

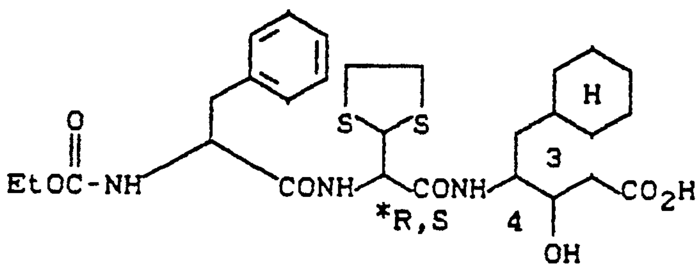

460 mg (0,754 mmol) eines Gemisches der Verbindungen aus Beispiel XX und Beispiel XXI werden in 20 ml Dioxan/Wasser 1/1 gelöst, mit 180 µl (1,4 eq) 6 N NaOH versetzt und über Nacht bei Raumtemperatur gerührt. Der Ansatz wird mit 1 N HCl auf pH 3 gestellt und vom Dioxan abrotiert. Der Niederschlag wird abgesaugt, mit wenig Wasser gewaschen und über KOH im Exsiccator getrocknet.
Ausbeute: 330 mg (73,5 % der Theorie)
DC-System I: $R_f$ = 0,34
DC-System IV : $R_f$ = 0,74
(+)FAB-MS: m/z 634 (M+K); m/z 672 (M+2K-H).

Beispiel XXV

N-tert.-Butoxycarbonyl-L-(4-methoxy)phenylalanyl-{2-R,S-amino-2-[2-(1,3-dithiolano)]}acetyl-4S-amino-3S-hydroxy-5-cyclohexylpentansäure

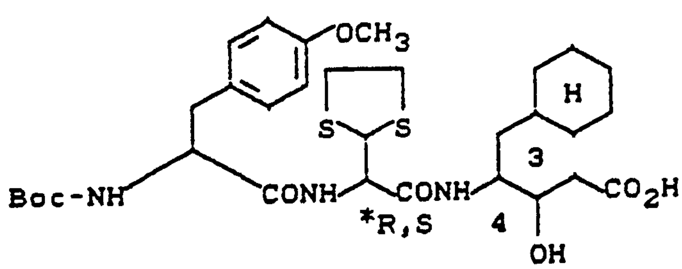

Die Titelverbindung wird analog Beispiel XXIV aus 790 mg (1,18 mmol) eines Gemisches aus den Verbindungen aus Beispiel XXII und Beispiel XXIII erhalten.
Ausbeute: 560 mg (73 % der Theorie)
DC-System I: $R_f$ = 0,18

DC-System III: $R_f$ = 0,05
DC-System IV: $R_f$ = 0,82
(+)FAB-MS: m/z = 660 (M+Li): m/z = 676 (M+Na).

Beispiel XXVI

N-tert.-Butoxycarbonyl-2-R,S-amino-2-[2-(1,3-dithiolano)]-acetyl-4S-amino-3S-hydroxy-5-cyclohexyl-pentanoyl-(2S-methyl)butylamid

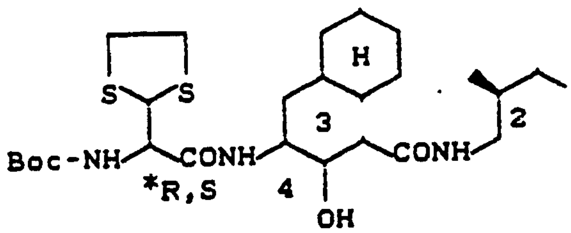

Die Titelverbindung wird analog Beispiel 1 durch Propanphosphonsäureanhydridkopplung aus 3,8 g (12 mmol) der Verbindung aus Beispiel 9 und 4 g (14,4 mmol) der Verbindung aus Beispiel 5 erhalten.
Ausbeute: 5,5 g (82,5 % der Theorie)

DC-System I: $R_f$ = 0,52 } Isomere nicht
HPLC-System II: $R_t$ = 13,38 min } unterscheidbar

(+)FAB-MS: m/z 545 (M+H); m/z 552 (M+Li); m/z 564 (M+Na)

Beispiel XXVII

2-R,S-Amino-2-(2-(1,3-dithiolano)]-acetyl-4S-amino-3S-hydroxy-5-cyclohexylpentanoyl-(2S-methyl)butylamid Hydrochlorid

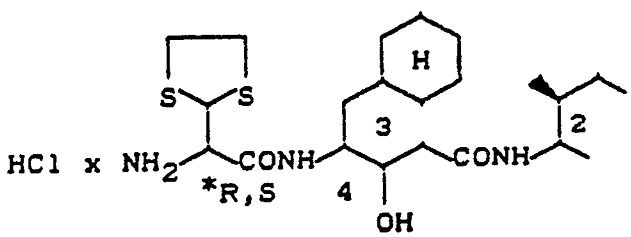

Die Titelverbindung wird analog Beispiel II durch Abspaltung der Boc-Schutzgruppe aus 5,4 g (9,9 mmol) der Verbindung aus Beispiel XXVI erhalten.
Ausbeute: 4,2 g (95 % der Theorie)
DC-System III: $R_f$ = 0,41 (Isomer A)
$R_f$ = 0,35 (Isomer B)

Beispiel XXVIII und Beispiel XXIX

N-Ethoxy-carbonyl-L-phenylalanyl-{2-S-amino-2-[2-(1,3-dithiolano)]}acetyl-4S-amino-3S-hydroxy-5-cyclohexylpentanoyl-(2S-methyl)-butylamid (Beispiel XXVIII)

N-Ethoxy-carbonyl-L-phenylalanyl-{2-R-amino-2-[2-(1,3-dithiolano)]}acetyl-4S-amino-3S-hydroxy-5-cyclohexylpentanoyl-(2S-methyl)-butylamid (Beispiel XXIX)

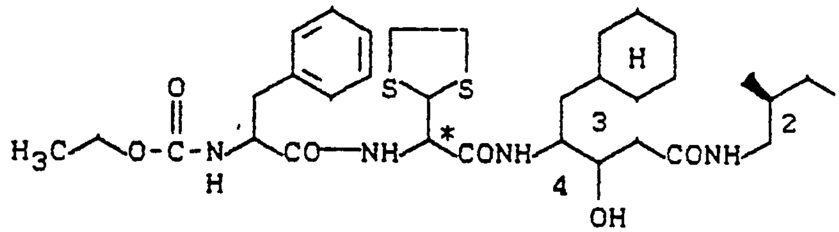

*S = Beispiel (XXVIII) (Isomer A)
*R = Beispiel (XXIX) (Isomer B)

Die Titelverbindungen wurden durch chromatographische Trennung (analog Beispiel IV und Beispiel V) des analog Beispiel III aus 2,1 g (4,7 mmol) der Verbindung aus Beispiel XXVII und 1,3 g (5,5 mmol) N-Ethoxycarbonyl-L-phenylalanin erhaltenen Isomerengemisches erhalten. Zuerst eluiert das Beispiel XXVIII (Isomer A, S-Isomer), dann das Beispiel XXIX (Isomer B, R-Isomer). Außerdem wird eine Mischfraktion aus Beispiel XXVIII und Beispiel XXIX erhalten.

Analytische Daten Isomer A (Beispiel XXVIII):
DC-System I: $R_f$ = 0,68
HPLC-System II: $R_t$ = 13,27 min
(+)FAB-MS: m/z = 671 (M+Li); m/z = 687 (M+Na).

Analytische Daten Isomer B (Beispiel XXIX):
DC-System I: $R_f$ = 0,65
HPLC-System II: $R_t$ = 11,61 min
(+)FAB-MS: m/z = 671 (M+Li); m/z = 687 (M+Na).

## Beispiel XXX und Beispiel XXXI

N-tert.-Butoxycarbonyl-L-(4-methoxy)phenylalanyl-{2-S-amino-2-[2-(1,3-dithiolano)]}acetyl-4S-amino-3S-hydroxy-5-cyclohexylpentanoyl-(2S-methyl)butylamid (Beispiel XXX)

N-tert.-Butoxycarbonyl-L-(4-methoxy)phenylalanyl-{2-R-amino-2-[2-(1,3-dithiolano)]}acetyl-4S-amino-3S-hydroxy-5-cyclohexylpentanoyl-(2S-methyl)butylamid (Beispiel XXXI)

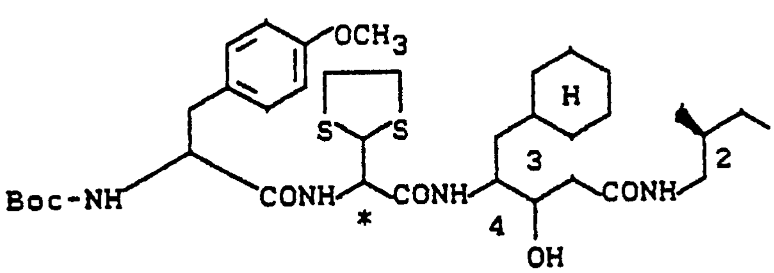

*S = Beispiel XXX (Isomer A)
*R = Beispiel XXXI (Isomer B)

Die Titelverbindungen werden analog Beispiel XXVIII und Beispiel XXIX ausgehend von der Verbindung aus Beispiel XXVII und N-tert.-Butoxycarbonyl-L-(4-methoxy)phenylalanin erhalten. Es tritt ebenfalls eine große Mischfraktion auf.

Analytische Daten Beispiel XXX:
DC-System II: $R_f$ = 0,38
HPLC-System II: $R_t$ = 13,63 min
(+)FAB-MS: m/z = 729 (M+Li)

Analytische Daten Beispiel XXXI:
DC-System II: $R_f$ = 0,34
HPLC-System II: $R_t$ = 11,91 min
(+)FAB-MS: m/z = 729 (M+Li)

Beispiel XXXII und Beispiel XXXIII

N-Ethoxycarbonyl-L-(4-methoxy)phenylalanyl-{2-S-amino-2-[2-(1,3-dithiolano)]}acetyl-4S-amino-3S-hydroxy-5-cyclohexylpentanoyl-L-isoleucin-(2-picolyl)amid (Beispiel XXXII)

N-Ethoxycarbonyl-L-(4-methoxy)phenylalanyl-{2-R-amino-2-[2-(1,3-dithiolano)]}acetyl-4S-amino-3S-hydroxy-5-cyclohexylpentanoyl-L-isoleucin-(2-picolyl)amid (Beispiel XXXIII)

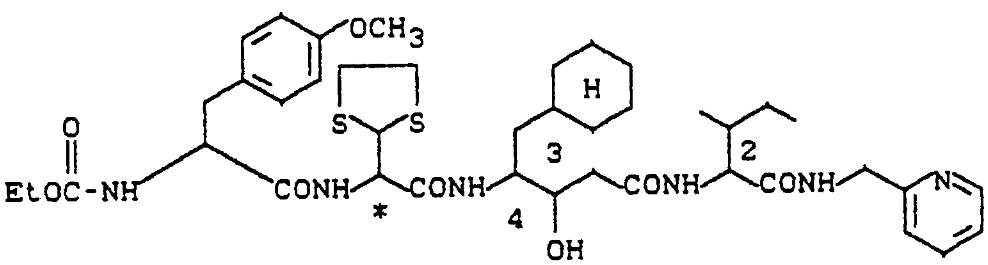

*S = Beispiel XXXII (Isomer A)
*R = Beispiel XXXIII (Isomer B)

Die Titelverbindungen werden durch chromatographische Trennung (analog Beispiel IV und Beispiel V) des analog Beispiel III aus N-Ethoxycarbonyl-L-(4-methoxy)-phenylalanin und der Verbindung aus Beispiel II synthetisierten Isomerengemisches erhalten. Zuerst eluiert das Beispiel XXXII (Isomer A, S-Isomer), dann das Beispiel XXXIII (Isomer B, R-Isomer).

Analytische Daten Isomer A (Beispiel XXXII):
DC-System III: $R_f$ = 0,72
DC-System IV: $R_f$ = 0,79
HPLC-System II: $R_t$ = 7,62 min
(+)FAB-MS: m/z = 829 (M+H); m/z = 851 (M+Na).

Analytische Daten Isomer B (Beispiel XXXIII):
DC-System III: $R_f$ = 0,69
DC-System IV : $R_f$ = 0,73
HPLC-System II: $R_t$ = 6,10 min
(+)FAB-MS: m/z = 829 (M+H); m/z = 851 (M+Na).

Beispiel XXXIV

L-Phenylalanyl-{2-R,S-amino-2-[2-(1,3-dithiolano)]}-acetyl-4S-amino-3S-hydroxy-5-cyclohexylpentanoyl-L-isoleucin-(2-picolyl)amid Dihydrochlorid

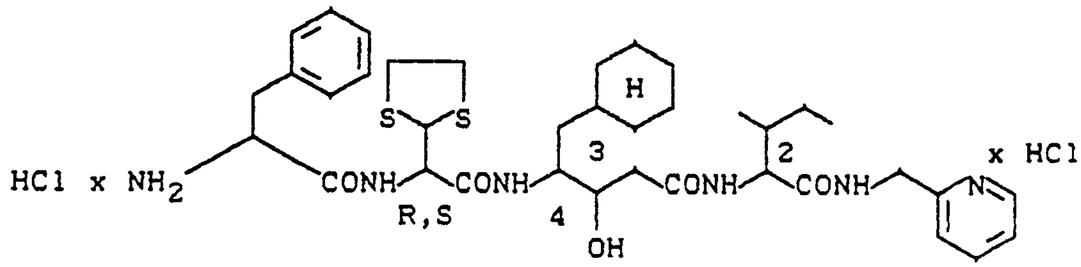

Die Titelverbindung wird analog Beispiel II durch Abspaltung der Boc-Schutzgruppe aus der Verbindung des Beispiels III bzw. eines Gemisches der Verbindungen aus Beispiel IV und Beispiel V erhalten.

Analytische Daten:

DC-System IV: $R_f$ = 0,43 (Isomer A, S-Isomer)

$R_f$ = 0,40 (Isomer B, R-Isomer)

Beispiel XXXV

N-tert.-Butoxycarbonyl-L-seryl-L-phenylalanyl-{2-R,S-amino-2-[2-(1,3-dithiolano)]}-acetyl-4S-amino-3S-hydroxy-5-cyclohexylpentanoyl-L-isoleucin-(2-picolyl)amid

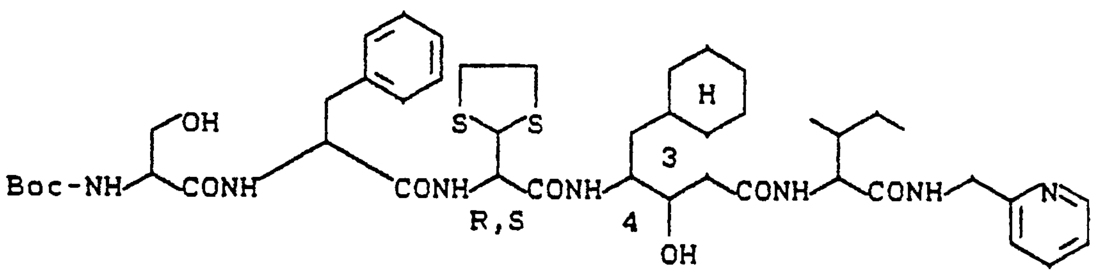

Die Titelverbindung wird analog Beispiel III aus N-tert.-Butoxycarbonyl-L-Serin und der Verbindung aus Beispiel XXXIV erhalten.

Analytische Daten:

DC-System II: $R_f$ = 0,16 (Isomer A)

$R_f$ = 0,14 (Isomer B)

HPLC-System II: $R_t$ = 5,04 (Isomer B)

$R_t$ = 5,70 (Isomer A)

(+)FAB-MS: m/z 914 (M+H); m/z 936 (M+Na).

Beispiel XXXVI

N-[3-(3-Pyridyl)propionyl]-L-phenylalanyl-[2-R-amino-2-[2-(1,3-dithiolano)]}-acetyl-4S-amino-3S-hydroxy-5-cyclohexylpentanoyl-L-isoleucin(2-picolyl)amid

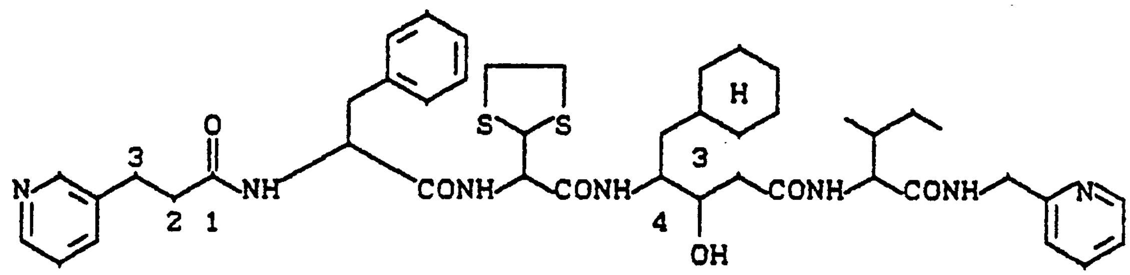

Die Titelverbindung wird analog Beispiel XXXV durch Propanphosphonsäureanhydridkupplung von 3-(3-Pyridyl)propionsäure mit dem durch Abspaltung der Boc-Schutzgruppe analog Beispiel XXXIV aus der R-Verbindung des Beispiels V zugänglichen Dihydrochlorids erhalten.

Analytische Daten:
DC-System III: $R_f$ = 0,47
HPLC-System II: $R_t$ = 6,36 min
(+)FAB-MS; m/z = 8,60 (M+H); m/z = 882 (M+Na)

## Beispiel XXXVII

N-tert.-Butoxycarbonyl-L-phenylalanyl-(2-R,S-amino-2-[2-(1,3-dithiolano)]}-acetyl-4S-amino-3S-hydroxy-5-cyclohexylpentanoyl-L-leucinmethylester

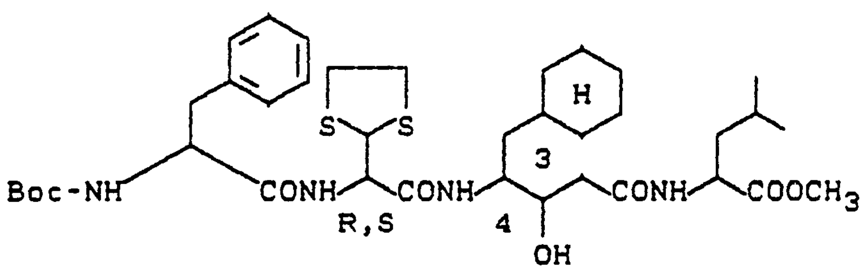

Die Titelverbindung wird analog Beispiel VIII erhalten, wobei zur Synthese der entsprechenden Verbindungen der Beispiele 6, 7, VI und VII der L-Leucinmethyl- statt des Ethylesters verwendet wird.

Analytische Daten:
DC-System I: $R_f$ = 0,85 (Isomer A, S-Isomer)
$R_f$ = 0,78 (Isomer B, R-Isomer)
(+)FAB-MS: m/z 751 (M+H); m/z 773 (M+Na); m/z 651.

## Beispiel XXXVIII

N-Ethoxycarbonyl-L-phenylalanyl-{2-R,S-amino-2-[2-(1,3-dithiolano)]}acetyl-4S-amino-3S-hydroxy-5-cyclohexylpentanoyl-L-leucinmethylester

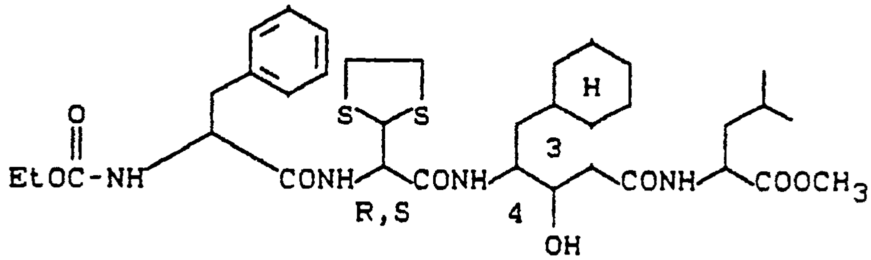

Die Titelverbindung wird analog Beispiel XXXI erhalten, wobei zur Synthese der entsprechenden Ausgangsverbindungen der Beispiele 6, 7, VI und VII der L-Leucinmethylstatt des Ethylesters verwendet wird.

Analytische Daten:.
DC-System I: $R_f$ = 0,81 (Isomer A, S-Isomer)
$R_f$ = 0,73 (Isomer B, R-Isomer)
HPLC-System II: $R_t$ = 11,66 min (Isomer B)
$R_t$ = 14,08 min (Isomer A)
(+)FAB-MS: m/z 723 (M+H); m/z 745 (M+Na).

Beispiel XXXIX und Beispiel XL

N-tert.-Butoxycarbonyl-L-phenylalanyl-{2-S-amino-2-[2-(1,3-dithiolano)]}acetyl-4S-amino-3S-hydroxy-5-cyclohexylpentanoyl-L-leucin-(3-aminomethyl)benzylamid (Beispiel XXXIX)

N-tert.-Butoxycarbonyl-L-phenylalanyl-{2-R-amino-2-[2-(1,3-dithiolano)]}acetyl-4S-amino-3S-hydroxy-5-cyclohexylpentanoyl-L-leucin-(3-aminomethyl)benzylamid (Beispiel XL)

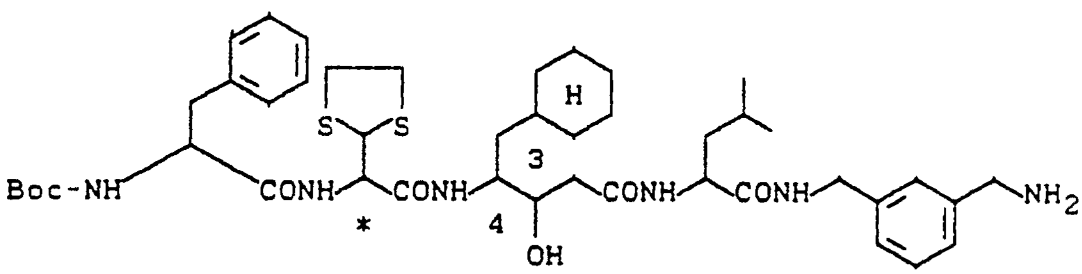

*S = Beispiel XXXIX (Isomer A)
*R = Beispiel XL (Isomer B)

0,95 g (1,29 mmol) der Verbindung aus Beispiel X und 2,50 ml (19,35 mmol) 1,3-Bisaminomethylbenzol werden in 30 ml $CH_2Cl_2$ gelöst. Der Ansatz wird auf -20° C gekühlt und mit 1,1 ml einer 50 %igen Lösung von n-Propylphosphonsäureanhydrid in $CH_2Cl_2$ (Hoechst AG) versetzt. Die Mischung wird über Nacht bei Raumtemperatur gerührt, mit 300 ml Diethylether versetzt und abgesaugt. Der Rückstand wird nochmals mit 300 ml Diethylether verrührt, abgesaugt und getrocknet. Das Rohprodukt (1,86 g) wird an 300 g Kieselgel 60 (Merck, Art.-Nr. 9385) 0,040-0,063 mm (230-400 mesh) mit einem Stufengradienten aus Methylenchlorid/Methanol/wäßriger $NH_3$-Lösung (25 %) 100/0/0; 99/1/0,2; 98/2/0,2; 97/3/0,2 und 95/5/0,2 (je 0,5 l) getrennt.

Zuerst eluiert das Beispiel XXXIX (Isomer A, S-Isomer), dann das Beispiel XL (Isomer B, R-Isomer).

Analytische Daten Isomer A (Beispiel XXXIX):
DC-System III: $R_f$ = 0,32
(+)FAB-MS: m/z 855 (M+H); m/z = 755
HPLC-System-II: keine Elution bzw. sehr breiter Peak.

Analytische Daten Isomer B (Beispiel XL):
DC-System III: $R_f$ = 0,29
(+)FAB-MS: m/z = 855 (M+H); m/z = 755
HPLC-System II: keine Elution bzw. sehr breiter Peak.

Beispiel XLI und Beispiel XLII

N-Ethoxycarbonyl-L-phenylalanyl-{2-S-amino-2-[2(1,3-dithiolano)]}acetyl-4S-amino-3S-hydroxy-5-cyclohexylpentanoyl-L-leucin-(3-aminomethyl)-benzylamid (Beispiel XLI)

N-Ethoxycarbonyl-L-phenylalanyl-{2-R-amino-2-[2-(1,3-dithiolano)]}acetyl-4S-amino-3S-hydroxy-5-cyclohexylpentanoyl-L-leucin-(3-aminomethyl)-benzylamid (Beispiel XLII)

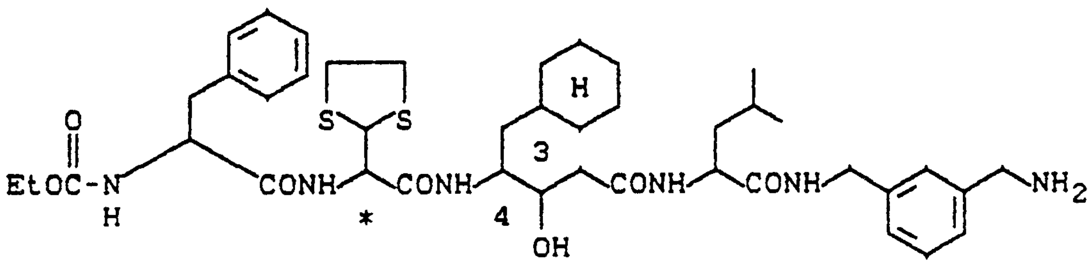

*S = Beispiel XLI (Isomer A)
*R = Beispiel XLII (Isomer B)

Die Titelverbindungen werden analog Beispiel XXXIX und Beispiel XL ausgehend von der Verbindung aus Beispiel XI erhalten.
Analytische Daten Isomer A (Beispiel XLI):
DC-System III: $R_f$ = 0,26
HPLC-System II: $R_t$ = 126 min
(+)FAB-MS: m/Z = 827 (M+H); 849 (M+Na).
Analytische Daten Isomer B (Beispiel XLII):
DC-System III: $R_f$ = 0,23
HPLC-System II: $R_t$ = 106 min
(+)FAB-MS: m/z = 827 (M+H); 849 (M+Na)

Beispiel XLIII und Beispiel XLIV

N-Ethoxycarbonyl-L-phenylalanyl-{2-S-amino-2-[2-(1,3-dithiolano)]}acetyl-4S-amino-3S-hydroxy-5-cyclohexylpentanoyl-L-isoleucinol (Beispiel XLIII)

N-Ethoxycarbonyl-L-phenylalanyl-{2-R-amino-2-[2-(1,3-dithiolano)]}acetyl-4S-amino-3S-hydroxy-5-cyclohexylpentanoyl-L-isoleucinol (Beispiel XLIV)

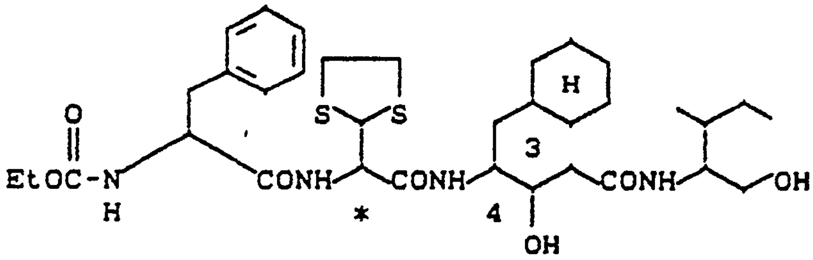

*S = Isomer A (Beispiel XLIII)
*R = Isomer B (Beispiel XLIV)

Die Titelverbindungen werden analog Beispiel 3 oder Beispiel I durch n-Propanphosphonsäureanhydridkopplung der Verbindung aus Beispiel XXIV mit L-Isoleucinol Hydrochlorid als Aminkomponente erhalten. Die Reindarstellung der Stereoisomeren erfolgt durch Kieselgelchromatographie (analog Beispiel IV und V) bzw. wahlweise auch durch reversed-Phase HPLC an $C_8$-Phasen (Säule z.B. Merck Hibar, Cat.-Nr. 51441, LiChrosorb, RP-8 (7 µm), Größe 250-25) mit Acetonitril/Wasser-Gemischen. Produktenthaltende Fraktionen der Kieselgelchromatographie werden vereinigt und einrotiert, bzw. bei der reversed-Phase Chromatographie vom Acetonitril abrotiert und lyophilisiert.

Analytische Daten Isomer A (S-Isomer, Beispiel XLIII):
DC-System I: $R_f$ = 0,54
DC-System II: $R_f$ = 0,44
DC-System IV: $R_f$ = 0,64
HPLC-System: $R_t$ = 7,10 min
(+)FAB-MS: m/z = 695 (M+H); m/z = 717 (M+Na)

Analytische Daten Isomer B (R-Isomer, Beispiel XLIV):
DC-System I: $R_f$ = 0,54
DC-System II: $R_f$ = 0,42
DC-System IV $R_f$ = 0,64
HPLC-System II: $R_t$ = 5,71 min
(+)FAB-MS: m/z = 695 (M+H); m/z = 717 (M+Na)

Beispiel XLV und Beispiel XLVI

N-Ethoxycarbonyl-L-phenylalanyl-{2-S-amino-2-[2-(1,3-dithiolano)]}acetyl-4S-amino-3S-hydroxy-5-cyclohexylpentanoyl-L-isoleucin-isopropylester (Beispiel XLV)

N-Ethoxycarbonyl-L-phenylalanyl-{2-R-amino-2-[2-(1,3-dithiolano]]}acetyl-4S-amino-3S-hydroxy-5-cyclohexylpentanoyl-L-isoleucin-isopropylester (Beispiel XLVI)

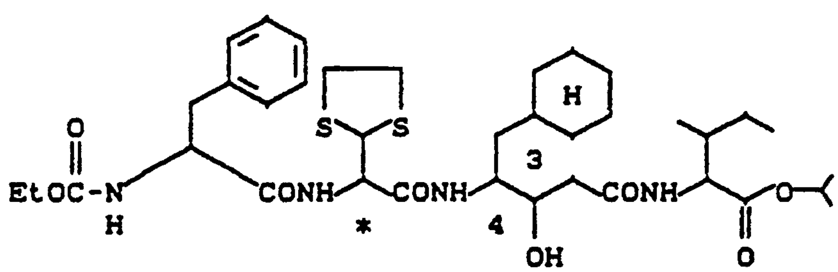

*S = Isomer A (Beispiel XLV)
*R = Isomer B (Beispiel XLVI)

Die Titelverbindungen werden analog Beispiel 3 oder 1 durch n-Propanphosphonsäureanhydridkopplung der Verbindung aus Beispiel XXIV mit L-Isoleucin-isopropylester Hydrochlorid als Aminkomponente hergestellt. Die Reindarstellung der Stereoisomeren erfolgt durch Chromatographie (wie in Beispiel XLIII und XLIV beschrieben).

Analytische Daten Isomer A (S-Isomer, Beispiel XLV):
DC-System I: $R_f$ = 0,68
DC-System II: $R_f$ = 0,57
HPLC-System II: $R_t$ = 27,61 min
(+)FAB-MS: m/z = 751 (M+H); m/z = 773 (M+Na)

Analytische Daten Isomer B (R-Isomer, Beispiel XLVI):
DC-System I: $R_f$ = 0,68
DC-System II: $R_f$ = 0,57
HPLC-System II: $R_t$ = 22,51 min
(+)FAB-MS: m/z = 751 (M+H); m/z = 773 (M+Na)

Beispiel XLVII und Beispiel XLVIII

N-Ethoxycarbonyl-L-phenylalanyl-{2-S-amino-2-[2-(1,3-dithiolano)]}acetyl-4S-amino-3S-hydroxy-5-cyclohexylpentanoyl-L-isoleucin-L-valin-(2-picolyl)amid (Beispiel XLVII)

N-Ethoxycarbonyl-L-phenylalanyl-{2-R-amino-2-[2-(1,3-dithiolano)]}acetyl-4S-amino-3S-hydroxy-5-cyclohexylpentanoyl-L-isoleucin-L-valin-(2-picolyl)amid (Beispiel XLVIII)

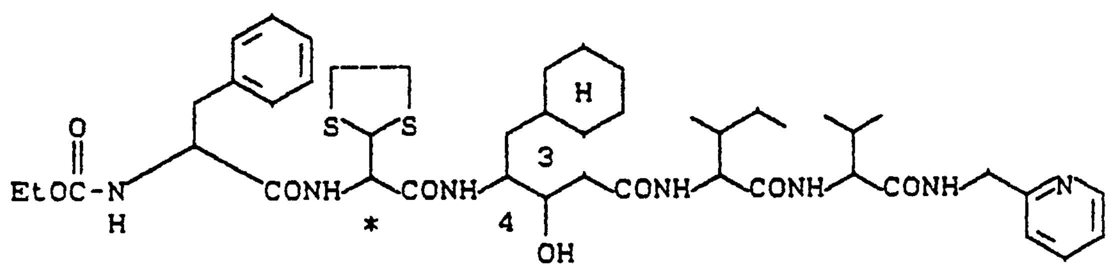

*S = Isomer A (Beispiel XLVII)
*R = Isomer B (Beispiel XLVIII)

Die Titelverbindungen werden analog Beispiel 3 oder I durch n-Propanphosphonsäureanhydridkopplung der Verbindung aus Beispiel XXIV mit L-Isoleucyl-L-valin-(2-picolyl)amid Dihydrochlorid als Aminkomponente hergestellt. Die Reindarstellung der Stereoisomeren erfolgt durch Chromatographie (wie in Beispiel XLIII und XLIV beschrieben).

Analytische Daten Isomer A (S-Isomer, Beispiel XLVII):
DC-System I: $R_f$ = 0,52
DC-System II: $R_f$ = 0,40
HPLC-System II: $R_t$ = 9,48 min
(+)FAB-MS: m/z = 898 (M+H)

Analytische Daten Isomer A (R-Isomer, Beispiel XLVIII):
DC-System I: $R_f$ = 0,52
DC-System II: $R_f$ = 0,40
HPLC-System II: $R_t$ = 8,05 min
(+)FAB-MS: m/z = 898 (M+H)

Beispiel XLIX und Beispiel L

N-Ethoxycarbonyl-L-phenylalanyl-{2-S-amino-2-[2-(1,3-dithiolano)]}acetyl-4S-amino-3S-hydroxy-5-cyclohexylpentansäure-(2-methyl)propylamid (Beispiel XLIX)

N-Ethoxycarbonyl-L-phenylalanyl-{2-R-amino-2-[2-(1,3-dithiolano)]}acetyl-4S-amino-3S-hydroxy-5-cyclohexylpentansäure-(2-methyl)propylamid (Beispiel L)

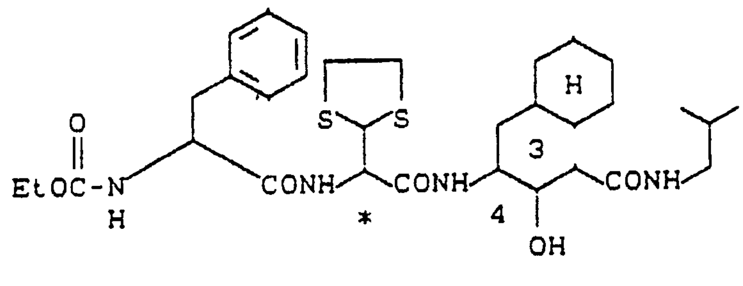

*S = Isomer A (Beispiel XLIX)
*R = Isomer B (Beispiel L)

Die Titelverbindungen werden analog Beispiel 3 oder I durch n-Propanphosphonsäureanhydridkopplung der Verbindung aus Beispiel XXIV mit (2-Methyl)-propylamin als Aminkomponente hergestellt. Die Reindarstellung der Stereoisomeren erfolgt durch Chromatographie (wie in Beispiel XLIII und XLIV) beschrieben).

Analytische Daten Isomer A (S-Isomer, Beispiel XLIX):
DC-System I: $R_f$ = 0,66
HPLC-System II: $R_t$ = 8,84 min
(+)FAB-MS: m/z = 657 (M+Li)

Analytische Daten Isomer B (R-Isomer, Beispiel L):
DC-System I: $R_f$ = 0,66
HPLC-System II: $R_t$ = 7,74 min
(+)FAB-MS: m/z = 657 (M+Li)

Beispiel LI

N-Ethoxycarbonyl-L-phenylalanyl-{2-R,S-amino-2-{2-(1,3-dithiano)]}acetyl-4S-amino-3S-hydroxy-5-cyclohexylpentanoyl-L-isoleucin-(2-picolyl)amid

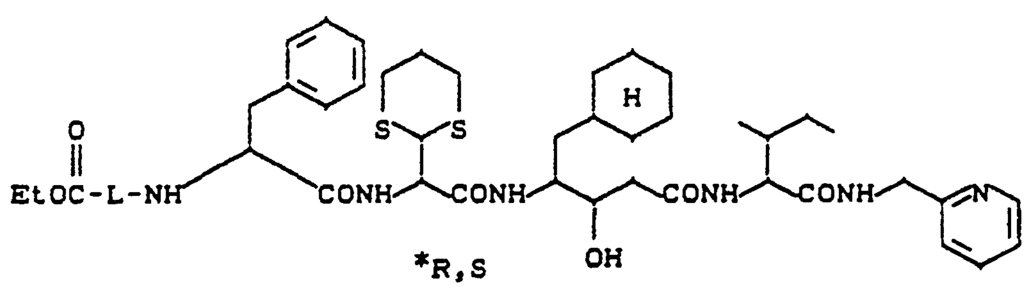

Die Titelverbindung wird analog Beispiel 3 oder 2 durch n-Propanphosphonsäureanhydridkopplung der Verbindung aus Beispiel 17 mit der Verbindung aus Beispiel 3 als Aminkomponente hergestellte.

Analytische Daten:

**DC-System II: $R_f$ = 0,32**
**HPLC-System II: $R_r$ = 8,34 min** } **beide Isomere ununterscheidbar**

(+)FAB-MS: m/z = 813 (M+H); m/z 835 (M+Na)

Beispiel LII und Beispiel LIII

N-tert.-Butoxycarbonyl-L-(4-methoxy)phenylalanyl-[2-S-amino-2-[2-(1,3-dithiolano)]}acetyl-4S-amino-3S-hydroxy-5-cyclohexylpentansäure-(morpholin)amid (Beispiel LII)

N-tert.-Butoxycarbonyl-L-(4-methoxy)phenylalanyl-[2-R-amino-2-[2-(1,3-dithiolano)]}acetyl-4S-amino-3S-hydroxy-5-cyclohexylpentansäure-(morpholin)amid (Beispiel LIII)

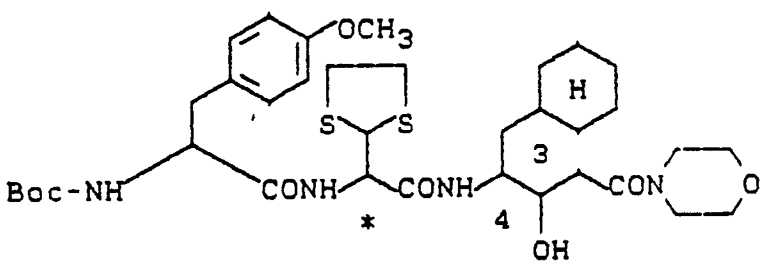

*S = Isomer A (Beispiel LII)
*R = Isomer B (Beispiel LIII)

Die Titelverbindungen werden analog Beispiel 3 oder I durch n-Propanphosphonsäureanhydridkopplung der Verbindung aus Beispiel XXV mit Morpholin als Aminkomponente hergestellt. Die Reindarstellung der Stereoisomeren erfolgt durch Chromatographie (wie in Beispiel XLIII und XLIV beschrieben).

Analytische Daten Isomer A (S-Isomer, Beispiel LII):
DC-System II: $R_f$ = 0,39
HPLC-System II: $R_t$ = 7,78 min
(+)FAB-MS: m/z = 723 (M+H); m/z = 729 (M+Li); m/z = 745 (M+Na)

Analytische Daten Isomer B (R-Isomer, Beispiel LIII):
DC-System II: $R_f$ = 0,36
HPLC-System II: $R_t$ = 7,07 min
(+)FAB-MS: m/z = 723 (M+H); m/z = 729 (M+Li); m/z = 745 (M+Na)

Beispiel LIV und Beispiel LV

N-tert.-Butoxycarbonyl-L-(4-methoxy)phenylalanyl-{2-S-amino-2-[2-(1,3-dithiolano)]}acetyl-4S-amino-3S-hydroxy-5-cyclohexylpentansäure-(4-methyl)butylamid (Beispiel LIV)

N-tert.-Butoxycarbonyl-L-(4-methoxy)phenylalanyl-{2-R-amino-2-[2-(1,3-dithiolano)]}acetyl-4S-amino-3S-hydroxy-5-cyclohexylpentansäure-(4-methyl)butylamid (Beispiel LV)

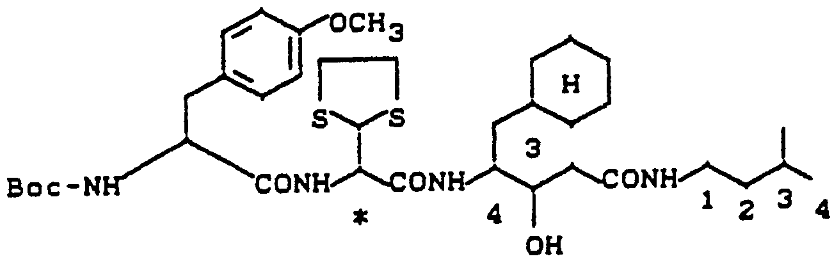

*S = Isomer A (Beispiel LIV)
*R = Isomer B (Beispiel LV)

Die Titelverbindungen werden analog Beispiel 3 oder I durch n-Propanphosphonsäureanhydridkopplung der Verbindung aus Beispiel XXV mit 3-Methylbutylamin als Aminkomponente hergestellt. Die Reindarstellung der Stereoisomeren erfolgt durch Chromatographie (wie in Beispiel XLIII und XLIV beschrieben).

Analytische Daten Isomer A (S-Isomer, Beispiel LIV):
DC-System II: $R_f$ = 0,38
HPLC-System II: $R_t$ = 21,14 min
(+)FAB-MS: m/z = 729 (M+Li)

Analytische Daten Isomer B (R-Isomer, Beispiel LV):
DC-System II: $R_f$ = 0,35
HPLC-System II: $R_t$ = 21,14 min
(+)FAB-MS: m/z = 729 (M+Li)

Beispiel LVI und Beispiel LVII

N-tert.-Butoxycarbonyl-L-(4-methoxy)phenylalanyl-{2-S-amino-2-[2-(1,3-dithiolano)]}acetyl-4S-amino-3S-hydroxy-5-cyclohexylpentansäure-n-hexylamid (Beispiel LVI)

N-tert.-Butoxycarbonyl-L-(4-methoxy)phenylalanyl-{2-R-amino-2-[2-(1,3-dithiolano)]}acetyl-4S-amino-3S-hydroxy-5-cyclohexylpentansäure-n-hexylamid (Beispiel LVII)

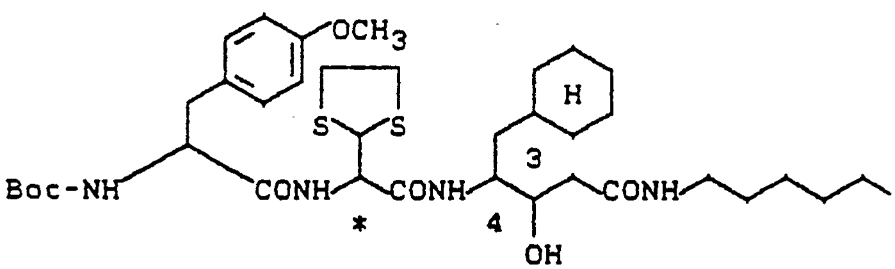

*S = Isomer A (Beispiel LVI)
*R = Isomer B (Beispiel LVII)

Die Titelverbindungen werden analog Beispiel 3 oder I durch n-Propanphosphonsäureanhydridkopplung der Verbindung aus Beispiel XXV mit n-Hexylamin-Hydrochlorid als Aminkomponente hergestellt. Die Reindarstellung der Stereoisomeren erfolgt durch Chromatographie (wie in Beispiel XLIII und XLIV beschrieben).

Analytische Daten Isomer A (S-Isomer, Beispiel LVI):
DC-System II: $R_f$ = 0,45
HPLC-System II: $R_t$ = 34,11 min
(+)FAB-MS: m/z = 737 (M+H); m/z = 743 (M+Li); m/z = 759 (M+Na)

Analytische Daten Isomer B (R-Isomer, Beispiel LVII):
DC-System II: $R_f$ = 0,41
HPLC-System II: $R_t$ = 34,11 min
(+)FAB-MS: m/z = 737 (M+H); m/z = 743 (M+Li); m/z = 759 (M+Na)

Beispiel LVIII und Beispiel LIX

N-tert.-Butoxycarbonyl-L-(4-methoxy)phenylalanyl-{2-S-amino-2-[2-(1,3-dithiolano)]}acetyl-4S-amino-3S-hydroxy-5-cyclohexylpentansäure-(N-benzylpiperazin)amid (Beispiel LVIII)

N-tert.-Butoxycarbonyl-L-(4-methoxy)phenylalanyl-{2-R-amino-2-[2-(1,3-dithiolano)]}acetyl-4S-amino-3S-hydroxy-5-cyclohexylpentansäure-(N-benzylpiperazin)amid (Beispiel LIX)

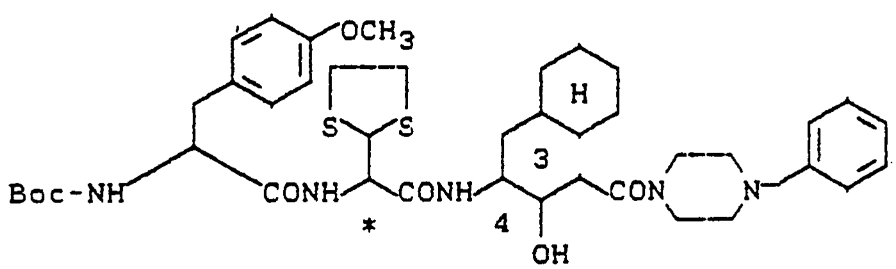

*S = Isomer A (Beispiel LVIII)
*R = Isomer B (Beispiel LIX)

Die Titelverbindungen werden analog Beispiel 3 oder I durch n-Propanphosphonsäureanhydridkopplung der Verbindung aus Beispiel XXV mit N-Benzylpiperazin als Aminkomponente hergestellt. Die Reindarstellung der Stereoisomeren erfolgt durch Chromatographie (wie in Beispiel XLIII und XLIV beschrieben).
Analytische Daten Isomer A (S-Isomer, Beispiel LVIII):
DC-System II: $R_f$ = 0,44
HPLC-System II: $R_t$ = 31,46 min
(+)FAB-MS: m/z = 818 (M+Li)
Analytische Daten Isomer B (R-Isomer, Beispiel LIX):
DC-System II: $R_f$ = 0,44
HPLC-System II: $R_t$ = 29,07 min
(+)FAB-MS: m/z = 818 (M+Li)

Beispiel LX

Methyl-2-R-amino-2-[2-(1,3-dithiolano)]-acetyl-4S-amino-3S-hydroxy-5-cyclohexylpentanoate

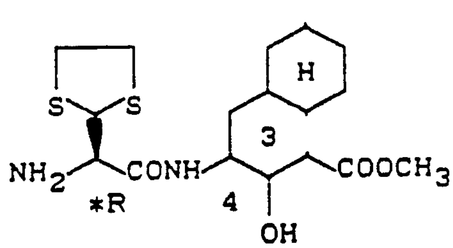

Die Titelverbindung wird durch chromatographische Trennung der Isomerenmischung (freie Base) aus dem Beispiel XIX an Kieselgel mit einem Stufengradienten aus Methylenchlorid/Methanol/25 %iger wäßriger Ammoniaklösung (wie in Beispiel XXXIX und XL beschrieben) erhalten Zuerst eluiert das S-Isomere, dann das Beispiel LX (R-Isomeres)

Analytische Daten:
DC-System III: $R_f$ = 0,34
(+)FAB-MS: m/z = 391 (M+H); m/z = 397 (M+Li).

Beispiel LXI

Methyl-N-tert.-butoxycarbonyl-2-R-amino-2-[2-(1,3-dithiolano)]-acetyl-4S-amino-3S-hydroxy-5-cyclohexylpentanoate

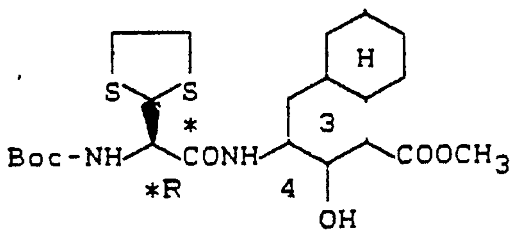

Die Titelverbindung wird analog Beispiel 5 durch Umsetzung der Verbindung aus dem Beispiel LX mit Di-tert.-butylcarbonat mit Triethylamin als Hilfsbase und Dioxan als Lösungsmittel (analog Beispiel 14) erhalten. Die Reaktionsmischung wird mit Wasser und 1N Salzsäure verdünnt und mit Methylenchlorid extrahiert. Die organische Phase wird abgetrennt, über Natriumsulfat getrocknet und eingeengt. Das Produkt wird durch Flash-Chromatographie (Kieselgel/Dichlormethan) gereinigt.

Analytische Daten:
DC-System IV: $R_f$ = 0,88
HPLC-System II: $R_t$ = 7,06 min
(+)FAB-MS: m/z = 497 (M+Li)

Beispiel LXII

N-tert.-Butoxycarbonyl-2-R-amino-2-[2-(1,3-dithiolano)]-acetyl-4S-amino-3S-hydroxy-5-cyclohexyl-pentansäure

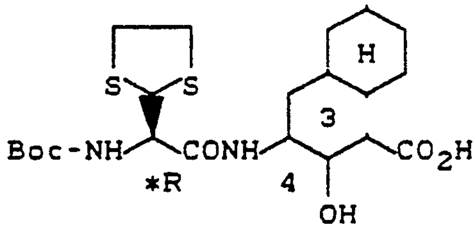

Die Titelverbindung wird durch basische Verseifung analog Beispiel X oder Beispiel XI aus 2,1 g (4,28 mmol) der Verbindung aus dem Beispiel LXI und Standardaufarbeitung erhalten.
Ausbeute: 1,95 g (95,6 % der Theorie)
DC-System IV: $R_f$ = 0,57
(+)FAB-MS: m/z = 477 (M+H), m/z 421, m/z 377.

Beispiel LXIII

N-tert.-Butoxycarbonyl-2-R-amino-2-[2-(1,3-dithiolano)]-acetyl-4S-amino-3S-hydroxy-5-cyclohexyl-pentanoyl-L-leucinol

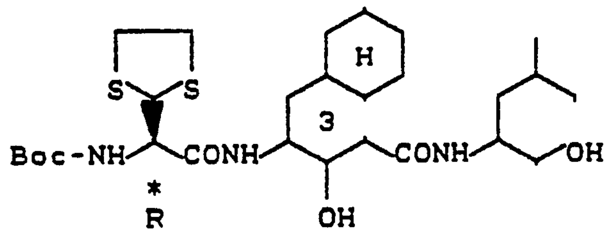

Die Titelverbindung wird analog Beispiel 3 oder I durch Propanphosphonsäureanhydridkupplung der Verbindung aus Beispiel LXII und L-Leucinol-hydrochlorid als Aminkomponente erhalten. Das Produkt wird chromatographisch gereinigt (analog Beispiel IV und Beispiel V)

Analytische Daten des Beispiels 63:

DC-System III: $R_f$ = 0,65

HPLC-System II: $R_t$ = 6,58 min

(+)FAB-MS: m/z = 582 (M+Li).

Beispiel LXIV

N-tert.-Butoxycarbonyl-L-(4-methoxy)phenylalanyl-{2-R-amino-2-[2-[1,3-dithiolano)]}acetyl-4S-amino-3S-hydroxy-5-cyclohexylpentanoyl-L-leucinol

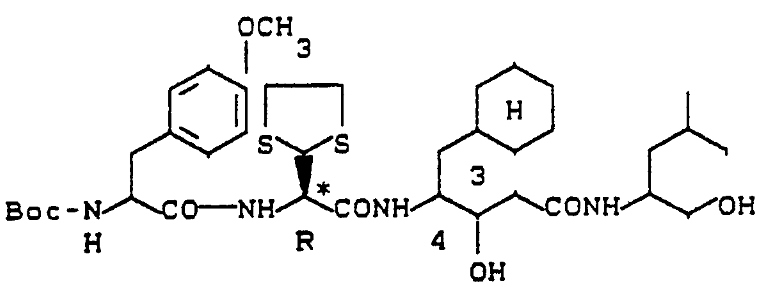

Die Titelverbindung wird ausgehend von der Verbindung des Beispiels LXIII durch a) Entfernen der Boc-Schutzgruppe (analog Beispiel 4) und b) Umsetzung des erhaltenen Hydrochlorids mit Boc-L-(4-Methoxy)phenyl-alanin (analog Beispiel XVI und XVII) hergestellt. Das Produkt wird durch Chromatographie gereinigt.

Analytische Daten:

DC-System IV: $R_f$ = 0,59

HPLC-System II: $R_t$ = 12,85 min

(+)FAB-MS: m/z = 759 (M+Li).

Beispiel LXV

2-R-Amino-2-(1,3-dithiolano)]-acetyl-4S-amino-3S-hydroxy-5-cyclohexyl-pentanoyl-L-isoleucine-(2-picolyl)-amid

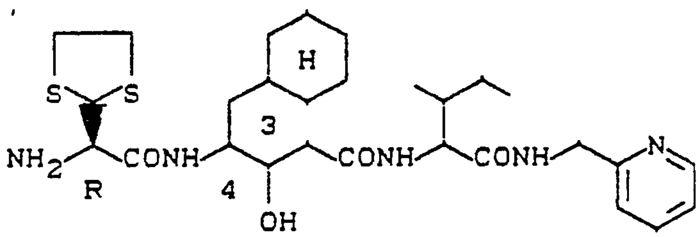

Die Titelverbindung wird analog Beispiel LX ausgehend vom R,S-Gemisch der Verbindung aus dem Beispiel II durch Chromatographie erhalten. Zuerst eluiert das S-, dann das R-Isomer (Beispiel LXV).
Analytische Daten:
DC-System III: $R_f$ = 0,33
(+)FAB-MS: m/z = 580 (M+H), m/z 602 (M+Na).

Beispiel LXVI

L-(4-Methoxy)phenylalanyl-{2-R-amino-2-[2-(1,3-dithiolano)]}acetyl-4S-amino-3S-hydroxy-5-cyclohexylpentanoyl-L-isoleucine-(2-picolyl)amid-dihydrochlorid

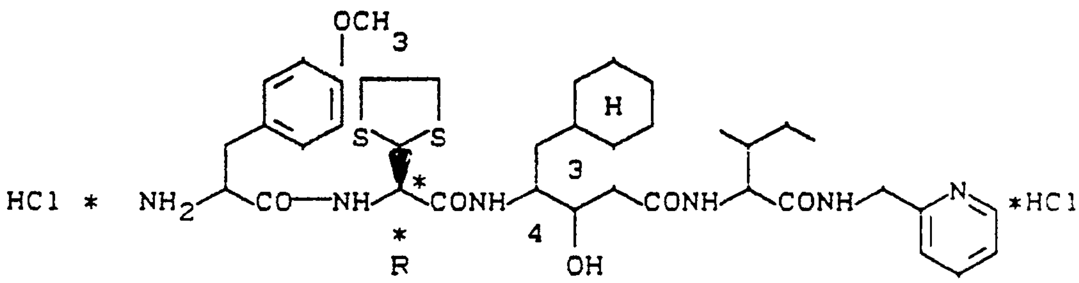

Die Titelverbindung wird analog Beispiel XXXIV durch Abspaltung der Boc-Schutzgruppe aus der Verbindung des Beispiels XVII erhalten.
Analytische Daten:
DC-System III: $R_f$ = 0,38

Beispiel LXVII

L-(4-Methoxy)phenylalanyl-{2-R,S-amino-2-[2-(1,3-dithiolano)]}acetyl-4S-amino-3S-hydroxy-5-cyclohexylpentanoyl-L-isoleucine-(2-picolyl)amid-dihydrochlorid

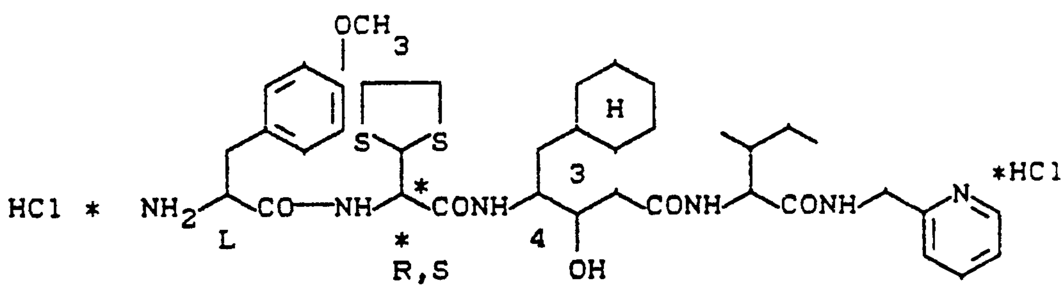

Die Titelverbindung wird analog Beispiel XXXIV durch Abspaltung der Boc-Schutzgruppe aus einer

Mischung der Verbindungen der Beispiele XVI und XVII erhalten.
Analytische Daten:
DC-System III: $R_f$ = 0,42;
$R_f$ = 0,38.

Beispiele LXVIII bis LXXVI (Tabelle 1)

Die in Tabelle 1 aufgeführten Beispiele werden analog Beispiel 3 oder 1 durch Propanphosphorsäureanhydridkopplung der Verbindung aus dem Beispiel XXV mit den jeweiligen Aminkomponenten analog zu den Beispielen LII bis LIX hergestellt. Die Reinigung und/oder Trennung der Stereoisomeren erfolgt durch Chromatographie (wie in den Beispielen XLIII und XLIV beschrieben).

Tabelle 1

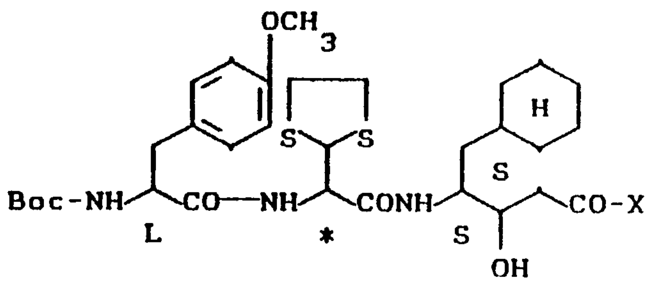

| Beispiel | Konfiguration * | X | DC-System $R_f$-Wert | HPLC-System II $R_t$-Wert | (+)FAB-MS |
|---|---|---|---|---|---|
| LXVIII | R | 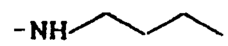 | II: 0,33 | 16,21 min | m/z 709 (M+H)<br>m/z 715 (M+Li) |
| LXIX | S | 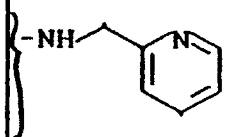 | III: 0,59 | 8,70 min | m/z 744 (M+H) |
| LXX | R | | III: 0,54 | 8,59 min | m/z 766 (M+Na) |
| LXXI | S | 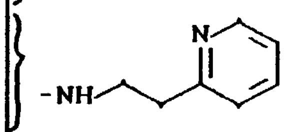 | III: 0,58 | 10,08 min | m/z 758 (M+H) |
| LXXII | R | | III: 0,51 | 9,65 min | m/z 758 (M+H) |
| LXXIII | R | 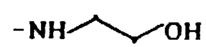 | I: 0,51 | 4,34 min | m/z 697 (M+H)<br>m/z 719 (M+H) |
| LXXIV | S | 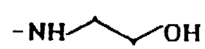 | I: 0,52 | 4,67 min | m/z 697 (M+H)<br>m/z 719 (M+H) |

**Tabelle 1** (Fortsetzung)

| Beispiel | Konfiguration * | X | DC-System $R_f$-Wert | HPLC-System II $R_t$-Wert | (+)FAB-MS |
|---|---|---|---|---|---|
| LXXV | R | 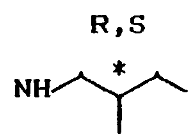 | IV: 0,71 | 23,04 min | m/z 723 (M+H) |
| LXXVI | R,S | 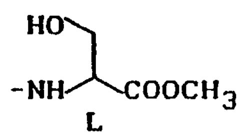 | I: 0,63<br>I: 0,60 | 5,60 min<br>6,00 min | m/z 755 (M+H)<br>m/z 777 (M+Na) |

Beispiele LXXVII bis LXXXI (Tabelle 2)

Die in Tabelle 2 aufgeführten Beispiele werden analog Beispiel XXXVI durch Umsetzen der Dihydrochloride aus den Beispielen LXVI und/oder LXVII mit den entsprechenden Säuren unter Verwendung von Propanphosphonsäureanhydrid als Kopplungsreagenz erhalten. Die Reinigung wird durch Chromatographie durchgeführt.

Tabelle 2

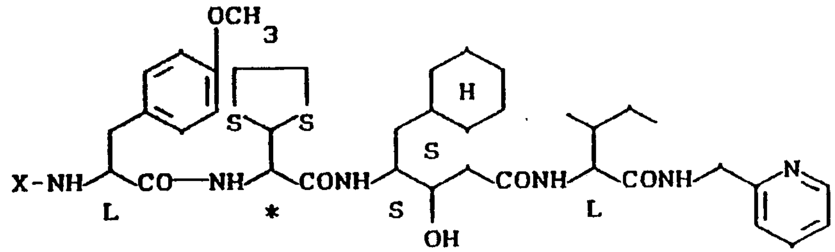

| Beispiel | X | Konfiguration* | DC-System $R_f$-Wert | HPLC-System II $R_t$-Wert | (+)FAB-MS |
|---|---|---|---|---|---|
| LXXVII | 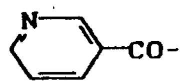 | R | I: 0,51 | 4,03 min | m/z 862 (M+H) |
| LXXVIII | 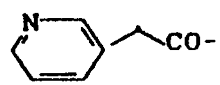 | R | I: 0,47 | 6,37 min | m/z 876 (M+H) |
| LXXIX | 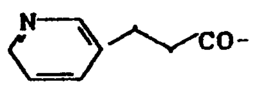 | R | I: 0,50 | 9,85 min | m/z 890 (M+H) |
| LXXX | 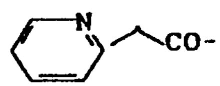 | R | III: 0,37 | 5,11 min | m/z 876 (M+H) |
| LXXXI | 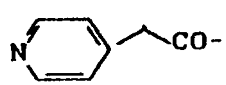 | R | II: 0,26 | 5,34 min | m/z 876 (M+H) |

Beispiele LXXXII bis LXXXVI (Tabelle 3)

Die in Tabelle 3 aufgeführten Beispiele werden durch Propanphosphonsäureanhydridkopplung der entsprechenden Boc-geschützten Aminosäuren (Bissendorf Biochemicals GmbH) mit der Verbindung aus

dem Beispiel LXV (analog Beispiel XVI und XVII und chromatographischer Reinigung erhalten.

Tabelle 3

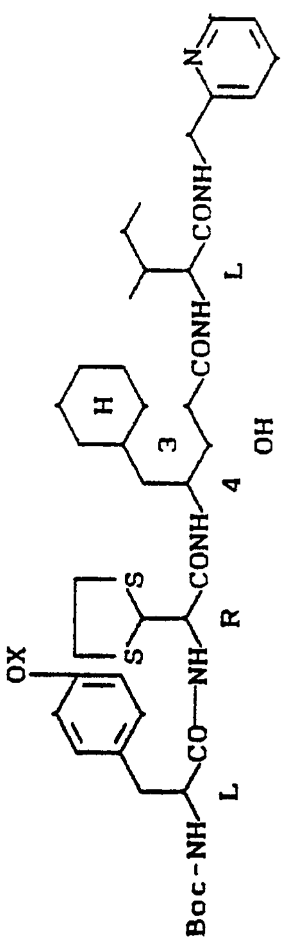

| Beispiel | X | DC-System $R_f$-Wert | HPLC-System II $R_t$-Wert | (+)FAB-MS |
|---|---|---|---|---|
| LXXXII | 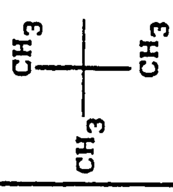 | IV: 0,73 | 26,35 min | m/z 905 (M+Li) |
| LXXXIII | 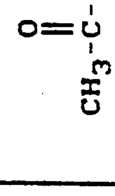 | IV: 0,68 | 10,36 min | m/z 891 (M+Li) |
| LXXXIV | $H_5C_2$- | IV: 0,74 | 17,63 min | m/z 877 (M+Li) |
| LXXXV | 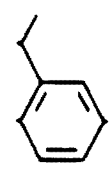 | IV: 0,75 | 38,91 min | m/z 933 (M+H); m/z 939 (M+Li) |
| LXXXVI | H- | I: 0,69 | 5,31 min | |

Beispiele LXXXVII bis XIC (Tabelle 4)

Die in Tabelle 4 aufgeführten Beispiele werden entweder durch Kopplung der entsprechenden Säuren (Beispiele LXXXVII bis IXC) mit der Verbindung aus dem Beispiel LXV unter Verwendung von n-Propanphosphonsäureanhydrid oder durch Umsetzung der Verbindung aus dem Beispiel LXV mit 'Z-OSO'

in Gegenwart von Triethylamin (Beispiel XC) hergestellt. Nach Standardaufarbeitung werden alle Verbindungen durch Chromatographie gereinigt.

Die Säuren werden nach bekannten Synthesemethoden hergestellt. Die Synthese der Säure, die für die Herstellung des Beispiels LXXXVII verwendet wird, ist im Beispiel 19 beschrieben.

Die Synthesen der Säuren für die Beispiele LXXXVIII und IXC sind woanders beschrieben (LXXXVIII: J.J. Plattner et.al., J. Med.Chem 1988, 31, 2277-2288; IXC: P. Buehlmayer et.al., J. Med.Chem 1988, 31, 1839-1846); Z-OSO' [(N-Benzyloxycarbonyloxy)-succinimid] ist kommerziell erhältlich (Aldrich Chemicals).

**Tabelle 4**

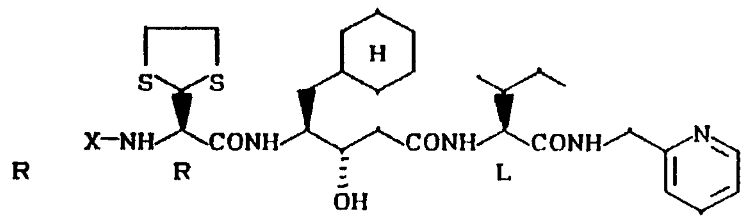

| Beispiel | X | DC-System $R_f$-Wert | HPLC-System II $R_t$-Wert | (x)FAB-MS |
|---|---|---|---|---|
| LXXXVII | 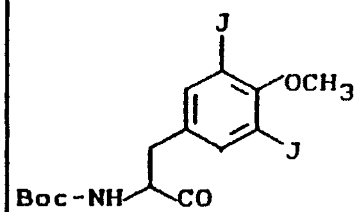 | I: 0,53 | 38,58 min | m/z 1115 (M+Li) |
| LXXXVIII | 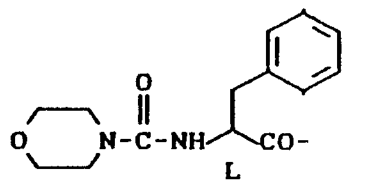 | IV: ≈0,63 | 4,79 min | m/z 840 (M+H)<br>m/z 862 (M+Na) |
| IXC | 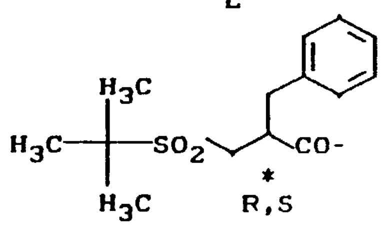 | 0,56;<br>III: 0,51 | 7,95 min;<br>12,14 min | m/z 846 (M+H);<br>m/z 868 (M+Na) |
| XC | 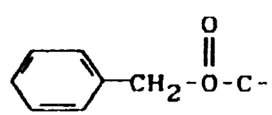 | I: 0,70 | 7,95 min | |

Erklärungen zum experimentellen Teil:

DC-Systeme:

Stationäre Phase

Merk DC-Fertigplatten Kieselgel 60 F-254, 5 x 10 cm, Schichtdicke 0,25 mm, Art.-Nr. 5719.

| Mobile Phasen (im Test als "DS-System") | |
|---|---|
| I : $CH_2Cl_2$/MeOH | 9:1 |
| II : $CH_2Cl_2$/MeOH | 95:5 |
| III : $NH_3$/$CH_2Cl_2$/MeOH | 0,2:9:1 |
| IV : HOAc/$CH_2Cl_2$/MeOH | 0,2:9:1 |
| V : Eisessig/n-Butanol/$H_2O$ | 1:3:1 |
| VI : EtOAc/n-Hexan | 2:1 |
| VII : EtOAc/n-Hexan | 1:3 |
| VIII : EtOAc/n-Hexan | 1:1 |
| IX : $CH_2Cl_2$/MeOH | 98:2 |
| X : $CH_2Cl_2$/MeOH | 7:3 |
| XI : $CH_2Cl_2$ | |

HPLC-Systeme:

HPLC-System I : Säule Merck Lichrosorb® RP-8, 250-4, 10 µm, Kat.-No 50318
HPLC-System II : Säule Merck Lichrosorb® RP-18, 250-4, 10 µm, Kat.-No 50334
Eluens bei System I und II
A: pH 7,00 Phosphatpuffer, Merck, Art.-Nr. 9439/$H_2O$ 1:50
B: Acetonitril
A/B wie 1/1, Fluß: 2 ml/min, isokratisch,
Detektion: 254 nm

Verzeichnis der benutzten Abkürzungen

1. allgemeine analytische Methoden

DC Dünnschichtchromatographie
PDC präparative Dickschichtchromatographie
GC Gaschromatographie
HPLC Hochdruckflüssigkeitschromatographie
SC Säulenchromatographie
NMR Kernspinresonanzspektroskopie (Protonen)
MS Massenspektrometrie (Elektronenstoßionisation)
(+)FAB-MS Fast-atomic-bombardement-Massenspektrometrie, positive Ionen, Matrixsubstanz: m-Nitrobenzylalkohol
MS-DCI Massenspektrometrie, chemische Ionisation

2. Schutzgruppen

Boc Tert-Butoxycarbonyl
Z Benzyloxycarbonyl
DNP Dinitrophenyl
Fmoc 9-Fluorenylmethoxycarbonyl
OEt Ethylester
OMe Methylester

EtOC Ethoxycarbonyl

## Ansprüche

1. Peptide der allgemeinen Formel (I)

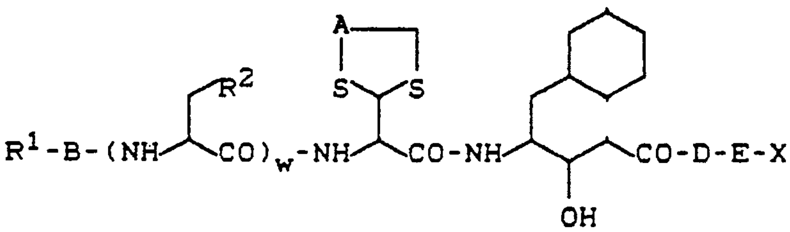 (I)

in welcher
$R^1$ - für Wasserstoff oder
- für Alkoxycarbonyl mit bis zu 8 Kohlenstoffatomen oder Benzyloxycarbonyl steht oder
- für eine Gruppe der Formel $R^3$-CO- steht
worin
$R^3$ - Morpholino oder die Reste

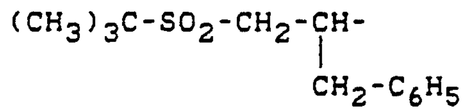

oder 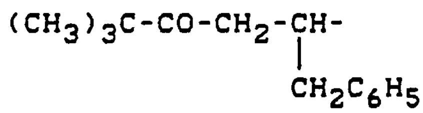

bedeutet
oder
-eine Gruppe der Formel $-NR^4R^5$ bedeutet
worin
$R^4$ und $R^5$ gleich oder verschieden sind und Wasserstoff, geradkettiges oder verzweigtes Alkyl mit bis zu 8 Kohlenstoffatomen oder Phenyl bedeuten
oder
- den Rest

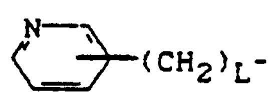

bedeutet
worin
L die Zahl 0, 1 oder 2 bedeutet
B - für eine direkte Bindung oder
- für eine Aminosäuregruppierung der Formel

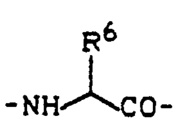 oder 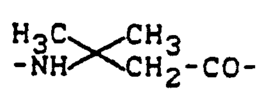

steht
worin
$R^6$ - geradkettiges oder verzweigtes Alkyl mit bis zu 8 Kohlenstoffatomen bedeutet, das gegebenenfalls durch Hydroxy, Carboxy, Alkoxy oder Alkoxycarbonyl mit bis zu 6 Kohlenstoffatomen oder Benzyloxy substituiert ist
oder
- für Prolin steht
in ihrer L-Form, D-Form oder als D,L-Isomerengemisch
$R^2$ - für Aryl mit 6 bis 10 Kohlenstoffatomen steht, das gegebenenfalls bis zu 3-fach gleich oder verschieden durch Halogen, Nitro, Cyano, Hydroxy, geradkettiges oder verzweigtes Alkoxy mit bis zu 8 Kohlenstoffatomen, Acetoxy, Benzyloxy oder durch eine Gruppe der Formel O-CO-$R^7$ substituiert ist
worin
$R^7$ geradkettiges oder verzweigtes Alkyl mit bis zu 8 Kohlenstoffatomen bedeutet
w - eine Zahl 0 oder 1 bedeutet
A - für eine $-CH_2-$ oder für eine $CH_2-CH_2$-Gruppe steht,
D und E gleich oder verschieden sind und
- für eine direkte Bindung oder
- für eine Aminosäuregruppierung der Formel

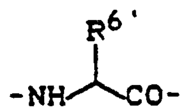

stehen
worin
$R^{6'}$ die oben angegebene Bedeutung von $R^6$ hat und mit dieser gleich oder verschieden ist
in ihrer L-Form, D-Form oder als D,L-Isomerengemisch
X - für Hydroxy, Benzyloxy oder Alkoxy mit bis zu 8 Kohlenstoffatomen oder Morpholino steht oder
- für eine Gruppe der Formel $-NHR^8$ steht
worin
$R^8$ - Wasserstoff,
- geradkettiges oder verzweigtes Alkyl mit bis zu 10 Kohlenstoffatomen bedeutet, das gegebenenfalls durch Hydroxy, Phenyl, Pyridyl oder durch den Rest der Formel

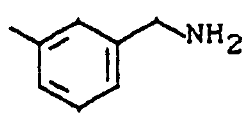

substituiert ist
oder
- für einen Rest der Formel

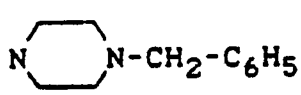

steht
und deren physiologisch unbedenklichen Salze.

2. Verbindungen der allgemeinen Formel (I) gemäß Anspruch 1, in welcher
$R^1$ - für Wasserstoff oder
- für Alkoxycarhonyl mit bis zu 6 Kohlenstoffatomen oder Benzyloxycarbonyl steht oder
- für eine Gruppe der Formel $R^3$-CO- steht
worin
$R^3$ - Morpholino oder die Reste

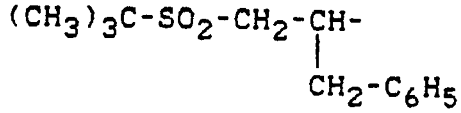

oder 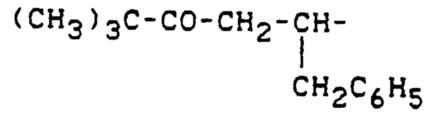

bedeutet
oder
- eine Gruppe der Formel $-NR^4R^5$ bedeutet
worin
$R^4$ und $R^5$ gleich oder verschieden sind und Wasserstoff, geradkettiges oder verzweigtes Alkyl mit bis zu 6 Kohlenstoffatomen oder Phenyl bedeuten
oder
- den Rest

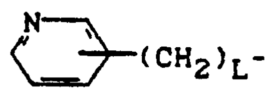

bedeutet
worin
L die Zahl 0, 1 oder 2 bedeutet
B - für eine direkte Bindung oder
- für eine Aminosäuregruppierung der Formel

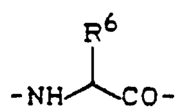

steht
worin
$R^6$ - geradkettiges oder verzweigtes Alkyl mit bis zu 6 Kohlenstoffatomen bedeutet, das gegebenenfalls durch Hydroxy, Alkoxy mit bis zu 4 Kohlenstoffatomen oder Benzyloxy substituiert ist
oder
- für Prolin steht
in ihrer L-Form, D-Form oder als D,L-Isomerengemisch
$R^2$ - für Phenyl oder Naphthyl steht, das gegebenenfalls bis zu 3-fach gleich oder verschieden durch Fluor, Chlor, Brom, Jod, Hydroxy, Nitro, Cyano, Alkoxy mit geradkettiges oder verzweigtes mit bis zu 6 Kohlenstoffatomen, Acetoxy oder Benzyloxy substituiert ist
w - eine Zahl 0 oder 1 bedeutet
A - für eine $-CH_2-$ oder für eine $-CH_2-CH_2-$Gruppe steht,
D und E gleich oder verschieden sind und
- für eine direkte Bindung oder
- für eine Aminosäuregruppierung der Formel

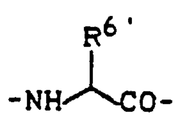

stehen
worin
$R^{6'}$ die oben angegebene Bedeutung von $R^6$ hat und mit dieser gleich oder verschieden ist
in ihrer L-Form, D-Form oder als D,L-Isomerengemisch
X - für Hydroxy, Benzyloxy oder Alkoxy mit bis zu 6 Kohlenstoffatomen oder Morpholino steht oder
- für eine Gruppe der Formel $-NHR^8$ steht
worin
$R^8$ - Wasserstoff
-geradkettiges oder verzweigtes Alkyl mit bis zu 8 Kohlenstoffatomen bedeutet, das gegebenenfalls durch Hydroxy, Phenyl, Pyridyl oder durch den Rest der Formel

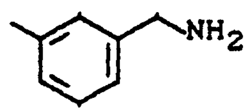

substituiert ist
oder
- für einen Rest der Formel

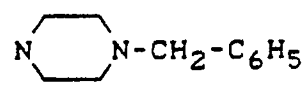

steht
und deren physiologisch unbedenklichen Salze.
3. Verbindungen der allgemeinen Formel (I) gemäß Anspruch 1,
in welcher
$R^1$ - für Wasserstoff oder
- für Alkoxycarbonyl mit bis zu 4 Kohlenstoffatomen oder Benzyloxycarbonyl steht,
- für eine Gruppe der Formel $R^3$-CO- steht
worin
$R^3$ - Morpholino oder eine Gruppe der Formel

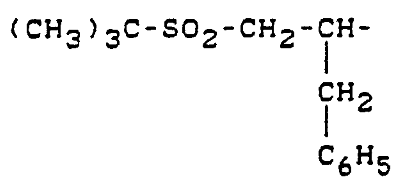

oder $-NR^4R^5$ bedeutet
worin
$R^4$ und $R^5$ gleich oder verschieden sind und Wasserstoff oder geradkettiges oder verzweigtes Alkyl mit bis zu 4 Kohlenstof fatomen oder Phenyl bedeuten
oder
- den Rest

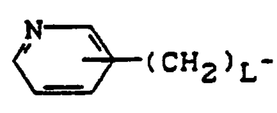

bedeutet
worin
L - die Zahl 0, 1 oder 2 bedeutet
B - für eine direkte Bindung oder

- für eine Aminosäuregruppierung der Formel

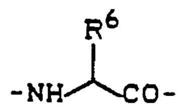

steht
worin
$R^6$ - geradkettiges oder verzweigtes Alkyl mit bis zu 4 Kohlenstoffatomen bedeutet, das gegebenenfalls durch Hydroxy substituiert ist
oder
- für Prolin steht
in ihrer L-Form, D-Form oder als D,L-Isomerengemisch
$R^2$ - für Phenyl oder Naphthyl steht, das gegebenenfalls durch Hydroxy, Methoxy, Ethoxy oder Propoxy, tert.-Butoxy, Benzyloxy oder Acetoxy substituiert ist,
w - eine Zahl 0 oder 1 bedeutet
A - für eine $-CH_2-$ oder für eine $-CH_2-CH_2-$Gruppe steht,
D und E gleich oder verschieden sind und
- für eine direkte Bindung oder
- für eine Aminosäuregruppierung der Formel

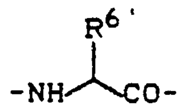

stehen
worin
$R^{6'}$ die oben angegebene Bedeutung von $R^6$ hat und mit dieser gleich oder verschieden ist
in ihrer L-Form, D-Form oder als D,L-Isomerengemisch
X - für Hydroxy oder Alkoxy mit bis zu 4 Kohlenstoffatomen oder Morpholino steht oder
- für eine Gruppe der Formel $-NHR^8$ steht
worin
$R^8$ - Wasserstoff oder
- geradkettiges oder verzweigtes Alkyl mit bis zu 6 Kohlenstoffatomen bedeutet, das gegebenenfalls durch Hydroxy, Phenyl, Pyridyl oder durch den Rest der Formel

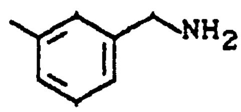

substituiert ist
oder
- für einen Rest der Formel

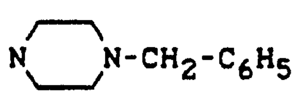

steht
und deren physiologisch unbedenklichen Salze.

4. Verfahren zur Herstellung von Peptiden der allgemeinen Formel (I), in welcher
$R^1$ - für Wasserstoff oder
- für Alkoxycarbonyl mit bis zu 8 Kohlenstoffatomen oder Benzyloxycarbonyl steht oder
- für eine Gruppe der Formel $R^3$-CO- steht

worin
$R^3$ - Morpholino oder die Reste

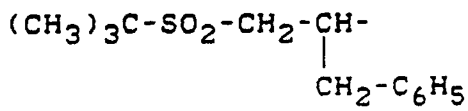

oder 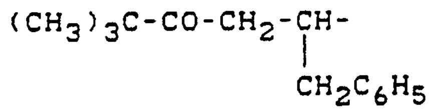

bedeutet
oder
-eine Gruppe der Formel $-NR^4R^5$ bedeutet
worin
$R^4$ und $R^5$ gleich oder verschieden sind und Wasserstoff, geradkettiges oder verzweigtes Alkyl mit bis zu 8 Kohlenstoffatomen oder Phenyl bedeuten
oder
- den Rest

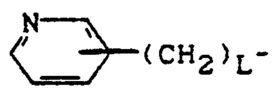

bedeutet
worin
L - die Zahl 0, 1 oder 2 bedeutet
B - für eine direkte Bindung oder
- für eine Aminosäuregruppierung der Formel

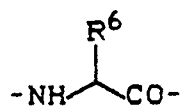

oder

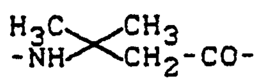

steht
worin
$R^6$ - geradkettiges oder verzweigtes Alkyl mit bis zu 8 Kohlenstoffatomen bedeutet, das gegebenenfalls durch Hydroxy, Carboxy, Alkoxy oder Alkoxycarbonyl mit bis zu 6 Kohlenstoffatomen oder Benzyloxy substituiert ist
oder
- für Prolin steht
in ihrer L-Form, D-Form oder als D,L-Isomerengemisch
$R^2$ - für Aryl mit 6 bis 10 Kohlenstoffatomen steht, das gegebenenfalls bis zu 3-fach gleich oder verschieden durch Halogen, Nitro, Cyano, Hydroxy, geradkettiges oder verzweigtes Alkoxy mit bis zu 8 Kohlenstoffatomen, Acetoxy, Benzyloxy oder durch eine Gruppe der ,Formel $O-CO-R^7$ substituiert ist
worin
$R^7$ geradkettiges oder verzweigtes Alkyl mit bis zu 8 Kohlenstoffatomen bedeutet
w - eine Zahl 0 oder 1 bedeutet
A - für eine $-CH_2-$ oder für eine $CH_2-CH_2$-Gruppe steht,
D und E gleich oder verschieden sind und
- für eine direkte Bindung oder
- für eine Aminosauregruppierung der Formel

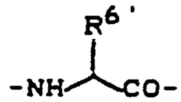

stehen
worin
$R^{6'}$ die oben angegebene Bedeutung von $R^6$ hat und mit dieser gleich oder verschieden ist
in ihrer L-Form, D-Form oder als D,L-Isomerengemisch
X - für Hydroxy, Benzyloxy oder Alkoxy mit bis zu 8 Kohlenstoffatomen oder Morpholino steht oder
- für eine Gruppe der Formel $-NHR^8$ steht
worin
$R^8$ Wasserstoff oder geradkettiges oder verzweigtes Alkyl mit bis zu 10 Kohlenstoffatomen bedeutet, das gegebenenfalls durch Hydroxy, Phenyl, Pyridyl oder durch den Rest der Formel

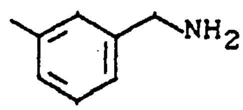

substituiert ist
oder
- für einen Rest der Formel

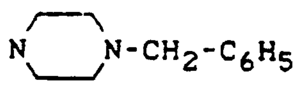

steht
und deren physiologisch unbedenklichen Salze,
dadurch gekennzeichnet, daß man
[A] Verbindungen der allgemeinen Formel (II)

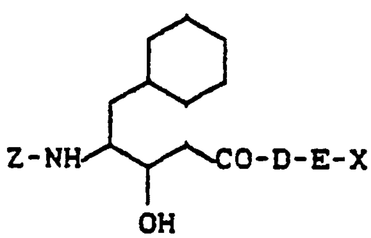

(II),

in welcher
D, E und X die oben angegebene Bedeutung haben und
Z - für eine Aminoschutzgruppe steht,
zunächst durch Abspaltung die Gruppe Z nach üblichen Methoden in Amine überführt und diese anschließend mit Verbindungen der allgemeinen Formel (III)

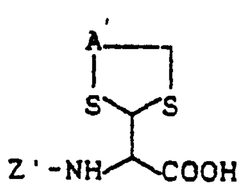

(III),

in welcher
A die oben angegebene Bedeutung hat,

und
Z' die oben angegebene Bedeutung von Z hat und mit dieser gleich oder verschieden ist,
unter Aktivierung der Carbonsäure nach üblichen Methoden in inerten Lösemitteln zu Verbindungen der allgemeinen Formel (Ia)

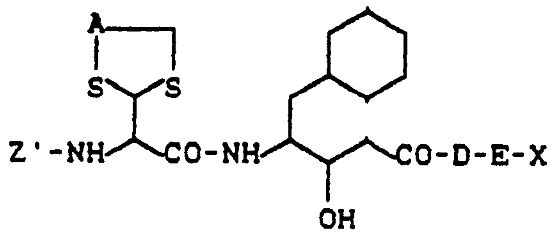
(Ia),

in welcher
Z', A, D, E und X die oben angegebene Bedeutung haben,
umsetzt, die Schutzgruppe Z' nach üblicher Methode abspaltet und in einem weiteren Schritt mit Verbindungen der allgemeinen Formel (IV)

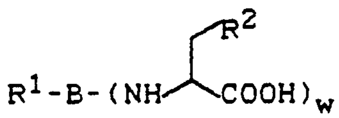
(IV),

in welcher
$R^1$, B, w und $R^2$ die oben angegebene Bedeutung haben,
gegebenenfalls unter der oben erwähnten Carbonsäureaktivierung kondensiert,
und gegebenenfalls die entsprechenden Ester nach üblicher Methode verseift, oder zunächst die Verbindungen der allgemeinen Formel (III) und (IV) nach der oben angegebenen Methode umsetzt und anschließend mit den Verbindungen der allgemeinen Formel (II) eine weitere Peptidknüpfung anschließt
oder indem man
[B] Verbindungen der allgemeinen Formel (Ib)

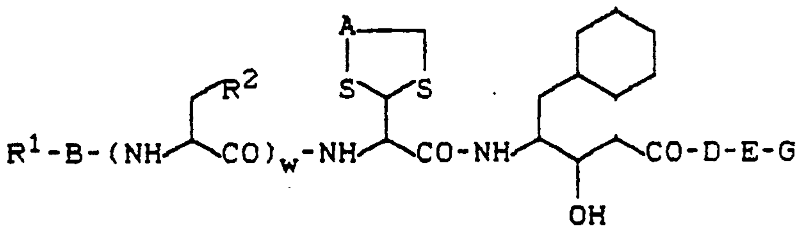
(Ib),

in welcher
A, B, $R^1$, $R^2$, w, D und E die oben angegebene Bedeutung haben,
und
G - für Alkoxycarbonyl mit bis zu 8 Kohlenstoffatomen steht,
nach üblicher Methode zunächst zu den entsprechenden Säuren verseift und in einem weiteren Schritt in Anwesenheit von Hilfsstoffen mit Aminen der allgemeinen Formel (V), oder Morpholin der Formel (VI) oder N-Benzylpiperazin der Formel (VII)
$H_2N-R^8$ (V),

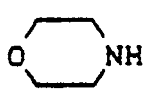 (VI) oder 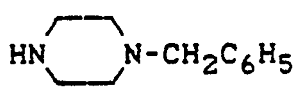 (VII)

in welcher

$R^8$ die oben angegebene Bedeutung hat,
kondensiert,
oder indem man

[C] entweder Verbindungen der allgemeinen Formel (Ic)

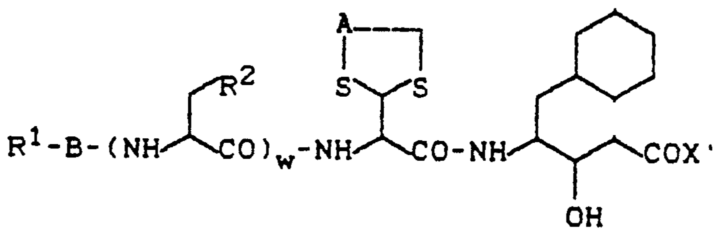

(Ic),

in welcher
A, B, $R^1$, w und $R^2$ die oben angegebene Bedeutung haben, und
X' - für Alkoxy mit bis zu 6 Kohlenstoffatomen oder Benzyloxy steht,
oder Verbindungen der allgemeinen Formel (Id)

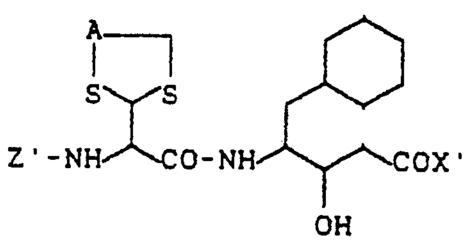

(Id),

in welcher
Z', A und X' die oben angegebene Bedeutung haben,
zunächst zu den entsprechenden Säuren nach üblicher Methode verseift und anschließend mit dem Bruchstück der allgemeinen Formel (VIII)
D-E-X (VIII),
in welcher
D, E und X die oben angegebene Bedeutung haben,
in inerten Lösemitteln, gegebenenfalls in Anwesenheit von Hilfsstoffen, umsetzt,
und im Fall der Verbindungen der allgemeinen Formel (Id) in einem nächsten Schritt nach der unter Verfahren [A] beschriebenen Methode unter schrittweiser Abspaltung der jeweiligen Schutzgruppe Z' mit Verbindungen der allgemeinen Formel (IV) oder (IVa)

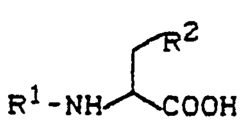 (IV) oder 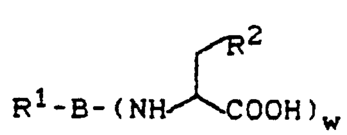 (IVa)

in welcher
$R^1$, B, w und $R^2$ die oben angegebene Bedeutung haben,
umsetzt.

5. Verbindungen der allgemeinen Formel (I) gemäß Anspruch 1 zur Verwendung bei der Bekämpfung von Krankheiten.

6. Arzneimittel enthaltend mindestens eine Verbindung der allgemeinen Formel (I) gemäß Anspruch 1.

7. Verfahren zur Herstellung von Arzneimitteln, dadurch gekennzeichnet, daß man Verbindungen der allgemeinen Formel (I) gemäß Anspruch 1, gegebenenfalls unter Verwendung von üblichen Hilfs- und Trägerstoffen in eine geeignete Applikationsform überführt.

8. Verbindungen der allgemeinen Formel (I) gemäß Anspruch 1 bei der Herstellung von kreislaufwirksamen Arzneimitteln.

9. Verbindungen der allgemeinen Formel (III)

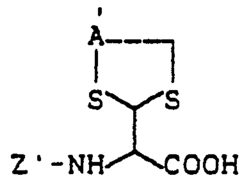

(III),

in welcher
A - für eine $-CH_2-$ oder für eine $-CH_2-CH_2-$Gruppe steht und
Z′ - für eine Aminoschutzgruppe steht.

10. Verfahren zur Herstellung von Verbindungen der allgemeinen Formel (III) gemäß Anspruch 9, dadurch gekennzeichnet, daß man Verbindungen der allgemeinen Formel (IX)

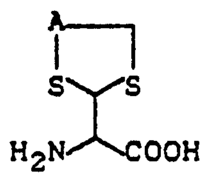

(IX),

in welcher
A die oben angegebene Bedeutung hat,
mit Aminoschutzgruppen einführenden Reagenzien nach üblichen Methoden in inerten Lösungsmitteln in Gegenwart einer Base bei Temperaturen zwischen 0° C und 150° C umsetzt.

Europäisches Patentamt

# EUROPÄISCHER RECHERCHENBERICHT

Nummer der Anmeldung

EP 90 11 0036

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| | Keine Entgegenhaltungen.<br>----- | | C 07 K 5/02<br>A 61 K 37/64 |
| | | | RECHERCHIERTE SACHGEBIETE (Int. Cl.5) |
| | | | C 07 K<br>A 61 K |

Der vorliegende Recherchenbericht wurde für alle Patentansprüche erstellt

| Recherchenort | Abschlußdatum der Recherche | Prüfer |
|---|---|---|
| DEN HAAG | 19-09-1990 | DEFFNER C-A.E. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)